(19) Europäisches Patentamt
European Patent Office
Office européen des brevets

(11) Veröffentlichungsnummer: **0 463 560 B1**

(12) **EUROPÄISCHE PATENTSCHRIFT**

(45) Veröffentlichungstag der Patentschrift: **25.10.95**

(51) Int. Cl.⁶: **C07F 9/38**, A61K 31/66, C07F 9/655, C07F 9/58, C07F 9/6553

(21) Anmeldenummer: **91110074.1**

(22) Anmeldetag: **19.06.91**

# (54) Neuartige Antiepileptika.

(30) Priorität: **22.06.90 CH 2092/90**
**13.02.91 CH 440/91**
**22.04.91 CH 1199/91**

(43) Veröffentlichungstag der Anmeldung:
**02.01.92 Patentblatt 92/01**

(45) Bekanntmachung des Hinweises auf die Patenterteilung:
**25.10.95 Patentblatt 95/43**

(84) Benannte Vertragsstaaten:
**AT BE CH DE DK ES FR GB GR IT LI LU NL SE**

(56) Entgegenhaltungen:
**EP-A- 0 181 833**
**EP-A- 0 319 479**
**FR-A- 2 282 431**

(73) Patentinhaber: **CIBA-GEIGY AG**
**Klybeckstrasse 141**
**CH-4002 Basel (CH)**

(72) Erfinder: **Marescaux, Christian, Prof.**
**22, rue du Faubourg de Pierre**
**F-67000 Strasbourg (FR)**

Erfinder: **Bernasconi, Raymond, Dr.**
**Amselstrasse 17A**
**CH-4104 Oberwil (CH)**
Erfinder: **Schmutz, Markus, Dr.**
**Baumgartenweg 24**
**CH-4124 Schönenbuch (CH)**
Erfinder: **Fröstl, Wolfgang, Dr.**
**St. Johanns-Vorstadt 6/2**
**CH-4056 Basel (CH)**
Erfinder: **Mickel, Stuart J., Dr.**
**Huppertstrasse 15**
**CH-4415 Lausen (CH)**

(74) Vertreter: **Zumstein, Fritz, Dr. et al**
**Patentanwälte,**
**Dr. F. Zumstein,**
**Dipl.-Ing. F. Klingseisen,**
**Bräuhausstrasse 4**
**D-80331 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 463 560 B1

## Beschreibung

Die Erfindung betrifft die Verwendung von Verbindungen mit $GABA_B$-antagonistischen Eigenschaften als Antiepileptika zur Behandlung von Epilepsien des "petit mal"-Formenkreises sowie zur Unterdrückung von "petit mal"-ähnlichen Zuständen, die bei der Behandlung mit Phenytoin, Carbamazepin, Vigabatrin® und Antiepileptika mit gleichem oder ähnlichen Wirkungsprofil auftreten können, bzw. zur Herstellung eines Antiepileptikums zur Behandlung von Epilepsien des "petit mal"-Formenkreises, diese enthaltende antiepileptische Arzneimittel bzw. ein Verfahren zur Herstellung solcher Arzneimittel sowie ein durch Verabreichung eines solchen gekennzeichnetes Verfahren zur Behandlung epileptischer Erkrankungen des "petit mal"-Formenkreises sowie zur Unterdrückung von "petit mal"ähnlichen Zuständen, die bei der Behandlung Phenytoin, Carbamazepin, Vigabatrin® und Antiepileptika mit gleichem oder ähnlichen Wirkungsprofil auftreten können, ebenso neue Verbindungen mit $GABA_B$-antagonistischen Eigenschaften, Verfahren zu ihrer Herstellung und diese enthaltende pharmazeutische Präparate.

Unter Verbindungen mit $GABA_B$-antagonistischen Eigenschaften sind Verbindungen zu verstehen, die eine Bindung an $GABA_B$-Rezeptoren einzugehen vermögen und an diesen als Antagonisten von GABA (g-aminobutyric acid) wirken. Beispielsweise handelt es sich um Verbindungen der Formel I

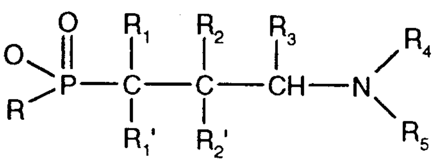

(I),

worin einer der Reste $R_1$, $R_2$ und $R_3$ Wasserstoff, $C_1$-$C_7$-Alkyl, $C_2$-$C_7$-Alkenyl, $C_2$-$C_7$ Alkinyl, $C_3$-$C_8$-Cycloalkyl, $C_3$-$C_8$-Cycloalkenyl-$C_1$-$C_4$-alkyl, Mono-, Di- oder Trihydroxy-$C_3$-$C_8$-cycloalkyl-$C_1$-$C_4$-alkyl, unsubstituiertes oder im Phenyl- bzw. Naphthylteil durch $C_1$-$C_4$-Alkyl, $C_1$-$C_4$-Alkoxy, Halogen und/oder Trifluormethyl ein- oder mehrfach substituiertes Phenyl- oder Naphthyl-$C_1$-$C_4$-alkyl, Diphenyl-$C_1$-$C_4$-alkyl oder Naphthyl-$C_1$-$C_4$-ralkyl, unsubstituiertes oder durch Halogen, $C_1$-$C_4$-Alkyl, $C_1$-$C_4$-Alkoxy und/oder Trifluormethyl substituiertes Phenyl oder Naphthyl, ein anderer Wasserstoff oder im Falle von $R_1$ oder $R_2$ Hydroxy oder im Falle von $R_1$ Halogen oder im Falle von $R_2$ gemeinsam mit $R_2$' Oxo bedeutet und der verbleibende Wasserstoff bedeutet, $R_1$' Wasserstoff oder Halogen ist, $R_2$' Wasserstoff, Hydroxy oder gemeinsam mit $R_2$ Oxo darstellt, $R_4$ und $R_5$ Wasserstoff darstellen oder $R_4$ unsubstituiertes oder im Phenyl-, Naphthyl-, Thienyl-, Furyl-oder Pyridylteil $C_1$-$C_4$-Alkyl, $C_1$-$C_4$-Alkoxy, Halogen und/oder Trifluormethyl ein- oder mehrfach substituiertes Phenyl-$C_1$-$C_4$-alkyl, Diphenyl-$C_1$-$C_4$-alkyl, Naphthyl-$C_1$-$C_4$-alkyl, Thienyl-$C_1$-$C_4$-alkyl, Furyl-$C_1$-$C_4$-alkyl oder Pyridyl-$C_1$-$C_4$-alkyl bedeutet und $R_5$ für Wasserstoff,, $C_1$-$C_7$-Alkyl, $C_2$-$C_7$-Alkenyl, $C_2$-$C_7$-Alkinyl oder eine Gruppe $R_4$ steht und R $C_2$-$C_7$-Alkyl,, $C_2$-$C_7$-Alkenyl, $C_2$-$C_7$-Alkinyl, $C_3$-$C_8$-Cycloalkyl, $C_3$-$C_8$-Cycloalkyl-$C_1$-$C_4$-alkyl, $C_3$-$C_8$-Cycloalkenyl-$C_1$-$C_4$-alkyl,, Mono-, Di- oder Trihydroxy-$C_3$-$C_8$-cycloalkyl-$C_1$-$C_4$-alkyl, $C_3$-$C_8$-Cycloalkyl(hydroxy)-$C_1$-$C_4$-alkyl, ($C_1$-$C_4$-Alkylthio)cycloalkyl-(hydroxy)-$C_1$-$C_4$-alkyl, unsubstituiertes oder im Phenyl- bzw. Naphthylteil durch $C_1$-$C_4$-Alkyl, $C_1$-$C_4$-Alkoxy, Halogen und/oder Trifluormethyl ein- oder mehrfach substituiertes Phenyl- oder Naphthyl-$C_1$-$C_4$-niederalkyl, unsubstituiertes oder durch Halogen, $C_1$-$C_4$-Alkyl, $C_1$-$C_4$-Alkoxy und/oder Trifluormethyl substituiertes Phenyl oder Naphthyl oder, sofern $R_1$ Wasserstoff oder Hydroxy, $R_2$ einen wie vorstehend definierten aromatischen Rest und $R_1$', $R_2$' sowie $R_3$ Wasserstoff darstellen, Methyl bedeutet, und ihre pharmazeutisch verwendbaren Salze.

Neue Verbindungen mit $GABA_B$-antagonistischen Eigenschaften sind vorzugsweise araliphatisch N-substituierte Aminoalkanphosphinsäuren der Formel I, worin $R_1$, $R_1$', $R_2$' und $R_3$ Wasserstoff bedeuten, $R_2$ Wasserstoff oder Hydroxy darstellt, $R_4$ einen im Phenyl- bzw. Naphthylteil durch $C_1$-$C_4$-Alkyl, $C_1$-$C_4$-Alkoxy, Hydroxy und/oder Halogen mono-, di-oder trisubstituierten Phenyl-$C_1$-$C_4$-alkyl, Diphenyl-$C_1$-$C_4$-alkyl- oder Naphthyl-$C_1$-$C_4$-alkylrest oder einen unsubstituierten oder im Thienyl-, Furyl- bzw. Pyridylteil durch Halogen substituierten Thienyl-, Furyl- oder Pyridyl-$C_1$-$C_4$-alkylrest bedeutet, $R_5$ Wasserstoff, $C_1$-$C_4$-Alkyl oder eine Gruppe $R_4$ darstellt und R $C_2$-$C_4$-Alkyl, $C_2$-$C_4$-Alkenyl, $C_2$-$C_7$-Alkinyl, Oxo-$C_2$-$C_7$-alkyl, Hydroxy-$C_2$-$C_7$-alkyl oder Dihydroxy-$C_2$-$C_7$-alkyl, $\alpha$-Hydroxy-$C_3$-$C_5$-alkenyl, Mono-, Di- oder Trifluor-$C_2$-$C_5$-alkyl, Mono-, Di- oder Trifluor-$C_2$-$C_5$-alkenyl, Mono-, Di- oder Trifluor-$\alpha$-hydroxy-$C_2$-$C_5$-alkyl, Mono-, Di-oder Trifluor-$\alpha$-hydroxy-$C_2$-$C_5$-alkenyl, $C_1$-$C_4$-Alkoxy-$C_1$-$C_4$-alkyl, Di-$C_1$-$C_4$-alkoxy-$C_1$-$C_4$-alkyl, $C_1$-$C_4$-Alkoxy $C_1$-$C_4$-Alkoxy-$C_2$-$C_7$-(hydroxy)-alkyl, $C_1$-$C_4$-Alkoxy-$C_2$-$C_5$-(halo)-alkyl, $C_1$-$C_4$-Alkylthio-$C_1$-$C_4$-alkyl, Di-$C_1$-$C_4$-alkylthio-$C_1$-$C_4$-alkyl, $C_3$-$C_8$-Cycloalkyl, $\alpha$-Hydroxy-$C_3$-$C_6$-cycloalkyl, Oxa- oder Thia-$C_3$-$C_6$-cycloalkyl, 1,3-Dioxa- oder 1,3-Dithia-$C_3$-$C_8$-cycloalkyl, $C_3$-$C_8$-Cycloalkyl-$C_1$-$C_4$-alkyl, $C_3$-$C_8$-Cycloalkenyl-$C_1$-$C_4$-alkyl, Mono-, Di- oder Trih-

ydroxy-$C_3$-$C_8$-cycloalkenyl, $C_3$-$C_6$-Cycloalkyl-(hydroxy)-$C_1$-$C_4$-alkyl, 1-($C_1$-$C_4$-Alkylthio-$C_3$-$C_6$-cycloalkyl)-1-hydroxy)-$C_1$-$C_4$-alkyl, unsubstituiertes oder durch $C_1$-$C_4$-Alkyl, $C_1$-$C_4$-Alkoxy, Halogen, Hydroxy und/oder Trifluormethyl mono-, di- oder trisubstituiertes Mono- oder Diphenyl-$C_1$-$C_4$-alkyl, Naphthyl-$C_1$-$C_4$-alkyl 1 oder unsubstituiertes oder durch Halogen substituiertes Thienyl-, Furyl- oder Pyridyl-$C_1$-$C_4$-alkyl bedeutet, und ihre pharmazeutisch verwendbaren Salze.

Reste $R_1$, $R_2$ bzw. $R_3$ vorzugsweise, beispielsweise durch Halogen, Niederalkyl, Niederalkoxy und/oder Trifluormethyl, substituiert, insbesondere durch Halogen monosubstituiert ist, sowie Naphthyl.

Vor- und nachstehend sind unter niederen Resten und Verbindungen beispielsweise solche zu verstehen, die bis und mit 7, vorzugsweise bis und mit 4, Kohlenstoffatome (C-Atome) aufweisen.

$C_2$-$C_7$-Alkyl R ist beispielsweise $C_3$-$C_5$-Alkyl, wie Propyl, Isopropyl, Butyl, Isobutyl, Sekundärbutyl, Tertiärbutyl oder Pentyl, kann aber auch oder eine $C_6$-$C_7$-Alkyl-, wie Hexyl- oder Heptylgruppe sein.

$C_1$-$C_7$-Alkyl $R_5$ ist beispielsweise $C_1$-$C_4$-Alkyl, wie Methyl, Äthyl, Propyl, Isopropyl oder Butyl, kann aber auch Isobutyl, Sekundärbutyl, Tertiärbutyl oder eine $C_5$-$C_7$-Alkyl-, wie Pentyl-, Hexyl- oder Heptylgruppe sein.

$C_2$-$C_4$-Alkenyl ist beispielsweise Vinyl, Allyl oder But-2-enyl, kann aber auch eine $C_5$-$C_7$-Alkenyl-, wie Pentenyl, Hexenyl oder Heptenyl sein.

$C_2$-$C_7$-Alkinyl bedeutet beispielsweise $C_3$-$C_5$-Alkinyl, welches die Doppelbindung in höherer als der $\alpha,\beta$-Stellung trägt, z.B. 2-Propinyl (Propargyl), But-3-in-1-yl, But-2-in-1-yl oder Pent-3-in-1-yl.

Oxo-$C_2$-$C_7$-alkyl trägt die Oxogruppe vorzugsweise in höherer als der $\alpha$-Stellung und bedeutet beispielsweise Oxo-$C_3$-$C_6$-alkyl, wie 2-Oxopropyl, 2- oder 3-Oxobutyl oder 3-Oxopentyl.

Hydroxy-$C_2$-$C_7$-alkyl trägt die Hydroxygruppen vorzugsweise in $\alpha$- oder $\beta$-Stellung und bedeutet beispielsweise 1-Hydroxyäthyl, 1-oder 2-Hydroxypropyl, 2-Hydroxyprop-2-yl, 1- oder 2-Hydroxybutyl, 1-Hydroxyisoburyl oder 2-Hydroxy-3-methyl-butyl.

Dihydroxy-$C_3$-$C_7$-alkyl trägt die Hydroxygruppen insbesondere in $\alpha,\beta$-Stellung und ist beispielsweise $\alpha,\beta$-Dihydroxy-$C_3$-$C_7$-alkyl, wie 1,2-Dihydroxyprop-2-yl.

Hydroxy-$C_3$-$C_5$-alkenyl trägt die Hydroxygruppen vorzugsweise in $\alpha$- und die Doppelbindung vorzugsweise in höherer als der $\alpha,\beta$-Stellung und bedeutet beispielsweise entsprechendes $\alpha$-Hydroxy-$C_3$-$C_5$-alkenyl, z.B. 1-Hydroxybut-2-enyl.

Mono-, Di- oder Trifluor-$C_2$-$C_5$-alkenyl ist beispielsweise 1-Fluorbut-2-enyl.
entsprechendes Mono-, Di- oder Trifluor-$\alpha$-hydroxy-$C_2$-$C_7$-alkyl trägt die Hydroxygruppe vorzugsweise in $\alpha$-Stellung und die Halogenatome vorzugsweise in höherer als der $\alpha$-Stellung und steht beispielsweise für 4,4,4-Trifluor-1-hydroxy-butyl.

Mono-, Di- oder Polyhalogenniederalkyl ist beispielsweise Mono-, Di- oder Trifluor-$C_2$-$C_5$-alkyl, wie 3,3,3-Trifluorpropyl, 4,4,4-Trifluorbutyl, 1- oder 2-Fluorbutyl oder 1,1-Difluorbutyl. Niederalkoxy ist beispielsweise $C_1$-$C_7$-Alkoxy, vorzugsweise $C_1$-$C_4$-Alkoxy, wie Methoxy, Äthoxy, Propyloxy, Isopropyloxy oder Butyloxy, kann aber auch Isobutyloxy, Sekundärbutyloxy, Tertiärbutyloxy oder eine $C_5$-$C_7$-Alkoxy-, wie Pentyloxy-, Hexyloxy- oder Heptyloxygruppe sein.
entsprechendes Mono-, Di- oder Trifluor-$\alpha$-hydroxy-$C_2$-$C_5$-alkenyl die Hydroxygruppe vorzugsweise in $\alpha$-Stellung und die Halogenatome vorzugsweise in höherer als der $\alpha$-Stellung und steht beispielsweise für 2-Fluor-1-hydroxy-buten-2-yl.

$C_1$-$C_4$-Alkoxy-$C_1$-$C_4$-alkyl ist beispielsweise Methoxy-oder Äthoxymethyl, 2-Methoxyäthyl, 2-Äthoxyäthyl, 3-Methoxy- oder 3-Äthoxypropyl oder 1- oder 2-Methoxybutyl.

Di-$C_1$-$C_4$-alkoxy-$C_1$-$C_4$-alkyl ist beispielsweise Dimethoxymethyl, Dipropyloxymethyl, 1,1- oder 2,2-Diäthoxyäthyl, Diisopropyloxymethyl, Dibutyloxymethyl oder 3,3-Dimethoxypropyl.
beispielsweise $C_1$-$C_4$-Alkoxy-$C_2$-$C_7$-(hydroxy)alkyl ist beispeilsweise 2-Hydroxy-3-methoxy-prop-2-yl.

$C_1$-$C_4$-Alkoxy-$C_2$-$C_5$-(halo)niederalkyl ist beispielsweise, wie 2-Fluor-3-methoxy-butyl.

$C_1$-$C_4$-Alkylthio-$C_1$-$C_4$-alkyl ist beispielsweise Methylthio- oder Äthylthiomethyl, 2-Methylthioäthyl, 2-Äthylthioäthyl, 3-Methylthio-oder 3-Äthylthiopropyl oder 1- oder 2-Methylthiobutyl.

Di-$C_1$-$C_4$-alkylthio-$C_1$-$C_4$-alkyl ist beispielsweise Dimethylthiomethyl, Dipropylthiomethyl, 1,1- oder 2,2-Diäthylthioäthyl, Diisopropylthiomethyl, Dibutylthiomethyl oder 3,3-Dimethylthiopropyl.

Mono- oder Diphenyl-$C_1$-$C_4$-alkyl ist beispielsweise Benzyl, 1- oder 2-Phenyläthyl oder 2-Phenylprop-2-yl, Diphenylmethyl oder in zweiter Linie 2-Phenyläthyl, 2-Phenylprop-1-yl oder 3-Phenylprop-1-yl.

Naphthyl-$C_1$-$C_4$-alkyl ist beispielsweise 1- oder 2-Naphthylmethyl.

Thienyl-, Furyl- oder Pyridyl-$C_1$-$C_4$-alkyl ist beispielsweise Thienyl-, Furyl- oder Pyridylmethyl, 1-Thienyl-, 1-Furyl- oder 1-Pyridyläthyl, 2-Thienyl-, 2-Furyl- oder 2-Pyridylprop-2-yl, oder in zweiter Linie 2-Thienyl-, 2-Furyl- oder 2-Pyridyläthyl, 2-Thienyl-, 2-Furyl- oder 2-Pyridylprop-1-yl oder 3-Thienyl-, 3-Furyl- oder 3-Pyridylprop-1-yl.

Halogen ist beispielsweise Halogen der Atomnummer bis und mit 53, wie Chlor, Jod oder Fluor, ferner Brom.

$C_3$-$C_8$-Cycloalkyl ist beispielsweise $C_3$-$C_6$-Cycloalkyl, wie Cyclopropyl, Cyclobutyl, Cyclopentyl oder Cyclohexyl.

$\alpha$-Hydroxy-$C_3$-$C_6$-cycloalkyl ist beispielsweise 1-Hydroxycyclopropyl, 1-Hydroxycyclobutyl oder 1-Hydroxycyclohexyl.

Oxa- oder Thia-$C_3$-$C_8$-cycloalkyl ist beispielsweise Oxa- oder Thia-$C_3$-$C_6$-cycloalkyl, wie 2-Oxacyclopropyl (Oxiranyl), 2- oder 3-Oxacyclobutyl (Oxetanyl), 2- oder 3-Thiacyclobutyl (Thietanyl), 2- oder 3-Oxacyclopentyl (Tetrahydrofuranyl), 2- oder 3-Thiacyclopentyl (Thiolanyl) oder 2-Oxacyclohexyl (Tetrahydropyranyl).

1,3-Dioxa-$C_3$-$C_8$-cycloalkyl ist beispielsweise 1,3-Dioxolan-2-yl oder 1,3-Dioxan-2-yl.

1,3-Dithia-$C_3$-$C_8$-cycloalkyl ist beispielsweise 1,3-Dithiolan-2-yl oder 1,3-Dithian-2-yl.

$C_3$-$C_8$-Cycloalkyl-$C_1$-$C_4$-alkyl ist beispielsweise $C_3$-$C_6$-Cycloalkyl-$C_1$-$C_4$-alkyl, wie $\alpha$-($C_3$-$C_6$-Cycloalkyl)-$C_1$-$C_4$-alkyl, z.B. Cyclopropylmethyl, Cyclobutylmethyl, Cyclopentylmethyl oder Cyclohexylmethyl.

$C_3$-$C_8$-Cycloalkenyl-$C_1$-$C_4$-alkyl ist beispielsweise $C_3$-$C_6$-Cycloalkenyl-$C_1$-$C_4$-alkyl, wie a-($C_3$-$C_6$-Cycloalkenyl)-$C_1$-$C_4$-alkyl, z.B. Cyclohex-3-enyl.

Mono-, Di- oder Trihydroxy-$C_3$-$C_8$-cycloalkyl-$C_1$-$C_4$-alkyl ist beispielsweise Mono-, Di- oder Trihydroxy-$C_3$-$C_6$-cycloalkyl-$C_1$-$C_4$-alkyl, wie a-(Mono-, Di- oder Trihydroxy-$C_3$-$C_6$-cycloalkyl)-$C_1$-$C_4$-alkyl, z.B. 3,4-Dihydroxycyclohexylmethyl oder 3,4,5-Trihydroxycyclohexylmethyl.

Cycloalkyl(hydroxy)niederalkyl ist beispielsweise $C_3$-$C_6$-Cycloalkyl-$C_1$-$C_4$-(hydroxy)alkyl, wie a-($C_3$-$C_6$-Cycloalkyl)-$\alpha$-hydroxy-$C_1$-$C_4$-alkyl, z.B. Cyclopropyl(hydroxy)methyl, Cyclobutyl(hydroxy)methyl, oder Cyclohexyl(hydroxy)methyl.

1-($C_1$-$C_4$-Alkylthio-$C_3$-$C_6$-cycloalkyl)-1-hydroxy-$C_1$-$C_4$-alkyl ist beispielsweise (2-Methylthiocycloprop-1-yl)-hydroxymethyl.

Die Verbindungen der Formel I liegen auf Grund ihres amphoteren Charakters in Form innerer Salze vor und können sowohl Säureadditionssalze als auch Salze mit Basen bilden.

Säureadditionssalze von Verbindungen der Formel I sind beispielsweise deren pharmazeutisch verwendbare Salze mit geeigneten Mineralsäuren, wie Halogenwasserstoffsäuren Schwefelsäure oder Phosphorsäure z.B. Hydrochloride, Hydrobromide, Sulfate, Hydrogensulfate oder Phosphate, oder Salze mit geeigneten aliphatischen oder aromatischen Sulfonsäuren oder N-substituierten Sulfaminsäuren, z.B. Methansulfonate, Benzolsulfonate, p-Toluolsulfonate oder N-Cyclohexylsulfaminate (Cyclamate).

Salze von Verbindungen der Formel I mit Basen sind beispielsweise deren Salze mit pharmazeutisch verwendbaren Basen, wie nicht-toxische, von Metallen der Gruppen Ia, Ib, IIa und IIb abgeleitete Metallsalze, z.B. Alkalimetall-, insbesondere Natrium- oder Kaliumsalze, Erdalkalimetall-, insbesondere Calcium- oder Magnesiumsalze, ebenso Ammoniumsalze mit Ammoniak oder organischen Aminen oder quaternären Ammoniumbasen, wie gegebenenfalls C-hydroxylierten aliphatischen Aminen, insbesondere Mono-, Di- oder Triniederalkylaminen, z.B. Methyl-, Äthyl- oder Diäthylamin, Mono-, Di- oder Tri-(hydroxyniederalkyl)aminen, wie Äthanol-, Diäthanol- oder Triäthanolamin, Tris-(hydroxymethyl)methylamin oder 2-Hydroxytertiärbutylamin, oder N-(Hydroxyniederalkyl)-N,N-diniederalkyl-aminen bzw. N-(Polyhydroxyniederalkyl)-niederalkylaminen, wie 2-(Dimethylamino)äthanol oder D-Glucamin, oder quaternären aliphatischen Ammoniumhydroxiden, z.B. Tetrabutylammoniumhydroxid.

Abhängig vom Vorhandensein asymmetrischer Kohlenstoffatome können die erfindungsgemässen Verbindungen in Form von Isomerengemischen, speziell als Racemate, oder in Form reiner Isomere, speziell von optischen Antipoden vorliegen.

In der FR-A-2282431 sind Verbindungen der Formel I, worin R, $R_1$, $R_2$ und $R_5$ Wasserstoff oder einen aliphatischen Kohlenwasserstoffrest bedeuten, $R_4$ für Alkyl, Cycloalkyl oder Aralkyl steht und $R_1'$, $R_2$ und $R_3$ für Wasserstoff stehen, als Antstatika, Grundanstriche für metallische Oberflächen, Desinfektionsmittel, als Flotations- und Emulgiermittel sowie als Hilfsmittel für die Ausrüstung und Färbung von Textilmaterialien vorgeschlagen worden.

Weiterhin sind in EP-A-181833 Verbindungen der Formel I, worin R eine Gruppe der Formeln -C($C_1$-$C_4$-Alkyl)($OR^a$)$OR^b$ oder -CH($OR^a$)$OR^b$ bedeutet und $R^a$ sowie $R^b$ für $C_1$-$C_4$-Alkyl stehen, einer der Reste $R_1$, $R_2$ und $R_3$ Wasserstoff, $C_1$-$C_8$-Alkyl, $C_3$-$C_6$-Cycloalkyl, gegebenenfalls durch $C_1$-$C_4$-Alkyl, $C_1$-$C_4$-Alkoxy, Halogen und/oder Trifluormethyl substituiertes Phenyl, gegebenenfalls durch Halogen, $C_1$-$C_4$-Alkyl, $C_1$-$C_4$-Alkoxy und/oder Trifluormethyl substituiertes $C_7$-$C_{10}$-Phenylalkyl darstellt und die anderen sowie $R_1'$, $R_2'$ und $R_5$ Wasserstoff bedeuten und $R_4$ 1-Aryl-$C_1$-$C_4$-alkyl ist, als Zwischenprodukte für Herstellung entsprechender am P-Atom unsubstituierter 3-Aminopropylphosphinsäuren beschrieben worden.

Insbesondere sind in EP-A-319,479 Verbindungen der Formel I, worin einer der Reste $R_1$, $R_2$ und $R_3$ Wasserstoff oder einen aliphatischen, cycloaliphatischen, araliphatischen oder aromatischen Rest, ein anderer Wasserstoff oder im Falle von $R_1$ oder $R_2$ Hydroxy und der verbleibende Wasserstoff darstellt, und

worin $R_1'$, $R_2'$, $R_4$ und $R_5$ Wasserstoff bedeuten, mit $GABA_B$-antagonistischen Eigenschaften und ihre pharmazeutisch verwendbaren Salze beschrieben und als nootropische, anxiolytische bzw. antidepressive Arzneimittelwirkstoffe vorgeschlagen.

Die neuen Verbindung der Formel I und ihre pharmazeutisch verwendbaren Salze weisen ebenfalls wertvolle $GABA_B$-antgonistische Eigenschaften auf. Insbesondere zeigen sie eine wirksame Bindung an den $GABA_B$-Rezeptor und erweisen sich als Antagonisten von GABA (g-aminobutyric acid) an diesem Rezeptor. Mechanistisch gesehen kann Antagonismus an $GABA_B$ -Rezeptoren die Freisetzung von schnellen Reiz-Aminosäuren-Transmittern, d.h. Glutamat und Aspargat, steigern und so die Informationsverarbeitung im Gehirn verbessern. Damit stimmt die Erkenntnis überein, dass das späte postsynaptische Inhibitionspotential im Hippocampus, das einem $GABA_B$ -Mechanismus zugeschrieben wird, durch die Antagonisten abgebaut wird und damit eine schnellere Nervenimpulsübertragungsfolge ermöglicht.

Andererseits wurde gefunden, dass die chronische Behandlung mit Antidepressiva und wiederholte Elektroschocks die Zahl der $GABA_B$-Rezeptoren in der Hirnrinde bei Ratten erhöht. In Übereinstimmung mit Rezeptortheorien sollte die chronische Behandlung mit GABAB-Antagonisten zu derselben Wirkung führen. Aus diesem und anderen Gründen können daher $GABA_B$-Antagonisten als Antidepressiva wirken.

Die erfindungsgemässen neuen $GABA_B$-Antagonisten wechselwirken am GABAB-Rezeptor mit $IC_{50}$-Werten ab etwa $10^{-8}$ M (Mol/l) an Rattenhirnrindenmembranen. Im Gegensatz zu $GABA_B$-Agonisten wie Baclofen potenzieren sie nicht die Stimulierung von Adenylatcyclase an Rattenhirnrindenschnitten durch Noradrenalin, sondern wirken als Antagonist der Baclofen-Wirkung. Der Antagonismus gegenüber Baclofen kann auch an elektrophysiologischen Modellen *in vitro* gezeigt werden, z.B. am Penicillin-induzierten "epileptischen" Hippocampus-Schnittpräparat, wo Baclofen bei einer Konzentration von 6 $\mu$M (Mikromol/Liter) "epilepsieartige" Entladungen von Pyramidenzellen inhibiert. Die erfindungsgemässen Verbindungen wirken als Antagonist der Baclofen-Wirkung bei Konzentrationen von etwa 10 bis etwa 100 $\mu$M (Mikromol/Liter). *In vivo* kann der Antagonismus durch Iontophorese von Baclofen an Rattenhirnrinde und durch systemische Anwendung von Antagonisten in Dosen von 10-100 mg/kg bewiesen werden. Bei Dosen von etwa 30 mg/kg tritt Antagonismus gegen die muskelrelaxierende Wirkung von Baclofen ein, die im Rotarod-Modell gemessen wird.

Die neuen $GABA_B$-Antagonisten zeigen nicht nur Antagonismus gegen Baclofen, sondern weisen auch eine eigenständige Wirkung als Antagonisten von endogenem GABA auf. Somit sind die Antagonisten aktiv bei herkömmlichen Verhaltensmodellen, die charakteristisch sind für antidepressive, anxiolytische und/oder nootrope Eigenschaften. Verbindungen der Formel (I), so wurde gefunden, sind bei oraler Anwendung aktiv im Schwimmtest nach Porsolt, im Geller-Test, dem verzögerten passiven Meidungstest (Einversuchs-Modifizierung) in Vorversuchs- und Nachversuchs-Situationen, im Zweikammertest und im komplexen Labyrinth. Ausserdem wurde bei Untersuchungen an Rhesusaffen ein gesteigerter Spieltrieb, Neugierde, soziales Pflegeverhalten und eine Verminderung von Angstmerkmalen beobachtet.

Die neuen Verbindungen der Formel I und ihre pharmazeutisch verwendbaren Salze sind deshalb vorzüglich geeignet als Nootropika, Antidepressiva und Anxiolytika, z.B. zur Behandlung von cerebralen Insuffizienzerscheinungen, Gemütsdepressionen und von Angstzuständen.

Auf Grund des Antagonismus der bereits bekannten $GABA_B$-Antagonisten gegenüber Baclofen wurde bislang davon ausgegangen, dass $GABA_B$-Antagonisten, wie Verbindungen der Formel I, keine antiepileptische Wirkungskomponente besitzen.

Es wurde nun überraschend festgestellt, dass $GABA_B$-Antagonisten, insbesondere Verbindungen der Formel I, *in vivo* ausgeprägte Antiabsenz-Eigenschaften aufweisen.

Diese können an einem bestimmten Rattenstamm anhand ihrer ausgeprägten Hemmwirkung auf spontane "Spike and Wave"-Entladungen in folgendem Tiermodell für Absenz-Epilepsie dokumentiert werden.

Etwa 30% der im Centre de Neurochimie in Strasbourg gezüchteten Wistar-Ratten zeigen spontane Verhaltensveränderungen, unter denen Elektro-Enzephalogramm (EEG) und Symptome den menschlichen Absenzen (Petit mal) vergleichbar sind. Synchrone "Spike and Wave"-Entladungen (SWE; Frontoparietal-Cortex; 7-8 Hz; 300-1000 $\mu$V, Dauer 0,5 bis 40 s, Mittelwert = 6,0'3,4s) und öfters Myoclonia facialis begleiten einen Verhaltensstillstand. Diese Absenz-ähnliche Zustände treten spontan und wiederholt auf. Durch selektive Züchtung dieser Ratten war es, möglich einen Stamm zu erhalten, bei welchem 100% der Ratten diese SWE zeigen (epileptische Ratten). Umgekehrt konnte ein Stamm gezüchtet werden, bei welchem 100% der Ratten SWE-frei sind (Kontroll-Ratten). Dieses pharmakologische Modell ist beschrieben in Vergnes M., Marescaux C., Micheletti G., Reis J., Depaulis A., Rumbach L. und Warter J.M., Neurosci. Lett. 33, 97-101 (1982).

In diesem Modell vermindern beispielsweise die Klinisch verwendeten Antiepileptika Ethosuximid, Diazepam, Trimethadion und Natriumvalproat in Dosen r 25 mg/kg (Ethosuximid,), ≥0,5 mg/kg (Diazepam)

bzw. ≥50 mg/kg (Trimethadion und Natriumvalproat) die Spike and Wave-Entladungen dosisabhängig. Carbamazepin und Phenytoin sind unwirksam oder verschlimmern bei hoher Dosierung die Anfälle. Phenobarbital ist wirksam bei 2,5 bis 10 mg/kg und ist unwirksam bei 20 mg/kg. Die Wirkung dieser Antiepileptika auf die Krisen bei der Ratte und Absenzen beim Menschen unterstützen die Hypothese, dass dieses Tiermodell ein pharmakologisches Modell für Absenz-Epilepsie darstellt. Sein prädiktiver Wert scheint mindestens so gut zu sein wie derjenige anderer gebräuchlicher Tiermodelle.

Die Verbindungen der Formel I und ihre pharmazeutisch verwendbaren Salze sind deshalb neben ihrer Eignung als Nootropika, Antidepressiva und Anxiolytika vorzüglich geeignet als Arzneimittelwirkstoffe in Antiepileptika zur Behandlung von Epilepsien des "petit mal"-Formenkreises, sowohl der spontanen Absenzepilepsien, wie spontanen Absenzepilepsien bei Kindern und Jugendlichen sowie von atypischen Absenzen, wie Absenzen des Lennox-Gastaut-Syndroms, als auch von solchen, die bei der Behandlung mit üblichen "grand mal"-Antiepileptika, wie Phenytoin, Carbamazepin oder Vigabatrin® und Antiepileptika mit gleichem oder ähnlichem Wirkungsprofil, als unerwünschte Nebenwirkungen auftreten.

Die Erfindung betrifft in erster Linie die Verwendung von Verbindungen der Formel I, worin einer der Reste $R_1$, $R_2$ und $R_3$ Wasserstoff, $C_1$-$C_4$-Alkyl, $C_3$-$C_6$-Cycloalkyl, unsubstituiertes oder im Phenyl- bzw. Naphthylteil durch $C_1$-$C_4$-Alkyl, $C_1$-$C_4$-Alkoxy, Halogen und/oder Trifluormethyl ein- oder mehrfach substituiertes Phenyl-$C_1$-$C_4$-alkyl, unsubstituiertes oder im Phenyl- bzw. Naphthylteil durch $C_1$-$C_4$-Alkyl, $C_1$-$C_4$-Alkoxy, Halogen und/oder Trifluormethyl ein- oder mehrfach substituiertes Phenyl, ein anderer Wasserstoff oder im Falle von $R_1$ oder $R_2$ Hydroxy und der verbleibende Wasserstoff bedeutet, $R_1$' Wasserstoff ist, $R_2$' Wasserstoff, Hydroxy oder gemeinsam mit $R_2$ Oxo darstellt, $R_4$ und $R_5$ Wasserstoff darstellen oder $R_4$ einen unsubstituierten oder im Phenylteil durch $C_1$-$C_4$-Alkyl, $C_1$-$C_4$-Alkoxy, Halogen und/oder Trifluormethyl ein- oder mehrfach substituierten Phenyl-$C_1$-$C_4$-alkylrest bedeutet und $R_5$ für Wasserstoff, $C_1$-$C_4$-Alkyl oder eine Gruppe $R_4$ steht und R $C_2$-$C_7$-Alkyl, $\alpha,\alpha$-Di-$C_1$-$C_4$-alkoxy-$C_2$-$C_7$-alkyl, $\alpha,\alpha$-Dihalogen-$C_2$-$C_7$-alkyl, $C_3$-$C_6$-Cycloalkyl, $C_3$-$C_6$-Cycloalkyl-$C_1$-$C_4$-alkyl, $C_3$-$C_8$-Cycloalkenyl-$C_1$-$C_4$-alkyl, Mono-, Di- oder Trihydroxy-$C_3$-$C_8$-cycloalkyl-$C_1$-$C_4$-alkyl, Benzyl oder, sofern $R_2$ Halogenphenyl und $R_1$, $R_1$', $R_2$' sowie $R_3$ Wasserstoff darstellen, Methyl bedeutet, und jeweils ihre pharmazeutisch verwendbaren Salze als Antiepileptika bzw. zur Herstellung eines Antiepileptikums zur Behandlung von Epilepsien des "petit mal"-Formenkreises, diese enthaltende antiepileptische Arzneimittel bzw. ein Verfahren zur Herstellung solcher Arzneimittel sowie.

Die Erfindung betrifft in erster Linie beispielsweise Verbindungen der Formel I, worin $R_1$, $R_1$', $R_2$' und $R_3$ Wasserstoff bedeuten, $R_2$ Wasserstoff oder Hydroxy darstellt, $R_4$ einen durch $C_1$-$C_4$-Alkyl, $C_1$-$C_4$-Alkoxy, Hydroxy und/oder Halogen substituierten Phenyl- oder Naphthyl-$C_1$-$C_4$-alkylrest oder einen unsubstituierten oder durch durch Halogen substituierten Thienyl-, Furyl- oder Pyridyl-$C_1$-$C_4$-alkylrest bedeutet, $R_5$ Wasserstoff, Niederalkyl oder eine Gruppe $R_4$ darstellt und R $C_2$-$C_4$-Alkyl, $C_2$-$C_4$-Alkenyl, $C_2$-$C_7$-Alkinyl, Oxo-$C_2$-$C_7$-alkyl, Hydroxy-$C_2$-$C_7$-alkyl oder Dihydroxy-$C_2$-$C_7$-alkyl, $\alpha$-Hydroxy-$C_3$-$C_5$-alkenyl. Mono-, Di- oder Mono-, Di- oder Trifluor-$C_2$-$C_5$-alkyl, Mono-, Di- oder Trifluor-$C_2$-$C_5$-alkenyl, Mono-, Di- oder Trifluor-$\alpha$-hydroxy-$C_2$-$C_5$-alkyl, Mono-, Di- oder Trifluor-$\alpha$-hydroxy-$C_2$-$C_5$-alkenyl, $C_1$-$C_4$-Alkoxy-$C_1$-$C_4$-alkyl, Di-$C_1$-$C_4$-alkoxy-$C_1$-$C_4$-alkyl, $C_1$-$C_4$-Alkoxy-$C_2$-$C_7$-(hydroxy)-alkyl, , $C_1$-$C_4$-Alkoxy-$C_2$-$C_5$-(halo)-alkyl, $C_1$-$C_4$-Alkylthio-$C_1$-$C_4$-alkyl, Di-$C_1$-$C_4$-alkylthio-$C_1$-$C_4$-alkyl, $C_3$-$C_8$-Cycloalkyl, $\alpha$-Hydroxy-$C_3$-$C_6$-cycloalkyl, Oxa- oder Thia-$C_3$-$C_6$-cycloalkyl, 1,3-Dioxa- oder 1,3-Dithia-$C_3$-$C_8$-cycloalkyl, $C_3$-$C_6$-Cycloalkyl-(hydroxy)-$C_1$-$C_4$-alkyl, 1-($C_1$-$C_4$-Alkylthio-$C_3$-$C_6$-cycloalkyl)-1-hydroxy)-$C_1$-$C_4$-alkyl oder unsubstituiertes oder durch $C_1$-$C_4$-Alkyl, $C_1$-$C_4$-Alkoxy, Hydroxy und/oder Halogen substituierten Phenyl-$C_1$-$C_4$-alkyl oder Naphthyl-$C_1$-$C_4$-alkyl bedeutet, und ihre Salze, insbesondere ihre pharmazeutisch verwendbaren Salze.

Die Erfindung betrifft vor allem die Verwendung von Verbindungen der Formel I, worin einer der Reste $R_1$, $R_2$ und $R_3$ Wasserstoff, $C_1$-$C_4$-Alkyl, $C_3$-$C_6$-Cycloalkyl, unsubstituiertes oder im Phenyl- bzw. Naphthylteil durch $C_1$-$C_4$-Alkyl, $C_1$-$C_4$-Alkoxy, Halogen und/oder Trifluormethyl ein- oder mehrfach substituiertes Phenyl- oder Naphthyl-$C_1$-$C_4$-alkyl, unsubstituiertes oder im Phenyl- bzw. Naphthylteil durch $C_1$-$C_4$-Alkyl, $C_1$-$C_4$-Alkoxy, Halogen und/oder Trifluormethyl ein- oder mehrfach substituiertes Phenyl oder Naphthyl, ein anderer Wasserstoff oder im Falle von $R_1$ oder $R_2$ Hydroxy und der verbleibende Wasserstoff bedeutet, $R_1$' Wasserstoff ist, $R_2$' Wasserstoff, Hydroxy oder gemeinsam mit $R_2$ Oxo darstellt, $R_4$ und $R_5$ Wasserstoff bedeuten oder $R_4$ einen unsubstituierten oder im Phenylteil durch $C_1$-$C_4$-Alkyl, $C_1$-$C_4$-Alkoxy, Halogen und/oder Trifluormethyl ein- oder mehrfach substituierten Phenyl-$C_1$-$C_4$-alkylrest bedeutet und $R_5$ für Wasserstoff, $C_1$-$C_4$-Alkyl oder eine Gruppe $R_4$ steht und R $C_2$-$C_7$-Alkyl, $\alpha,\alpha$-Di-$C_1$-$C_4$-alkoxy-$C_2$-$C_7$-alkyl, $\alpha,\alpha$-Dihalogen-$C_2$-$C_7$-alkyl, $C_3$-$C_6$-Cycloalkyl, $C_3$-$C_6$-Cycloalkyl-$C_1$-$C_4$-alkyl, $C_3$-$C_8$-Cycloalkenyl-$C_1$-$C_4$-alkyl, wie a-($C_3$-$C_6$-Cycloalkenyl)-$C_1$-$C_4$-alkyl, z.B. Cyclohex-3-enyl, Mono-, Di- oder Trihydroxy-$C_3$-$C_8$-cycloalkyl-$C_1$-$C_4$-alkyl, wie a-(Mono-, Di- oder Trihydroxy-$C_3$-$C_6$-cycloalkyl)-$C_1$-$C_4$-alkyl, z.B. 3,4-Dihydroxycyclohexylmethyl oder 3,4,5-Trihydroxycyclohexylmethyl, Benzyl oder, sofern $R_2$ Halogenphenyl und $R_1$, $R_1$', $R_2$' sowie $R_3$ Wasserstoff darstellen, Methyl bedeutet, und ihre pharmazeutisch verwendbaren Salze als Antiepileptika bzw. zur Herstellung eines Antiepileptikums zur Behandlung von Epilepsien des "petit mal"-Formenkreises, diese enthaltende antiepileptische Arzneimittel bzw. ein Verfahren zur Herstellung solcher Arzneimittel sowie Verbindungen der Formel I, worin R $C_3$-$C_7$-Alkyl, wie Propyl, Isopropyl, Butyl,Isobutyl oder Pentyl, $\alpha,\alpha$-Di-$C_1$-$C_4$-alkoxy-$C_1$-$C_4$-alkyl, insbesondere $\alpha,\alpha$-Di-$C_1$-$C_4$-alkoxymethyl, wie Dimethoxy- oder Diäthoxymethyl, $C_3$-$C_6$-Cycloalkyl-$C_1$-$C_4$-alkyl, wie Cyclopropyl- oder Cyclohexylmethyl, $C_3$-$C_6$-Cycloalkenyl-$C_1$-$C_4$-alkyl, wie a-($C_3$-$C_6$-Cycloalkenyl)-$C_1$-$C_4$-alkyl, z.B. Cyclohex-3-enyl, Mono-, Di- oder Trihydroxy-$C_3$-$C_6$-cycloalkyl-$C_1$-$C_4$-alkyl, wie a-(Mono-, Di- oder Trihydroxy-$C_3$-$C_6$-cycloalkyl)-$C_1$-$C_4$-alkyl, z.B. 3,4-Dihydroxycyclohexylmethyl oder 3,4,5-Trihydroxycyclohexylmethyl, oder unsubstituiertes oder durch $C_1$-$C_4$-Alkyl, wie Methyl, $C_1$-$C_4$-Alkoxy, wie Methoxy, Hydroxy und/oder Halogen, wie Fluor, Chlor oder Jod, mono-, di- oder trisubstituiertes Benzyl bedeutet, $R_2$ Wasserstoff oder Hydroxy darstellt, $R_1$, $R_1'$, $R_2'$ und $R_3$ Wasserstoff bedeuten, $R_4$ durch $C_1$-$C_4$-Alkyl, wie Methyl, $C_1$-$C_4$-Alkoxy, wie Methoxy, und/oder Halogen, wie Fluor, Chlor oder Jod, mono-, di- oder trisubstituiertes Phenyl- oder Diphenyl-$C_1$-$C_4$-alkyl, wie Benzyl, 1-Phenyläthyl oder 2-Phenylprop-2-yl, oder unsubstituiertes oder durch Halogen der Atomnummer bis und mit 35, wie Chlor, monosubstituiertes Naphthyl-$C_1$-$C_4$-alkyl, wie 1-oder 2-Naphthylmethyl, Thienyl-$C_1$-$C_4$-alkyl, wie Thienylmethyl, Furyl-$C_1$-$C_4$-alkyl, wie Furylmethyl, oder Pyridyl-$C_1$-$C_4$-alkyl, wie Pyridylmethyl, darstellt und $R_5$ Wasserstoff, $C_1$-$C_4$-Alkyl, wie Methyl, oder eine Gruppe $R_4$ bedeutet, und ihre Salze, insbesondere ihre pharmazeutisch verwendbaren Salze als solche.

Die Erfindung betrifft vor allem die Verwendung beispielsweise von Verbindungen der Formel I, worin

a) $R_1$, $R_1'$, $R_2'$, $R_3$, $R_4$ und $R_5$ für Wasserstoff stehen, $R_2$ $C_1$-$C_4$-Alkyl, $C_3$-$C_6$-Cycloalkyl, Benzyl, Phenyl, Halogenphenyl oder Naphthyl und $R_2'$ Wasserstoff oder Hydroxy oder $R_2$ Hydroxy und $R_2'$ Wasserstoff bzw. $R_2$ und $R_2'$ gemeinsam eine Oxogruppe bedeuten, oder

b) $R_1$ für Hydroxy, $C_1$-$C_4$-Alkyl, $C_3$-$C_6$-Cycloalkyl, Benzyl, Phenyl, Halogenphenyl oder Naphthyl steht und $R_1'$, $R_2$, $R_2'$, $R_3$, $R_4$ und $R_5$ Wasserstoff bedeuten oder

c) $R_1$ für Halogen steht wobei $R_1'$ Wasserstoff oder Halogen und $R_2$, $R_2'$, $R_3$, $R_4$ und $R_5$ Wasserstoff bedeuten und

R $C_2$-$C_7$-Alkyl, $\alpha,\alpha$-Di-$C_1$-$C_4$-alkoxy-$C_2$-$C_7$-alkyl, $\alpha,\alpha$-Dihalogen-$C_2$-$C_7$-alkyl, $C_3$-$C_6$-Cycloalkyl, $C_3$-$C_6$-Cycloalkyl-$C_1$-$C_4$-alkyl, Benzyl oder, sofern $R_2$ Halogenphenyl oder Naphthyl und $R_1$, $R_1'$, $R_2'$ sowie $R_3$ Wasserstoff darstellen, Methyl bedeutet, und ihre pharmazeutisch verwendbaren Salze und ihrer pharmazeutisch verwendbaren Salze als Antiepileptika bzw. zur Herstellung eines Antiepileptikums zur Behandlung von Epilepsien des "petit mal"-Formenkreises, diese enthaltende antiepileptische Arzneimittel bzw. ein Verfahren zur Herstellung solcher Arzneimittel sowie Verbindungen der Formel I, worin $R_1$, $R_1'$, $R_2'$ und $R_3$ Wasserstoff bedeuten, $R_2$ Wasserstoff oder Hydroxy darstellt, $R_4$ durch $C_1$-$C_4$-Alkyl, wie Methyl, oder $C_1$-$C_4$-Alkoxy, wie Methoxy, monosubstituiertes oder durch Halogen der Atomnummer bis und mit 35, wie Chlor, mono- oder disubstituiertes Phenyl-$C_1$-$C_4$-alkyl, wie Benzyl, 1-Phenyläthyl oder 2-Phenylprop-2-yl, oder unsubstituiertes oder durch Halogen der Atomnummer bis und mit 35, wie Chlor, monosubstituiertes Naphthyl-, Thienyl-, Furyl- oder Pyridyl-$C_1$-$C_4$-alkyl, wie 1- oder 2-Naphthyl-, Thienyl-, Furyl- oder Pyridylmethyl, darstellt, $R_5$ Wasserstoff, $C_1$-$C_4$-Alkyl, wie Methyl, oder eine Gruppe $R_4$ bedeutet und R $C_3$-$C_7$-Alkyl, wie Propyl, Isopropyl, Butyl, Isobutyl oder Pentyl, $\alpha,\alpha$-Di-$C_1$-$C_4$-alkoxy-$C_1$-$C_4$-alkyl, insbesondere $\alpha,\alpha$-Di-$C_1$-$C_4$-alkoxymethyl, wie Dimethoxy- oder Diäthoxymethyl, $C_3$-$C_6$-Cycloalkyl-$C_1$-$C_4$-alkyl, wie Cyclopropyl- oder Cyclohexylmethyl, oder unsubstituiertes oder durch $C_1$-$C_4$-Alkyl, wie Methyl, $C_1$-$C_4$-Alkoxy, wie Methoxy, oder Halogen der Atomnummer bis und mit 35, wie Chlor, substituiertes Benzyl bedeutet, und ihre Salze, insbesondere ihre pharmazeutisch verwendbaren Salze als solche.

Die Erfindung betrifft insbesondere die Verwendung von Verbindungen der Formel I, worin $R_1$, $R_1'$, $R_2'$ und $R_3$ Wasserstoff bedeuten, $R_2$ für Wasserstoff oder Hydroxy steht, $R_4$ und $R_5$ Wasserstoff darstellen oder $R_4$ einen unsubstituierten oder im Phenylteil durch $C_1$-$C_4$-Alkyl, wie Methyl, $C_1$-$C_4$-Alkoxy, wie Methoxy, Halogen, wie Chlor, und/oder Trifluormethyl ein- oder mehrfach substituierten Phenyl-$C_1$-$C_4$-alkyl-, wie Benzylrest bedeutet und $R_5$ für Wasserstoff oder $C_1$-$C_4$-Alkyl, wie Methyl oder Äthyl, steht und R $C_2$-$C_7$-Alkyl, wie Butyl, $\alpha,\alpha$-Di-$C_1$-$C_4$-alkoxy-$C_1$-$C_4$-alkyl, wie Diäthoxymethyl, $\alpha,\alpha$-Dihalogen-$C_2$-$C_7$-alkyl, wie 1,1,-Difluorbutyl, $C_3$-$C_6$-Cycloalkyl, wie Cyclohexyl, $C_3$-$C_6$-Cycloalkyl-$C_1$-$C_4$-alkyl, wie Cyclohexylmethyl, a-($C_3$-$C_6$-Cycloalkenyl)$C_1$-$C_4$-alkyl, z.B. Cyclohex-3-enyl, a-(Mono-, Di- oder Trihydroxy-$C_3$-$C_6$-cycloalkyl)-$C_1$-$C_4$-alkyl, z.B. 3,4-Dihydroxycyclohexylmethyl oder 3,4,5-Trihydroxycyclohexylmethyl, oder einen unsubstituierten oder im Phenylteil durch $C_1$-$C_4$-Alkyl, wie Methyl, $C_1$-$C_4$-Alkoxy, wie Methoxy, Halogen, wie Chlor, und/oder Trifluormethyl ein- oder mehrfach substituierten Phenyl-$C_1$-$C_4$-alkyl-, wie Benzyl- oder 1-Phenyläthylrest bedeutet, und ihrer pharmazeutisch verwendbaren Salze als Antiepileptika bzw. zur Herstellung eines Antiepileptikums zur Behandlung von Epilepsien des "petit mal"-Formenkreises, diese enthaltende antiepileptische Arzneimittel bzw. ein Verfahren zur Herstellung solcher Arzneimittel sowie Verbindungen der Formel I, worin $R_1$, $R_1'$, $R_2'$ und $R_3$ Wasserstoff bedeuten, $R_2$ Wasserstoff oder Hydroxy darstellt, $R_4$ durch Halogen der Atomnummer bis und mit 35, wie Chlor, mono - oder disubstituiertes $\alpha$-Phenyl-$C_1$-$C_4$-alkyl, wie Benzyl, 1-Phenyläthyl oder 2-Phenylprop-2-yl, oder unsubstituiertes $\alpha$-Naphthyl-$C_1$-$C_4$-alkyl, wie

1- oder 2-Naphthylmethyl, α-Thienyl-$C_1$-$C_4$-alkyl, wie Thienylmethyl, α-Furyl-$C_1$-$C_4$-alkyl, wie Furylmethyl, oder α-Pyridyl-$C_1$-$C_4$-alkyl, wie Pyridylmethyl, darstellt, $R_5$ Wasserstoff, $C_1$-$C_4$-Alkyl, wie Methyl, oder eine Gruppe $R_4$ bedeutet und R $C_3$-$C_5$-Alkyl, wie Butyl, α,α-Di-$C_1$-$C_4$-alkoxymethyl, wie Diäthoxymethyl, $C_3$-$C_6$-Cycloalkyl-$C_1$-$C_4$-alkyl, wie Cyclopropyl- oder Cyclohexylmethyl, α-($C_3$-$C_6$-Cycloalkenyl)-$C_1$-$C_4$-alkyl, z.B. Cyclohex-3-enyl, a-(Mono-, Di- oder Trihydroxy-$C_3$-$C_6$-cycloalkyl)-$C_1$-$C_4$-alkyl, z.B. 3,4-Dihydroxycyclohexylmethyl oder 3,4,5-Trihydroxycyclohexylmethyl, oder Benzyl bedeutet, und ihre Salze, insbesondere ihre pharmazeutisch verwendbaren Salze als solche.

Die Erfindung betrifft insbesondere beispielsweise die Verwendung von Verbindungen der Formel I, worin $R_1$, $R_1'$, $R_2'$, $R_3$, $R_4$ und $R_5$ Wasserstoff bedeuten, $R_2$ für Wasserstoff oder Hydroxy steht und R $C_2$-$C_7$-Alkyl, α,α-Di-$C_1$-$C_4$-alkoxy-$C_1$-$C_4$-alkyl, wie Diäthoxymethyl, α,α-Dihalogen-$C_2$-$C_7$-alkyl, wie 1,1-Difluorbutyl, $C_3$-$C_6$-Cycloalkyl, wie Cyclohexyl, $C_3$-$C_6$-Cycloalkyl-$C_1$-$C_4$-alkyl, wie Cyclohexylmethyl, oder Benzyl bedeutet, und ihrer pharmazeutisch verwendbaren Salze als Antiepileptika bzw. zur Herstellung eines Antiepileptikums zur Behandlung von Epilepsien des "petit mal"-Formenkreises, diese enthaltende antiepileptische Arzneimittel bzw. ein Verfahren zur Herstellung solcher Arzneimittel sowie Verbindungen der Formel I, worin $R_1$, $R_1'$, $R_2'$ und $R_3$ Wasserstoff bedeuten, $R_2$ Wasserstoff oder Hydroxy darstellt, $R_4$ durch Halogen der Atomnummer bis und mit 35, wie Chlor, mono- oder disubstituiertes Phenyl-$C_1$-$C_4$-alkyl, wie Benzyl, 1-Phenyläthyl oder 2-Phenylprop-2-yl, oder unsubstituiertes Naphthyl-, Thienyl-, Furyl- oder Pyridyl-$C_1$-$C_4$-alkyl, wie 1- oder 2-Naphthyl-, Thienyl-, Furyl- oder Pyridylmethyl, darstellt, $R_5$ Wasserstoff, $C_1$-$C_4$-Alkyl, wie Methyl, oder eine Gruppe $R_4$ bedeutet und R $C_3$-$C_5$-Alkyl, wie Butyl, α,α-Di-$C_1$-$C_4$-alkoxymethyl, wie Diäthoxymethyl, $C_3$-$C_6$-Cycloalkyl-$C_1$-$C_4$-alkyl, wie Cyclopropyl- oder Cyclohexylmethyl, oder Benzyl bedeutet, und ihre Salze, insbesondere ihre pharmazeutisch verwendbaren Salze, als solche.

Die Erfindung betrifft namentlich die Verwendung der nachstehend aufgeführten bekannten Verbindungen

3-Aminopropyl(cyclohexylmethyl)phosphinsäure,
3-Aminopropyl(n-butyl)phosphinsäure,
3-Aminopropyl(diäthoxymethyl)phosphinsäure,
3-Aminopropyl(benzyl)phosphinsäure,
3-Aminopropyl(1,1-difluorbutyl)phosphinsäure,
3-Amino-2-(p-chlorphenyl)-propyl(methyl)phosphinsäure
3-Amino-2-hydroxy-propyl(cyclohexylmethyl)phosphinsäure,
3-Amino-2(S)-hydroxy-propyl(cyclohexylmethyl)phosphinsäure,
3-Amino-2(R)-hydroxy-propyl(cyclohexylmethyl)phosphinsäure und
3-Amino-2(S)-hydroxy-propyl(benzyl)phosphinsäure

und ihrer pharmazeutisch verwendbaren Salze als Antiepileptika bzw. zur Herstellung eines Antiepileptikums zur Behandlung von Epilepsien des "petit mal"-Formenkreises, diese enthaltende antiepileptische Arzneimittel bzw. ein Verfahren zur Herstellung solcher Arzneimittel sowie ein durch Verabreichung eines solchen gekennzeichnetes Verfahren zur Behandlung epileptischer Erkrankungen des "petit mal"-Formenkreises sowie die in den Herstellungsbeispielen genannten Verbindungen, namentlich

3-(p-Chlorbenzylamino)propyl(diäthoxymethyl)phosphinsäure,
3-(p-Chlorbenzylamino)-2(S)-hydroxy-propyl(benzyl)phosphinsäure,
3-(p-Chlorbenzylamino)-2-hydroxy-propyl(n-butyl)phosphinsäure,
3-(3,4-Dichlorbenzylamino)-2(S)-hydroxy-propyl(benzyl)phosphinsäure,
3-(3,4-Dichlorbenzylamino)-2(S)-hydroxy-propyl(diäthoxymethyl)phosphinsäure und
3-(p-Chlorbenzylamino)propyl(cyclohexylmethyl)phosphinsäure

und ihrer pharmazeutisch verwendbaren Salze als solche.

Das Verfahren zur Herstellung der erfindungsgemäss bereitgestellten neuen, araliphatisch N-substituierte Aminoalkanphosphinsäuren der Formel I ist dadurch gekennzeichnet, dass man in einer Verbindung der Formel II

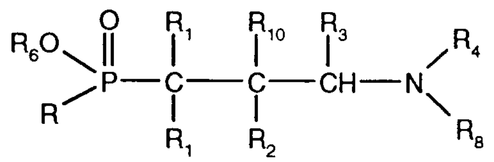

(II),

worin $R_1'$, $R_2'$ und $R_3$ Wasserstoff bedeuten, $R_6$ für eine Hydroxyschutzgruppe, $R_8$ für eine Gruppe $R_5$ oder für eine Aminoschutzgruppe und $R_10$ für Wasserstoff oder geschütztes Hydroxy steht, wobei R, $R_1$, $R_4$ und

$R_5$ die angegebene Bedeutung haben, oder in einem Salz davon die Hydroxygruppen durch Ersatz der Hydroxyschutzgruppe $R_6$ durch Wasserstoff freisetzt und gegebenenfalls die Aminoschutzgruppe $R_8$ abspaltet und

gegebenefalls die Hydroxygruppen $R_2$ aus der geschützten Hydroxygruppe $R_{10}$ freisetzt und gewünschtenfalls eine erhaltene Verbindung in eine andere Verbindung der Formel I überführt, ein verfahrensgemäss erhältliches Isomerengemisch in die Komponenten auftrennt und das jeweils bevorzugte Isomere abtrennt und/oder eine verfahrensgemäss erhältliche freie Verbindung in ein Salz oder eine verfahrensgemäss erhältliches Salz in die entsprechende freie Verbindung überführt..

Geschützte Hydroxygruppen $R_6O$ sind beispielsweise verätherte Hydroxygruppen, vorzugsweise mit einem aliphatischen, cycloaliphatischen, araliphatischen oder aromatischen Alkohol oder mit einem Silanol verätherte Hydroxygruppen, wie insbesondere Niederalkoxy, Niederalkenyloxy, gegebenenfalls, beispielsweise durch Niederalkyl, Niederalkoxy, Halogen und/oder Nitro substituiertes Phenyloxy oder Phenylalkoxy, wie Benzyloxy, oder Triniederalkylsilyloxy, wie Trimethylsilyloxy, Tributylsilyloxy oder Tertiärbutyl-(dimethyl)silyloxy.

Der Ersatz der Schutzgruppe $R_6$ in den Verbindungen der Formel II durch Wasserstoff kann erfolgen durch Behandlung mit einem geeigneten basischen oder sauren Mittel wie einem Alkalimetallhydroxid, z.B. Natriumhydroxid oder Lithiumhydroxid, einem Alkalimetallhalogenid, insbesondere -bromid oder -jodid wie Lithiumbromid oder Natriumjodid, Thioharnstoff, einem Alkalimetallthiophenolat wie Natriumthiophenolat oder einer protonischen oder Lewis-Säure wie einer Mineralsäure, z.B. Salzsäure oder einem Tri- Niederalkylhalosilan, z.B. Trimethylchlorsilan. Die Austauschreaktion kann in Abwesenheit oder Gegenwart eines Lösungsmittels und erforderlichenfalls unter Erhitzen oder unter Kühlung in einem geschlossenen Gefäss und/oder unter Inertgasatmosphäre durchgeführt werden.

Andererseits kann der Ersatz der $R_6$-Schutzgruppe, z.B. einer Silyl- oder Alkylgruppe, in Verbindungen der Formel II durch Wasserstoff auch durch Behandeln mit einer Säure unter hydrolytischen Bedingungen bewirkt werden, insbesondere mit einer Mineralsäure, wie einer Halogenwasserstoffsäure, z.B. Salzsäure, die in verdünnter oder konzentrierter wässriger Form verwendet wird, oder durch Behandlung mit einem organischen Silylhalogenid, wie Trimethyljodsilan oder -bromid und erforderlichenfalls anschliessende Hydrolyse. Die Reaktion wird vorzugsweise bei erhöhter Temperatur durchgeführt, z.B. durch Halten des Reaktionsgemisches am Rückfluss und gegebenenfalls unter Verwendung eines organischen Verdünnungsmittels in einem geschlossenen Gefäss und/oder unter einer Inertgasatmosphäre. Die Art und Weise des Ersatzes der Schutzgruppe $R_6$ hängt beispielsweise von dem Substituenten R ab, der in der Verbindung der Formel II enthalten ist und bei der Umwandlung der Verbindung II in eine Verbindung der Formel I erhalten bleiben muss. Die genannte Umwandlung kann z.B. erfolgen, wie in den Herstellungsbeispielen beschrieben.

Aminoschutzgruppen $R_8$ in Verbindungen der Formel II können nach bekannten Verfahren, die je nach der Art der Aminoschutzgruppe ausgewählt werden, abgespalten werden, z.B. durch solvolytische oder hydrogenolytische Verfahren, z.B. Hydrolyse in Gegenwart einer Säure oder Base, Acidolyse, z.B. Behandlung mit Trifluoressigsäure, Behandlung mit Hydrazin oder Hydrogenolyse in Gegenwart eines metallischen Hydrierungskatalysators, oder durch irgendein anderes geeignetes Verfahren.

Je nach den beteiligten Gruppen kann der Austausch und die Umwandlung nacheinander oder gleichzeitig nach an sich bekannten Methoden erfolgen.

Vorzugsweise werden alle Schutzgruppen, Hydroxy-Schutzgruppen $R_6$ bzw. $R_{10}$, und Aminoschutzgruppen $R_8$ in einer einzigen Stufe durch Behandlung mit einem Triniederalkylsilylhalogenid, wie Trimethylbromsilan, oder mit einer Säure, vorzugsweise einer Halogenwasserstoffsäure, speziell Salzsäure unter hydrolytischen Bedingungen, durch Wasserstoff ersetzt.

Die Ausgangsstoffe der Formel II können auf verschiedenen Wegen hergestellt werden, beispielsweise indem man

a) in eine Verbindung der Formel III

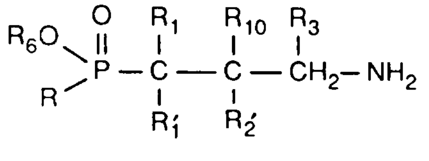

(III),

worin $R_1$, $R_1'$, $R_2'$ und $R_3$ Wasserstoff bedeuten, $R_6$ die angegebene Bedeutung hat und $R_{10}$ für Wasserstoff oder geschütztes Hydroxy steht, die Gruppe $R_4$ und gewünschtenfalls einen von Wasserstoff

verschiedenen Rest $R_5$ einführt oder
b) eine Verbindung der Formel IV

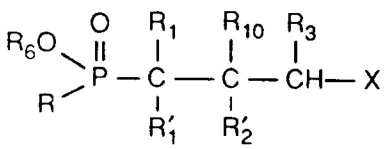

(IV),

worin $R_1$, $R_1'$, $R_2'$ und $R_3$ Wasserstoff bedeuten, X eine reaktionsfähige veresterte Hydroxygruppen bedeutet und $R_{10}$ für Wasserstoff oder geschütztes Hydroxy steht, oder ein Salz davon mit einer Verbindung der Formel

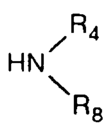

(V),

worin $R_4$ und $R_8$ die angegebenen Bedeutungen haben, umsetzt oder
c) eine Verbindung der Formel VI

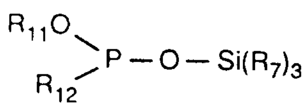

(VI),

worin $R_{11}$ eine Gruppe $R_6$ oder $-Si(R_7)_3$, $R_{12}$ einen an einer gegebenenfalls vorhandenen Hydroxygruppe durch eine Gruppe $-Si(R_7)_3$ geschützten Rest R und die Reste $R_7$ gleiche oder verschiedene aliphatische Kohlenwasserstoffreste, beispielsweise Niederalkyl, insbesondere Methyl und/oder Tertiärbutyl, bedeuten, mit einer Verbindung der Formel VII

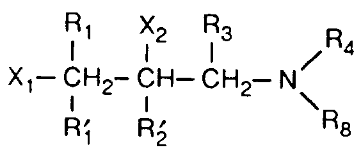

(VII),

worin $R_1$, $R_1'$, $R_2'$ und $R_3$ Wasserstoff bedeuten, $X_1$ reaktionsfähige verestertes Hydroxy und $X_2$ Wasserstoff bedeutet oder $X_1$ und $X_2$ gemeinsam für Epoxy stehen und $R_8$ eine Gruppe $R_5$ oder eine Aminoschutzgruppe bedeutet, kondensiert oder
d) eine Verbindung der Formel VIII

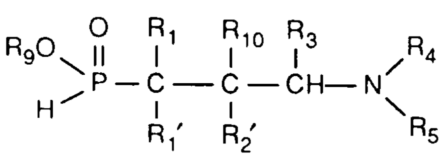

(VIII),

worin $R_1$, $R_1'$, $R_2'$ und $R_3$ Wasserstoff bedeuten, $R_9$ Wasserstoff oder eine Gruppe $R_6$ darstellt und $R_{10}$ für Wasserstoff oder geschütztes Hydroxy steht, mit einem Silylierungsmittel umsetzt und die erhaltene silylaktivierte Verbindung der Formel IX

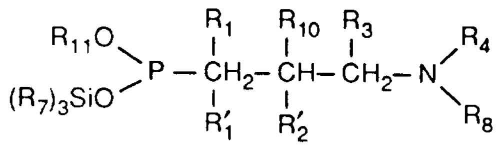

(IX),

in der $R_8$ für eine von Wasserstoff verschiedene Gruppe $R_5$ oder eine Gruppe der Formel $-Si(R_7)_3$ steht, $R_{11}$ eine Gruppe $R_6$ oder eine Gruppe $-Si(R_7)_3$ bedeutet und $R_{10}$ Wasserstoff oder eine Gruppe der Formel $-OSi(R_7)_3$ darstellt, wobei die Reste $R_7$ gleiche oder verschiedene aliphatische Kohlenwasserstoffreste, beispielsweise Niederalkyl, insbesondere Methyl und/oder Tertiärbutyl, bedeuten, mit einem reaktionsfähigen Ester eines aliphatischen, cycloaliphaitischen, cyloaliphatisch-aliphatischen oder araliphatischen Alkohols, mit einem in $\alpha,\beta$-Stellung eine gegebenenfalls zusätzlich Doppelbindung aufweisenden aliphatischen, cycloaliphatischen, cyloaliphatisch-aliphatischen, araliphatischen oder heteroarylaliphatischen Kohlenwasserstoff, mit einem aliphatischen, cycloaliphatischen, cyloaliphatisch-aliphatischen, araliphatischen oder heteroarylaliphatischen Aldehyd oder Keton oder mit einem aliphatischen Epoxid umsetzt oder

e) zur Herstellung einer Verbindung der Formel II, in der $R_2$ Hydroxy bedeutet, eine Verbindung der Formel X

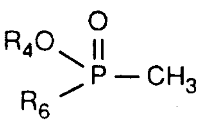

(X)

in der Form eines Metallsalzes der Formel XI

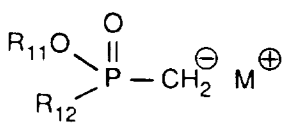

(XI),

worin $R_{11}$ eine Gruppe $R_6$ oder $-Si(R_7)_3$ und $R_{12}$ einen an einer gegebenenfalls vorhandenen Hydroxygruppe durch eine Gruppe $-Si(R_7)_3$ geschützten Rest R bedeutet, in dem die Reste $R_7$ gleiche oder verschiedene aliphatische Kohlenwasserstoffreste, beispielsweise Niederalkyl, insbesondere Methyl und/oder Tertiärbutyl, bedeuten, und $M^+$ ein Alkali-, Erdalkali- oder Übergangsmetall-Kation darstellt, mit einem Aldehyd der Formel XII

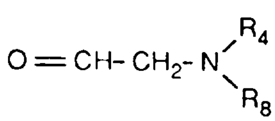

(XII)

umsetzt.

Gewünschtenfalls kann man jeweils in eine primär erhaltene Verbindung der Formel II, worin $R_5$ Wasserstoff darstellt, einen von Wasserstoff verschiedenen Rest $R_5$ einführen.

Die Einführung des Restes $R_4$ und gegebenenenfalls des Restes $R_5$ gemäss der Verfahrensvariante a) erfolgt in üblicher Weise, beispielsweise durch Umsetzung mit einer Verbindung der Formel $X-R_2$ (IIIa), worin X eine reaktionsfähige veresterte Hydroxygruppen bedeutet, insbesondere in Gegenwart eines basischen Kondensationsmittels, wie einer tertiären organischen Base, beispielsweise eines Triniederalkylamin, z.B. von Triäthylamin, Triisopropylamin oder Tertiärbutyl(dimethyl)amin, oder von Pyridyl, oder einer quaternären organischen Ammoniumbase, z.B. von Benzyl(trimethyl)ammoniumhydroxid. Als reaktionsfähige Hydroxygruppen kommen dabei vorzugsweise mit einer Mineralsäure veresterte Hydroxygruppen, wie Halogen, insbesondere Brom, Chlor oder Jod, oder Gruppen der Formel $R_4-O-SO_4-O-$ in Betracht.

Der Rest $R_4$ kann aber auch durch Umsetzung mit einer Verbindung der Formel $O=R_4''$ (IIIb), worin $R_4''$ für einen zweiwertigen araliphatischen oder heteroarylaliphatischen Rest steht, dessen freie Valenzen vom gleichen C-Atom ausgehen, unter reduktiven Bedingungen, insbesondere in Gegenwart eines Alkalimetallborhydrides, z.B. von Natriumcyanoborhydrid vorzugsweise in einem Niederalkanol, wie Äthanol, Methanol oder Butanol, erfolgen.

Die Kondensation von Verbindung der Formel IV mit Aminen der Formel V gemäss der Verfahrensvariante b) erfolgt in analoger Weise wie vorstehend für die Umsetzung von Verbindungen der Formeln III beschrieben.

In Verbindungen der Formel VII gemäss der Verfahrensvariante c) bedeutet reaktionsfähiges verestertes Hydroxy vorzugsweise Halogen, wie Brom, Jod oder Chlor, oder Sulfonyloxy, wie Niederalkansulfonyloxy, z.B. Methansulfonyloxy, oder gegebenenfalls substituiertes Benzolsulfonyloxy, z.B. Benzol-, p-Toluol- (oder p-Brombenzolsulfonyloxy. Aminoschutzgruppen sind insbesondere Silylgruppen, beispielsweise der Formel $-Si(R_7)_3$, wie Triniederalkylsilyl, z.B. Trimethylsilyl. Die Umsetzung von Verbindungen der Formeln VI und reaktionsfähigen Estern VII kann in an sich bekannter Weise erfolgen, vorzugsweise unter den Bedingungen der Arbusow-Reaktion, vorteilhaft im Temperaturbereich von etwa 60° C bis etwa 180° C, z.B. bei etwa 120° C bis etwa 160° C. Bei der Umsetzung von Verbindungen der Formel VI mit Epoxiden (VII; $X_1 + X_2$ = Epoxy) arbeitet man hingegen vorzugsweise in Gegenwart einer milden Lewissäure, insbesondere von Zinkchlorid, vorteilhaft in einem aprotischen Lösungmittels.

Gemäss der Verfahrensvariante d) verwendbare Silylierungsmittel sind insbesondere Triniederalkylhalogensilane der Formel $(R_7)_3Si$-Hal (VIIIa), worin $R_7$ Niederalkyl und Hal Halogen, wie Chlor, Brom oder Jod, bedeutet, wie Trimethylchlorsilan oder -bromid, oder Hexaniederalkyldisilazane der Formel $(R_7)_3Si-NH-Si-(R_7)_3$ (VIIIb), worin $R_7$ Niederalkyl bedeutet, wie Hexamethyldisilazan. Das silylaktivierte Zwischenprodukte ist vorzugsweise eine Verbindung der Formel IXa

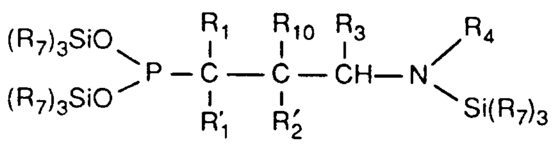

(IXa).

Die Umsetzung des Zwischenproduktes der Formel IX beziehungsweise IXa mit der den Rest R einführenden Komponente erfolgt vorzugsweise in Gegenwart eines basischen Kondensationsmittels, wie einer tertiären organischen Base, beispielsweise eines Triniederalkylamin, z.B. von Triäthylamin, Triisopropylamin oder Tertiärbutyl(dimethyl)amin, oder von Pyridin, oder einer quaternären organischen Ammoniumbase, z.B. von Benzyl(trimethyl)ammoniumhydroxid.

In Ausgangsstoffen der Formel X für die Verfahrensvariante e) sind Übergangsmetall-Kationen beispielsweise Lithium-, Natrium- oder Kalium-Kationen oder Gruppen der Formel -Mg-Hal oder -Zn-Hal, wobei Hal für Chlor, Brom oder Jod steht. Die Kondensation von Verbindungen der Formeln XI und XII erfolgt in der für derartige metalloraninischen Reaktionen üblichen Weise.

Die Einführung eines von Wasserstoff verschiedenen Restes $R_5$ erfolgt in üblicher Weise, insbesondere wie unter der Verfahrensvariante a) angegebenen Weise.

Erhaltene Salze können in an sich bekannter Weise in die freien Verbindungen umgewandelt werden, z.B. durch Behandeln mit einer Base, wie einem Alkalimetallhydroxid, einem Metallcarbonat oder -hydrogencarbonat, oder Ammoniak, oder einer anderen eingangs genannten salzbildenden Base bzw. mit einer Säure, wie einer Mineralsäure, z.B. mit Chlorwasserstoff, oder einer anderen eingangs genannten salzbildenden Säure.

Erhaltene Salze können in an sich bekannter Weise in andere Salze überführt werden, Säureadditionssalze z.B. durch Behandeln mit einem geeigneten Metallsalz, wie einem Natrium-, Barium- oder Silbersalz, einer anderen Säure in einem geeigneten Lösungsmittel, in welchem ein sich bildendes anorganisches Salz unlöslich ist und damit aus dem Reaktionsgleichgewicht ausscheidet, und Basesalze durch Freisetzung der freien Säure und erneute Versalzung.

Die Verbindungen der Formel I, einschliesslich ihrer Salze, können auch in Form von Hydraten erhalten werden oder das zur Kristallisation verwendete Lösungsmittel einschliessen.

Infolge der engen Beziehung zwischen den neuen Verbindungen in freier Form und in Form ihrer Salze sind vorstehend und nachfolgend unter den freien Verbindungen und ihren Salzen sinn- und zweckgemäss gegebenenfalls auch die entsprechenden Salze bzw. freien Verbindungen zu verstehen.

Erhaltene Diastereomerengemische und Racematgemische können auf Grund der physikalisch-chemischen Unterschiede der Bestandteile in bekannter Weise in die reinen Diastereomeren bzw. Racemate aufgetrennt werden, beispielsweise durch Chromatographie und/oder fraktionierte Kristallisation.

Erhaltene Racemate lassen sich ferner nach bekannten Methoden in die optischen Antipoden zerlegen, beispielsweise durch Umkristallisation aus einem optisch aktiven Lösungsmittel, mit Hilfe von Mikroorganismen oder durch Umsetzung des erhaltenen Diastereomerengemisches bzw. Racemates mit einer optisch aktiven Hilfsverbindung, z.B. entsprechend der in Verbindungen der Formel I enthaltenen sauren, basischen oder funktionell abwandelbaren Gruppen mit einer optisch aktiven Säure, Base oder einem optisch aktiven Alkohol, in Gemische diastereomerer Salze bzw. funktioneller Derivate, wie Ester, Trennung derselben in die Diastereomeren, aus denen das jeweils gewünschte Enantiomere in der jeweils üblichen Weise freigesetzt werden kann. Dafür geeignete Basen. Säuren bzw. Alkohole sind beispielsweise optisch aktive Alkaloidbasen, wie Strychnin, Cinchonin oder Brucin, oder D- oder L-(1-Phenyl)äthylamin, 3-Pipecolin, Ephedrin, Amphetamin und ähnliche synthetisch zugängliche Basen, optisch aktive Carbon- oder Sulfonsäuren, wie Chinasäure oder D- oder L-Weinsäure, D- oder L-Di-o-toluylweinsäure, D- oder L-Äpfelsäure, D- oder L-Mandelsäure, oder D- oder L-Camphersulfonsäure, bzw. optisch aktive Alkohole, wie Borneol oder D- oder L-(1-Phenyl)äthanol.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Salzes verwendet oder insbesondere unter den Reaktionsbedingungen bildet.

Die neuen Ausgangsstoffe, die speziell für die Herstellung der erfindungsgemässen Verbindungen entwickelt wurden, insbesondere die zu den eingangs als bevorzugt gekennzeicheten Verbindungen der Formel I führende Ausgangsstoffauswahl, die Verfahren zu ihrer Herstellung und ihre Verwendung als Zwischenprodukte bilden ebenfalls einen Gegenstand der Erfindung.

Verbindungen der Formel I, worin $R_4$ und $R_5$ Wasserstoff bedeutet, welche auch als Ausgangsstoffe für die Verfahrensvariante a) dienen, können beispielsweise hergestellt werden, indem man eine an der gegebenenfalls vorhandenen Hydroxygruppe geschützte Verbindung der Formel XIII

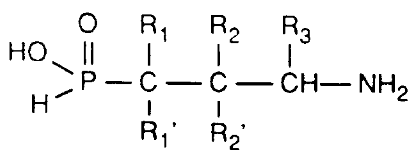

(XIII),

worin $R_1$, $R_1'$, $R_2$, $R_2'$ und $R_3$ die angegebene Bedeutung haben, mit einem Triniederalkylhalogensilan, wie Trimethylchlorsilan, oder einem Hexaniederalkyldisilazan, z.B. mit Hexamethyldisilazan, umsetzt und die erhaltene silylaktivierte Verbindung der Formel XIV

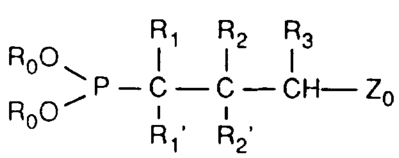

(XIV),

worin $R_0$ eine Triniederalkylsilylgruppe, wie Trimethylsilyl, und $Z_0$ N,N-Di(triniederalkylsilyl)amino darstellt, mit einem entsprechenden aliphatischen, cycloaliphatisch-aliphatischen oder araliphatischen Halogenid der Formel R-Hal (XV; Hal = Halogen)oder einem aliphatischen, cycloaliphatisch-aliphatischen oder araliphatischen Aldehyd der Formel R'-CH = O (XVI), worin R' einen um eine terminale CH2-Gruppe verminderten Rest R bedeutet, zur entsprechenden Verbindung der Formel XVII

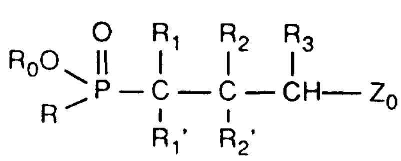

(XVII)

kondensiert, worin R einen aliphatischen, cycloaliphatisch-aliphatischen, oder araliphatischen Rest oder eine Gruppe der Formel R-CH(OH)- darstellt, bzw. die Verbindung der Formel XIV mit einem aliphatischen, cycloaliphatischen, cycloaliphatisch-aliphatischen oder araliphatischen Keton der Formel R''-C(=O)-R''' (XVIII), worin R'' eine aliphatischen, cycloaliphatischen oder aromatischen Rest und R''' einen aliphatischen Rest darstellt, zur entsprechenden Verbindung der Formel V, worin R einen Rest der Formel R''-C(R''')(OH)-bedeutet, oder mit einer entsprechenden aliphatischen, cycloaliphatisch-aliphatischen oder araliphatischen Epoxid oder einem entsprechenden, mindestens eine zusätzliche olefinische Doppelbindung aufweisenden aliphatischen, cycloaliphatischen, cycloaliphatisch-aliphatischen oder araliphatischen Verbindung weiterumsetzt und jeweils das Primärprodukt, beispielsweise der Formel XVII, hydrolytisch aufarbeitet.

Verbindungen der Formel I, worin $R_1$ und $R_1$' Wasserstoff, $R_2$ einen aromatischen Rest, $R_2$' Hydroxy und $R_3$, $R_4$ und $R_5$ Wasserstoff darstellen, welche ebenfalls als Ausgangstoffe für die Verfahrensvariante a) dienen, können insbesondere hergestellt werden, indem man Verbindungen der Formeln XIX und XX

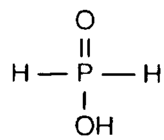

(XIX)

und

$HO\text{-}CH_2 \bullet C{\equiv}CH$ (XX)

in einem araliphatischen Lösungsmittel, wie Toluol, zur entsprechenden Verbindung der Formel XXI

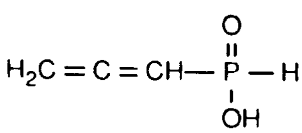

(XXI)

umsetzt und diese durch Umsetzung zunächst mit einem Halogenameisensäureniederalkylester, wie Chlorameisensäureäthylester, in Gegenwart eines Triniederalkylamins, wie Triäthylamin, und nachfolgend mit einem Orthoessigsäuretriniederalkylester, wie Orthoessigsäureträthylester, in Gegenwart einer Lewissäure, wie Bortrifluorid, in eine Verbindung der Formel XXII

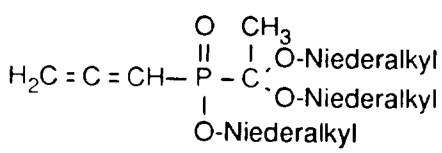

(XXII)

überführt. Diese wird dann in Gegenwart des 1:1-Komplexes aus Kupfer-I-bromid und Dimethylsulfid mit einer Verbindung der Formel $R_2$-M (XXIII), worin M einen metallischen Rest, insbesondere eine Halogenmagnesiumgruppe, bedeutet, zu der entsprechenden Verbindung der Formel XXIV

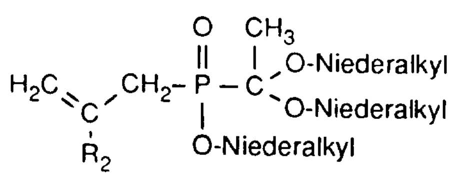

(XXIV)

umgesetzt. In einer Eintopfreaktion kann man dann die P-Schutzgruppen (Niederalkylreste) durch Behandlung mit Trimethylchlorsilan in Dichlormethan/Äthanol abspalten und durch Umsetzung mit einer Verbindung der Formeln XV, XVI oder XVIII, mit einem aliphatischen, cycloaliphatisch-aliphatischen oder araliphatischen Epoxid oder einer entsprechenden aliphatischen, cycloaliphatischen cycloaliphatisch-aliphatischen oder

araliphatischen ungesättigten Verbindung in der für die Umsetzung derselben mit Verbindungen der Formel XIV beschrieben den Rest R einführen. Die die entsprechende Verbindung der Formel XXV

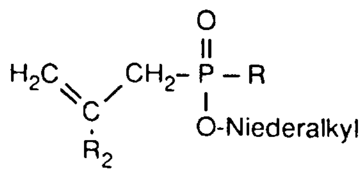

(XXV)

wird dann in einem Acetonitril/Tertiärbutanol/Wasser-Gemisch in Gegenwart von Silbernitrat und Osmiumtetroxid mit einem Reagenz umgesetzt, das man durch Umsetzung von Tertiärbutylurethan mit Tertiärbutoxychlorid in einem Niederalkanol in Gegenwart von Natriumhydroxid erhält. Das so erhaltene Primärprodukt wird schliesslich mit Trimethylbromsilan in Dichlormethan behandelt und ergibt eine Verbindung der Formel XXVI

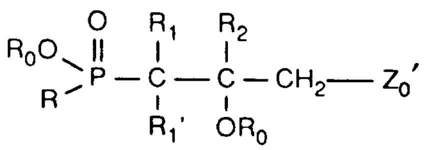

(XXVI),

in der $R_0$ eine Silylgruppe und $Z_0'$ Tertiärbutyloxycarbonyl bedeutet. Behandlung mit wässrigem Methanol und anschliessend mit Propylenoxid ergibt die gewünschte Verbindung der Formel I.

Die neuen Verbindungen der Formel I können z.B. in Form pharmazeutischer Präparate Verwendung finden, welche eine therapeutisch wirksame Menge der Aktivsubstanz, gegebenenfalls zusammen mit anorganischen oder organischen, festen oder flüssigen, pharmazeutisch verwendbaren Trägerstoffen enthalten, die sich zur enteralen, z.B. oralen, oder parenteralen Verabreichung eignen.

Pharmazeutische Präparate, die bekannte $GABA_B$-Antagonisten, z.B. bekannte Verbindungen der Formel I enthalten, sind vorzugsweise bestimmt als Antiepileptika bestimmt.

Rei den erfindungsgemässen pharmazeutischen Präparaten handelt es sich jeweils solche in einer Dosiseinheitsform, welche eine therapeutisch wirksame Menge der Aktivsubstanz, allein oder zusammen mit einem pharmazeutisch anwendbaren Träger, insbesondere mit anorganischen oder organischen, festen oder flüssigen, pharmazeutisch verwendbaren Trägerstoffen enthalten, sodass sie sich zur enteralen, wie oralen, ferner rektalen, und parenteralen Verabreichung an Warmblüter eigen.

Die erfindungsgemäss bereitgestellten pharmazeutischen Präparate, wie Antiepileptika, enthalten z.B. von etwa 10 % bis etwa 80 %, vorzugsweise von etwa 20 % bis etwa 60 %, des Wirksioffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, ferner Injektions- oder Infusionslösungen, vorzugsweise in Ampullen. Diese Formulierungen werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragakanth, Methylcellulose und/oder Polyvinylpyrrolidon, wenn erwünscht, Sprengmittel, wie die obengenannten Stärken, ferner Carboxymethylstärke, quervernetztes Polyvinylpyrrolidon. Agar, Alginsäure oder ein Salz davon, wie Natriumalginat, Hilfsmittel sind in erster Linie Fliess-, Fliessregulier- und Schmiermittel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaftresistenten Überzügen versehen, wobei man u.a. konzentrierte Zuckerlösungen, welche gegebenenfalls arabischen Gummi, Talk, Polyvinylpyrrolidon, Polyäthylenglykol und/oder Titandioxid enthalten, Lacklösungen in geeigneten organischen Lösungsmitteln oder Lösungsmittelgemische oder, zur Herstellung

von Magensaft-resistenten Überzügen, Lösungen von geeigneten Cellulosepräparaten, wie Acetylcellulosephthalat oder Hydroxypropylmethylcellulosephthalat, verwendet. Den Tabletten oder Dragée-Überzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulates, z.B. im Gemisch mit Füllstoffen, wie Lactose, Bindemitteln, wie Stärken, und/oder Gleitmitteln, wie Talk oder Magnesiumstearat, und gegebenenfalls Stabilisatoren, enthalten. In weichen Kapseln ist der Wirkstoff vorzugsweise in geeigneten Flüssigkeiten, wie fetten Ölen, Paraffinöl oder flüssigen Polyäthylenglykol, gelöst oder suspendiert, wobei ebenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetisch Triglyceride, Paraffinkohlenwasserstoffe, Polyäthylenglykol oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride. Polyäthylenglykole oder Paraffinkohlenwasserstoffe in Frage.

Zur parenteralen Verabreichung eignen sich in erster Linie wässrige Lösungen eines Wirkstoffs in wässerlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Öle, z.B. Sesamöl, oder synthetische Fettsäureester, z.B. Äthyloleat oder Triglyceride, verwendet oder wässrige Injektionssuspensionen, welche viskositätserhöhende Stoffe, z.B. Natrium-carboxymethylcellulose, Sorbit und/oder Dextran, und gegebenenfalls auch Stabilisatoren enthalten.

Die pharmazeutischen Präparate können sterilisiert sein und, wenn erwünscht, weitere pharmakologisch wirksame Stoffe und/oder Hilfsstoffe, z.B. Konservierungs-, Stabilisier-, Netz- und/oder Emulgiermittel, Löslichkeitsvermittler, Salze zur Regulierung des osmotischen Druckes und/oder Puffer enthalten.

Die Dosierung kann von verschiedenen Faktoren, wie Applikationsweise, Spezies, Alter und/oder individuellem Zustand abhängen. Die täglich zu verabreichenden Dosen liegen bei oraler Applikation zwischen etwa 5 und etwa 60 mg/kg, insbesondere 10 und etwa 40 mg/kg und für Warmblüter mit einem Körpergewicht von etwa 40 kg vorzugsweise zwischen etwa 200 mg und etwa 2400 mg, insbesondere etwa 400 bis etwa 1600 mg, die zweckmässigerweise auf 2 bis 6, z.B. 3 oder 4 Einzeldosen aufgeteilt wird.

Die nachfolgenden Beispiele dienen zur Illustration der Erfindung; Temperaturen sind in Celsiusgraden, Drucke in mbar angegeben.

Herstellungsbeispiel 1: Zu einer Lösung von 1,61 g 3-(p-Chlorbenzylamino)propyl(diäthoxymethyl)-phosphinsäureäthylester in 3 ml Äthanol fügt man eine Lösung von 0,36 g Lithiumhydroxid-Monohydrat in 7 ml Wasser hinzu und erwärmt 24 Stunden auf 60°. Dann kühlt man auf Raumtemperatur und zieht unter vermindertem Druck das Lösungsmittel ab. Der Eindampfrückstand wird in Wasser aufgenommen und mit Phosphorsäure neutral gestellt. Es bildet sich eine weisser Niederschlag. Dieser wird abfiltriert und das Filtrat zur Trockne eingedampft. Der weisse Eindampfrückstand wird unter vermindertem Druck getrocknet und aus Toluol/Diäthyläther kristallisiert. Nach Absaugen und Trocknen erhält man 3-(p-Chlorbenzylamino)-propyl(diäthoxymethyl)-phosphinsäure vom Smp. 177-179°

Das Ausgangsmaterial kann z.B. folgendermassen hergestellt werden:

Eine Lösung von 2,53 g 3-Aminopropyl(diäthoxymethyl)phosphinsäureäthylester in 10 ml wasserfreiem Methanol wird mit 1,41 g p-Chlorbenzaldehyd versetzt und die erhaltene klare Lösung 30 Minuten bei Raumtemperatur gerührt. Dann fügt man zunächst 0,6 g Eisessig und dann tropfenweise 0,21 g Natriumcyanoborhydrid, gelöst in 5 ml Methanol hinzu. Es setzt eine exotherme Reaktion ein. Man rührt 3 Stunden bei 20°, stellt auf pH 8 ein und zieht das Lösungsmittel ab. Der Rückstand wird in Dichlormethan gelöst und mit Wasser gewaschen. Die organische Phase wird abgetrennt, über Natriumsulfat getrocknet und zur Trockne eingedampft. Das zurückbleihende Öl wird an Kieselgel chromatographisch gereinigt. Man erhält 3-(p-Chlorbenzylamino)propyl(diäthoxymethyl)phosphinsäureäthylester als fahlgelbes Öl.

Herstellungsbeispiel 2: Eine Lösung von 1,80 g 3-(p-Chlorbenzylamino)-2(S)-hydroxypropyl(benzyl)-phosphinsäureäthylester in 30 ml halbkonzentrierter Salzsäure wird zum Rückfluss erhitzt, wobei sich nach 30 Minuten eine eine weissliche Suspension bildet. Man erhitzt weitere 20 Stunden zum Rückfluss, kühlt auf 0°, filtriert den Feststoffanteil ab, wäscht mit Wasser und trocknet unter vermindertem Druck bei 60°. Umkristallisation aus wässrigem Methanol liefert nach dem Trocknen 3-(p-Chlorbenzylamino)-2(S)-hydroxypropyl(benzyl)phosphinsäure-hydrochlorid vom Smp. 211,5-212°.

Das Ausgangsmaterial kann wie folgt hergestellt werden:

Eine Suspension von 13,2 g 99 %-igem Natriumhydrid in 500 ml Tetrahydrofuran wird innerhalb von 90

Minuten unter Argon mit einer Lösung von Diäthoxymethylphosphinsäureäthylester versetzt, wobei die Temperatur bei 20° gehalten wird. Die Reaktion ist exotherm und mit Gasentwicklung verbunden. Man lässt 90 Minuten nachrühren und fügt dann innerhalb von 20 Minuten 85,5 g Benzylbromid hinzu. Man lässt 24 Stunden bei Raumtemperatur nachrühren, kühlt auf 0° und gibt vorsichtig 100 ml Wasser hinzu. Die organische Lösungsmittel werden unter vermindertem Druck abgezogen, der Rückstand wird zwischen Wasser und Dichlormethan verteilt. Die organische Phase wird abgetrennt, mit Wasser gewaschen, über Natriumsulfat getrocknet und eingedampft. Destillation des Rückstandes unter vermindertem Druck ergibt Diäthoxymethyl(benzyl)phosphinsäureäthylester vom Kp = 103-110° ($3x10^{-4}$ bar).

Eine Suspension von 128 g Diäthoxymethyl(benzyl)phosphinsäureäthylester in 400 ml Salzsäure wird 20 Stunden zum Rückfluss erhitzt, auf Raumtemperatur abgekühlt mit Diäthyläther/Hexan (1:1) gewaschen und zur Trockne eingedampft. Der Rückstand wird in Dichlormethan aufgenommen, über Natriumsulfat getrocknet und erneut eingedampft. Die zurückbleibende, viskose Benzylphosphinsäure wird im Hochvakuum nachgetrocknet.

13,34 g Benzylphosphinsäure werden in 150 ml Dichlormethan gelöst, auf 5° gekühlt und tropfenweise mit 8,64 g Träthylamin versetzt. Es erfolgt exotherme Reaktion. Man kühlt erneut auf 5° und fügt innerhalb von 30 Minuten tropfenweise 9,3 g Chlorameisensäureäthylester hinzu. Unter exothermer Reaktion und Gasentwicklung bildet sich ein Niederschlag. Man lässt auf Raumtemperatur erwärmen und 3 Stunden bei Raumtemperatur nachrühren, nimmt in Dichlormethan auf, wäscht mit Wasser und zieht das organische Lösungsmittel ab. Das Rohprodukt wird im Hochvakuum destilliert. Man erhält Benzylphosphinsäureäthylester vom Kp = 96-100° ($5x10^{-6}$ bar).

26,7 g Benzylphosphinsäureäthylester und 16,0 g Triäthylamin werden in 250 ml Tetrahydrofuran gelöst, mit 17,33 g Trimethylchlorsilan versetzt und über Nacht bei Raumtemperatur gerührt. Der gebildete weisse Niederschlag wird unter Argon abfiltriert und das Filtrat unter vermindertem Druck eingedampft. Der Rückstand wird mit 14,71 g (R)-Epichlorhydrin und 2,5 g Zinkchlorid versetzt. Nach Abklingen der exothermen Reaktion wird 7,5 Stunden bei 60° zum Rückfluss erhitzt. Man lässt auf Raumtemperatur abkühlen, verdünnt mit Dichlormethan und wäscht mit Wasser. Die organische Phase wird abgetrennt, über Nariumsulfat getrocknet und eingedampft. Der ölige Rückstand wird mit 1 %-iger methanolischer Essigsäure aufgenommen, 20 Stunden bei Raumtemperatur stehengelassen und eingedampft. Der Rückstand wird an Silicagel chromatographisch gereinigt. Man erhalt 3-Chlor-2(R)-hydroxy-propyl(benzyl)-phosphinsäureäthylester; $^1$H-NMR-Spektrum (in $CDCl_3$): $\delta$ = 7,3 (5H,m); 2,19-3,92 (3H,m); 3,6-3,42 (2H,m); 3,21 (211.d: J= 15,0 Hz); 2,16-1,81 (2H,m); 1,30 (3H,t); $^{31}$P-NMR-Spektrum (in $CDCl_3$): $\delta$ = 52.1.

Eine Mischung von 2.76 g 3-Chlor-2(R)hydroxy-propyl(benzyl)phosphinsäureäthylester, 3.54 g p-Chlorbenzylamin, 3,25 g N-Äthyl-N,N-diisopropylamin und 20 ml Äthanol werden 48 Stunden zum Rückfluss erhitzt. Nach Abziehen des Lösungsmittels und Chromatographie erhält man 3-(p-Chlorbenzylamino)-2(S)-hydroxy-propyl(benzyl)phosphinsäureäthylester; $^1$H-NMR-Spektrum (in $CDCl_3$): $\delta$ = 7,4-7,17 (9H,m); 4,14-3,83 (3H,m); 3,71 (2H,ABq); 3,20 (2H,d); 2,6-2,5 (2H,m); 1,96-1,62 (2H,m); 1,25 (3H,t); $^{31}$P-NMR-Spektrum (in $CDCl_3$): $\delta$ = 53,9; 52,7.

Herstellungsbeispiel 3: Eine Lösung von 1,86 g 3-(p-Chlorbenzylamino)propyl-(n-butyl)-phosphinsäureäthylester in 30 ml halbkonzentrierter Salzsäure wird über Nacht zum Rückfluss erhitzt, wobei sich bereits nach 10 Minuten eine klare Lösung bildet. Anschliessend kühlt man auf 0°, woraufhin sich eine weisser Niederschlag bildet. Dieser wir abfiltriert, mit Wasser gewaschen und unter vermindertem Druck bei 60° getrocknet. Umkristallisation aus Wasser liefert nach dem Trocknen 3-(p-Chlorbenzylamino)-propyl-(n-butyl)phosphinsäure-hydrochlorid vom Smp. 212-214°.

Das Ausgangsmaterial kann wie folgt hergestellt werden:

24 g einer 55 %-igen Suspension von Natriumhydrid in Mineralöl werden unter Argon mit Hexan gewaschen und dann in 100 ml Tetrahydrofuran aufgenommen. Man kühlt auf 0° und gibt unter Argon tropfenweise eine Lösung von 104,4 g Diäthoxymethylphosphinsäureäthylester in 100 ml Tetrahydrofuran hinzu, wobei die Temperatur bei 20° gehalten wird. Die Reaktion ist exotherm und mit Gasentwicklung verbunden. Man lässt 90 Minuten nachrühren und fügt dann bei 20° 209,7 g n-Butylbromid hinzu. Man lässt 2,5 Stunden bei Raumtemperatur nachrühren, kühlt auf 0° und gibt vorsichtig 100 ml Wasser hinzu. Die organische Lösungsmittel werden unter vermindertem Druck abgezogen, der Rückstand wird zwischen Wasser und Dichlormethan verteilt. Die organische Phase wird abgetrennt, mit Wasser gewaschen, über Natriumsulfat getrocknet und eingedampft. Destillation des Rückstandes unter vermindertem Druck ergibt Diäthoxymethyl(n-butyl)-phosphinsäureäthylester vom Kp = 71,5-74° ($10^{-6}$ bar).

Eine Lösung von 109 g Diäthoxymethyl(n-butyl)phosphinsäureäthylester in 160 ml 4n-Salzsäure wird 24 Stunden zum Rückfluss erhitzt, auf Raumtemperatur abgekühlt mit Diäthyläther gewaschen und zur Trockne eingedampft. Der Rückstand wird einer azeotropen Destillation mit Äthanol unterworfen und erneut eingedampft. Die zurückbleibende n-Butylphosphinsäure wird bei 50° 20 Stunden im Hochvakuum getrocknet. Man erhält ein Öl; $^{1}$H-NMR-Spektrum (in $CDCl_3$): $\delta$ = 11,13 (1H,s); 7,11 (1H,dt; J = 52,0 und 0,2 Hz); 1,77 (2H,m); 1,58 (2H,m); 0,3 (3H,t).

51 g n-Butylphosphinsäure werden in 200 ml Dichlormethan gelöst, auf 10° gekühlt und tropfenweise mit 42,3 g Triäthylamin versetzt. Es erfolgt exotherme Reaktion. Man kühlt erneut auf 10° und fügt innerhalb von 75 Minuten tropfenweise 45,3 g Chlorameisensäureäthylester hinzu. Unter exothermer Reaktion und Gasentwicklung bildet sich ein Niederschlag. Dann gibt man weitere 200 ml Dichlormethan hinzu, lässt lässt auf Raumtemperatur erwärmen, 2 Stunden bei Raumtemperatur nachrühren, wäscht mit Wasser, trocknet über Natriumsulfat und zieht das organische Lösungsmittel ab. Das Rohprodukt wird im Hochvakuum destilliert. Man erhält n-Butylphosphinsäureäthylester vom Kp = 95° ($5x10^{-5}$ bar).

15,0 g n-Butylphosphinsäureäthylester und 5,3 g Acrylnitril werden unter Argon in 25 ml Äthanol gelöst. Min kühlt auf 10°, und versetzt tropfenweise mit einer Lösung von 1,15 g Natrium in 50 ml Äthanol, wobei exotherme Reaktion erfolgt. Dann erhitzt man 1 Stunde zum, Rückfluss, kühlt auf Raumtemperatur und versetzt mit 3,3 g Eisessig. Das Lösungsmittel wird abgezogen und der Rückstand wird in Dichlormethan aufgenommen. Die erhaltene Lösung wird mit Wasser gewaschen, über Natriumsulfat getrocknet und eingedampft. Das zurückbleibende Öl wird destilliert. Man erhält 2-Cyanäthyl(n-butyl)-phosphinsäureäthylester vom Kp = 120° ($10^{-1}$ bar).

16,46 g 2-Cyanäthyl(n-butyl)phosphinsäureäthylester werden in 165 ml Äthanol gelöst, mit 16,5 g Ammoniak und 3,0 g Raney-Nickel vermischt und bei 70-75° und einem Anfangsdruck von 100 bar 2 Stunden hydriert. Man lässt auf Raumtemperatur abkühlen, filtriert vom Katalysator ab, zieht das Lösungsmittel ab und destilliert den Rückstand. Man erhält 3-Aminopropyl(n-butyl)phosphinsäureäthylester vom Kp = 100° ($10^{-5}$ bar).

4,14 g 3-Aminopropyl(n-butyl)phosphinsäureäthylester werden mit 2,51 g p-Chlorbenzaldehyd vermischt und zunächst mit 1,2 g Eisessig und dann mit einer Lösung von 0,42 g Natriumcyanoborhydrid in 5 ml Methanol versetzt, woraufhin exotherme Reaktion erfolgt. Man lässt 2,5 Stunden bei Raumtemperatur nachrühren, stellt auf pH = 8 ein und zieht die flüchtigen Bestandteile unter vermindertem Druck ab. Der Rückstand wird in Dichlormethan gelöst, mit Wasser gewaschen, über Natriumsulfat getrocknet und eingedampft. Der Eindampfrückstand wird an Silicagel chromatographisch gereinigt. Man erhält 3-(p-Chlorbenzylamino)propyl(n-butyl)phosphinsäureäthylester; $^{1}$H-NMR-Spektrum (in $CDCl_3$): $\delta$ = 7.30-7,32 (4H,m); 4,02 (2H,q); 3,74 (2H,s); 2,67 (2H,t); 2,5-2,22 (1H,S); 1.79-1.64 (6H.m): 1,53 (2H,m); 1,42 (2H,m); 1,31 (3H,t); 0,81 (3H,t); $^{31}$P-NMR-Spektrum (in $CDCl_3$): $\delta$ = 58,16.

<u>Herstellungsbeispiel 4</u>: In analoger Weise wie in Herstellungsbeispiel 1 beschrieben kann man ausgehend von 3-Aminopropyl(cyclohexylmethyl)phosphinsäureäthylester und p-Chlorbenzaldehyd 3-(p-Chlorbenzylamino)propyl(cyclohexylmethyl)phosphinsäure vom Smp` 218-219° herstellen.

<u>Herstellungsbeispiel 5:</u> In analoger Weise wie in Herstellungsbeispiel 2 beschrieben kann man ausgehend von Benzylphosphinsäureäthylester, (S)-Epichlorhydrin und p-Chlorbenzaldehyd 3-(p-Chlorbenzylamino)-2(R)-hydroxy-propyl(benzyl)phosphinsäure vom Smp. 218-222° herstellen.

<u>Herstellungsbeispiel 6:</u> In analoger Weise wie in Herstellungsbeispiel 1 beschrieben kann man ausgehend von 3-Aminopropyl(diäthoxymethyl)phosphinsäureäthylester und 3,4-Dichlorbenzaldehyd 3-(3,4-Dichlorbenzylamino)propyl(diäthoxymethyl)phosphinsäure vom Smp. 175-176° herstellen.

<u>Herstellungsbeispiel 7:</u> In analoger Weise wie in Herstellungsbeispiel 1 beschrieben kann man ausgehend von 3-Aminopropyl(diäthoxymethyl)phosphinsäureäthylester und p-Chloracetophenon 3-[1-(p-Chlorphenyl)äthylamino]propyl(diäthoxymethyl)phosphinsäure, hygroskopische Kristalle, $^{31}$P-NMR ($CD_3OD$): $\delta$ = 47,1 ppm, herstellen.

<u>Herstellungsbeispiel 8</u>: In analoger Weise wie in Herstellungsbeispiel 1 beschrieben kann man ausgehend von 3-Aminopropyl(diäthoxymethyl)phosphinsäureäthylester und 1-Formylnaphthalin 3-(Naphth-1-ylmethylamino)propyl(diäthoxymethyl)phosphinsäure von, Smp. 222-225°, herstellen.

<u>Herstellungsbeispiel 9</u>: In analoger Weise wie in Herstellungsbeispiel 3 beschrieben kann man ausgehend von 3-Aminopropyl(cyclohexylmethyl)phosphinsäureäthylester und 3,4-Dichlorbenzaldehyd 3-(3,4-Dichlorbenzylamino)propyl(cyclohexylmethyl)phosphinsäure-hydrochlorid Smp. 115-226°, herstellen.

<u>Herstellungsbeispiel 10</u>: In analoger Weise wie in Herstellungsbeispiel 3 beschrieben kann man ausgehend von 3-Chlor-2(R)-hydroxy-propyl(benzyl)phosphinsäureäthylester und 2-Aminomethylpyridin 3-(Pyrid-2-ylmethylamino)-2(S)-hydroxy-propyl(benzyl)phosphinsäure-hydrochlorid, $[\alpha]_{20}^{D}$ = 10,2±02 (c = 1% in $CH_3OH$), herstellen.

<u>Herstellungsbeispiel 11</u>: In analoger Weise wie in Herstellungsbeispiel 2 beschrieben kann man ausgehend von 3-Chlor-2(R)-hydroxy-propyl(cyclohexylmethyl)phosphinsäure-äthylester und p-Chlorbenzylamin 3-(p-Chlorbenzylaminoyl)-2(S)-hydroxy-propyl(cyclohexylmethyl)phosphinsäure-hydrochlorid vom Smp. 206-210° herstellen.

Herstellungsbeispiel 12: In analoger Weise wie in Herstellungsbeispiel 2 beschrieben kann man ausgehend von 3-Chlor-2(R)-hydroxy-propyl(benzyl)phosphinsäureäthylester und 3,4-Dichlorbenzylamin 3-(3,4-Dichlorbenzylaminoyl)-2(S)-hydroxy-propyl(benzyl)phosphinsäure-hydrochlorid vom Smp. 186-187° herstellen.

Herstellungsbeispiel 13: In analoger Weise wie in Herstellungsbeispiel 1 beschrieben kann man ausgehend von 3-Aminopropyl(diäthoxymethyl)phosphinsäureäthylester und p-Fluorbenzaldehyd 3-(p-Fluorbenzylamino)propyl(diäthoxymethyl)phosphinsäure vom Smp. 128-129° herstellen.

Herstellungsbeispiel 14: In analoger Weise wie in Herstellungsbeispiel 1 beschrieben kann man ausgehend von 3-Aminopropyl(diäthoxymethyl)phosphinsäureäthylester und 4-Chlor-3-trifluormethyl-benzaldehyd 3-(4-Chlor-3-trifluormethyl-benzylamino)propyl(diäthoxymethyl)phosphinsäure vom Smp. 138-139° herstellen.

Herstellungsbeispiel 15: In analoger Weise wie in Herstellungsbeispiel 1 beschrieben kann man ausgehend von 3-Aminopropyl(diäthoxymethyl)phosphinsäureäthylester und m-Chlorbenzaldehyd 3-(m-Chlorbenzylamino)propyl(diäthoxymethyl)phosphinsäure vom Smp. 158-160° herstellen.

Herstellungsbeispiel 16: In analoger Weise wie in Herstellungsbeispiel 2 beschrieben kann man ausgehend von 3-Chlor-2(R)-hydroxy-propyl(cyclohexylmethyl)phosphinsäureäthylester und 3,4-Dichlorbenzylamin 3-(3,4-Dichlorbenzylamino)-2(S)-hydroxy-propyl(cyclohexylmethyl)phosphinsäure-hydrochlorid vom Smp. 193-196° herstellen.

Herstellunsbeispiel 17: In analoger Weise wie in Herstellungsbeispiel 1 beschrieben kann man ausgehend von 3-Aminopropyl(diäthoxymethyl)phosphinsäureäthylester und 3,4-Dichloracetophenon 3-[1-(3,4-Dichlorphenyl)äthylamino]propyl(diäthoxymethyl)phosphinsäure vom Smp. 85-90° herstellen.

Herstellungsbeispiel 18: In analoger Weise wie in Herstellungsbeispiel 2 beschrieben kann man ausgehend von 3-Chlor-2(R)-hydroxy-propyl(benzyl)phosphinsäureäthylester und 1-(p-Chlorphenyl)-äthylamin 3-[1-(p-Chlorphenyl)äthylamino]-2(S)-hydroxypropyl(benzyl)phosphinsäure-hydrochlorid vom Smp. 93-95° herstellen.

Herstellungsbeispiel 19: In analoger Weise wie in Herstellungsbeispiel 1 beschrieben kann man ausgehend von 3-Aminopropyl(diäthoxymethyl)phosphinsäureäthylester und p-Jodbenzaldehyd 3-(p-Jodbenzylamino)propyl(diäthoxymethyl)phosphinsäure vom Smp. 108-110° herstellen.

Herstellungsbeispiel 20: In analoger Weise wie in Herstellungsbeispiel 1 beschrieben kann man ausgehend von 3-Aminopropyl(diäthoxymethyl)phosphinsäureäthylester und 2,4-Dichlorbenzaldehyd 3-(2,4-Dichlorbenzylamino)propyl(diäthoxymethyl)phosphinsäure vom Smp. 173-175° herstellen.

Herstellungsbeispiel 21: In analoger Weise wie in Herstellungsbeispiel 1 beschrieben kann man ausgehend von 3-Aminopropyl(diäthoxymethyl)phosphinsäureäthylester und o-Chlorbenzaldehyd 3-(o-Chlorbenzylamino)propyl(diäthoxymethyl)phosphinsäure vom Smp. 162-163° herstellen.

Herstellungsbeispiel 22: In analoger Weise wie in Herstellungsbeispiel 1 beschrieben kann man ausgehend von 3-Aminopropyl(cyclopropylmethyl)phosphinsäureäthylester und 3,4-Dichlorbenzaldehyd 3-(3,4-Dichlorbenzylamino)propyl(cyclopropylmethyl)phosphinsäure vom Smp. 214-215° herstellen.

Herstellungsbeispiel 23: Zu einer Lösung von 0,41 g von 3-(p-Chlorbenzylamino)propyl(tetrahydrofuran-2-yl)phosphinsäureäthylester fügt man 0,38 g Trimethylbromsilan hinzu, lässt 24 Stunden bei Raumtemperatur rühren und zieht die flüchtigen Bestandteile unter vermindertem Druck ab. Das zurückbleibende Öl wird in 99 %igem Methanol aufgenommen, 30 Minuten bei Raumtemperatur gerührt und erneut unter vermindertem Druck eingedampft. Umkristallisation des festen, gelblichen Rückstandes ergibt 3-(p-Chlorbenzylamino)propyl(tetrahydrofuran-2-yl)phosphinsäure-hydrochlorid vom Smp. 212-213°; $^{1}$H-NMR-Spektrum (in $CD_3OD$): $\delta = 7,5$ (4H,m), 4,21 (2H,s), 4,03(1H, ABq), 3.84(2H, t,d), 3,17 (2H,t), 2,30-1,83 (8H,m); $^{31}$P-NMR-Spektrum (in $CD_3OD$): $\delta =$ 49.4.

Herstellungsbeispiel 24: Eine Lösung von 2,0 g 3-[N-(p-Chlorbenzyl)-N-methylamino])propyl-(diäthoxymethyl)phosphinsäureäthylester in 5,5 ml Äthanol wird mit 0,43 g Lithiumhydroxid-Monohydrat in 6 ml Wasser versetzt und 25 Stunden auf 60° erhitzt. Das Reaktionsgemisch wird auf Raumtemperatur abgekühlt, das Lösungsmittel wird unter vermindertem Druck abgezogen und der Eindampfrückstand wird in Wasser aufgenommen und mit Phosphorsäure neutralisiert. Die gebildete Suspension wird zur Trockne eingedampft, der Eindampfrückstand wird in heissem Methanol aufgenommen, filtriert und erneut eingedampft. Kristallisation des Eindampfrückstandes aus Propanol ergibt 3-[N-(p-Chlorbenzyl)-N-methyl-amino]-propyl(diäthoxymethyl)phosphinsäure vom Smp. 170-171,5°.

Das Ausgangsmaterial kann beispielsweise wie folgt hergestellt werden:

2,5 g 3-(p-Chlorbenzylamino)propyl(diäthoxymethyl)phosphinsäureäthylester werden in 10 ml Methanol gelöst, mit 0,36 g (0,53 ml) einer 35 %-igen wässrigen Formaldehydlösung versetzt und 1 Stunde bei Raumtemperatur gerührt. Man fügt 0,4 g Eisessig und 0.40 g Natriumcyanoborhydrid hinzu, lässt 2 Stunden bei Raumtemperatur rühren, dampft unter vermindertem Druck ein, nimmt in Dichlormethan auf und wäscht

mit 5 %-iger wässriger Natriumhydrogencarbonatlösung. Die organische Phase wird abgetrennt, über Natriumsulfat getrocknet und unter vermindertem Druck eingedampft. Der ölige Rückstand wird an Silicagel chromatographisch gereinigt. Man erhält 3-(p-Chlorbenzylamino)propyl(tetrahydrofuran-2-yl)-phosphinsäureäthylester als farbloses Öl.

Herstellungsbeispiel 25: In analoger Weise wie in Herstellungsbeispiel 2 beschrieben kann man 3-(p-Chlorbenzylamino)-2(S)-hydroxy-propyl(p-chlorbenzyl)phosphinsäurehydrochlorid vom Smp. 226-228° herstellen.

Herstellungsbeispiel 26: In analoger Weise wie in Herstellungsbeispiel 2 beschrieben kann man 3-(3,4-Dichlorbenzylamino)-2(S)-hydroxy-propyl(p-chlorbenzyl)phosphinsäurehydrochlorid herstellen.

Herstellungsbeispiel 27: In analoger Weise wie in Herstellungsbeispiel 2 beschrieben kann man 3-(p-Chlorbenzylamino)-2(S)-hydroxy-propyl(p-methylbenzyl)phosphinsäurehydrochlorid vom Smp. 209-210° herstellen. Auflösen desselben Äthanol und Behandlung mit Propylenoxid ergibt 3-(p-Chlorbenzylamino)-2-(S)-hydroxy-propyl(p-methylbenzyl)pho sphinsäure vom Smp. 239,5-241°.

Herstellungsbeispiel 28: In analoger Weise wie in Herstellungsbeispiel 2 beschrieben kann man 3-(3,4-Dichlorbenzylamino)-2(S)-hydroxy-propyl(p-methylbenzyl)phosphinsäure-hydrochlorid herstellen. Auflösen desselben in Äthanol und Behandlung mit Propylenoxid ergibt 3-(3,4-Dichlorbenzylamino)-2(S)-hydroxy-propyl(p-methylbenzyl )phosphinsäure vom Smp. 222,5-224°.

Herstellungsbeispiel 29: In analoger Weise wie in Herstellungsbeispiel 2 beschrieben kann man 3-(m-Chlorbenzylamino)-2(S)-hydroxy-propyl(benzyl)phosphinsäure vom Smp. 222,5-224° herstellen.

Herstellungsbeispiel 30: In analoger Weise wie in den Herstellungsbeispielen 1 bis 24 beschrieben kann man ferner herstellen:
3-(p-Chlorbenzylamino)-2(S)-hydroxy-propyl(diäthoxymethyl)phosphinsäure;
3-[1-(p-Chlorphenyl)äthylamino]-2(S)-hydroxy-propyl(diäthoxymethyl)phosphinsäure;
3-(3,4-Dichlorbenzylamino)-2(S)-hydroxy-propyl(diäthoxymethyl)phosphinsäure;
3-[1-(3,4-Dichlorphenyl)äthylamino]-2(S)-hydroxy-propyl(diäthoxymethyl)phosphinsäure;
3-[N-(p-Chlorbenzyl)-N-methyl-amino]-2(S)-hydroxy-propyl(benzyl)phosphinsäure;
3-(p-Chlorbenzylamino)-2(S)-hydroxy-propyl(p-methoxybenzyl)phosphinsäure;
3-(p-Chlorbenzylamino)-2(S)-hydroxy-propyl(3,4-dimethoxybenzyl)phosphinsäure;
3-(3,4-Dichlorbenzylamino)-2(S)-hydroxy-propyl(3,4-dimethoxybenzyl)phosphinsäure;
3-[2-(p-Chlorphenyl)prop-2-ylamino]propyl(diäthoxymethyl)phosphinsäure;
3-[2-(3,4-Dichlorphenyl)prop-2-ylamino]propyl(diäthoxymethyl)phosphinsäure;
3-[2-(p-Chlorphenyl)prop-2-ylamino]-2(S)-hydroxy-propyl(benzyl)phosphinsäure;
3-[2-(3,4-Dichlorphenyl)prop-2-ylamino)-2(S)-hydroxy-propyl(benzyl)phosphinsäure;
3-(3,4-Dichlorbenzylamino)-2(S)-hydroxy-propyl(3,4,5-trihydroxycyclohexylmethyl)-phosphinsäure;
3-(4-Chlor-3-methoxy-benzylamino)propyl(diäthoxymethyl)phosphinsäure;
3-(4-Chlor-3-methoxy-benzylamino)-2(S)-hydroxy-propyl(benzyl)phosphinsäure;
3-(3-Chlor-4-methoxy-benzylamino)-2(S)-hydroxy-propyl(benzyl)phosphinsäure;
3-(2-Phenyläthylamino)propyl(diäthoxymethyl)phosphinsäure;
3-(2-Phenyläthylamino)-2(S)-hydroxy-propyl(diäthoxymethyl)phosphinsäure;
3-(p-Chlorbenzylamino-2(S)-hydroxy-propyl(4-methoxycyclohexylmethyl)phosphinsäure;
3-(3,4-Dichlor-6-jod-benzylamino)-2(S)-hydroxy-propyl(benzyl)phosphinsäure;
3-(3,4-Dichlor-6-jod-benzylamino)propyl(diäthoxymethyl)phosphinsäure;
3-(4-Chlor-3-jod-benzylamino)propyl(diäthoxymethyl)phosphinsäure;
3-(3-Chlor-4-jod-benzylamino)propyl(diäthoxymethyl)phosphinsäure;
3-(4-Chlor-3-jod-benzylamino)-2(S)-hydroxy-propyl(benzyl)phosphinsäure;
3-(3-Chlor-4-jod-benzylamino)-2(S)-hydroxy-propyl(benzyl)phosphinsäure;
3-Di(p-chlorbenzyl)aminopropyl(diäthoxymethyl)phosphinsäure;
3-Di(3,4-dichlorbenzyl)amino-2(S)-hydroxy-propyl(benzyl)phosphinsäure;
3-(3,4-Dichlorbenzyl)amino-2(S)-hydroxy-propyl(cyclohex-3-enylmethyl)phosphinsäure 3-[1-(3,4-Dichlorphenyl)äthylamino]-2(S)-hydroxy-propyl(cyclohex-3-enylmethyl)phosphinsäure;
3-(3,4-Dichlorbenzyl)amino-2(S)-hydroxy-propyl(*cis*-4,5-dihydroxycyclohexylmethyl)phosphinsäure und
3-[1-(3,4-Dichlorphenyl)äthylamino]-2(S)-hydroxy-propyl(*cis*-4,5-dihydroxycyclohexylmethyl)phosphinsäure
sowie ihre pharmazeutisch verwendbaren Salze, z.B. ihre Hydrochloride.

Formulierungsbeispiel 1: Tabletten, enthaltend je 200 mg 3-Aminopropyl(cyclohexylmethyl)-phosphinsäure oder ein Salz, z.B. das Hydrochlorid, davon können wie folgt hergestellt werden:

Zusammensetzung (10000 Tabletten)

| | |
|---|---|
| Wirkstoff | 2000,0 g |
| Lactose | 500,0 g |
| Kartoffelstärke | 352,0 g |
| Gelatine | 8,0 g |
| Talk | 60,0 g |
| Magnesiumstearat | 10,0 g |
| Siliciumdioxid(hochdispers) | 20,0 g |
| Äthanol | q.s. |

Der Wirkstoff wird mit der Lactose und 292 g Kartoffelstärke vermischt, die Mischung mit einer äthanolischen Lösung der Gelatine befeuchtet und durch ein Sieb granuliert. Nach dem Trocknen mischt man den Rest der Kartoffelstärke, das Magnesiumstearat, das Talk und das Siliciumdioxid zu und presst das Gemisch zu Tabletten von je 295,0 mg Gewicht und 200,0 mg Wirkstoffgehalt, die gewünschtenfalls mit Teilkerben zur feineren Anpassung der Dosierung versehen sein können.

Formulierungsbeispiel 2: Lacktabletten, enthaltend je 400 mg 3-Aminopropyl(cyclohexylmethyl)-phosphinsäure oder ein Salz, z.B. das Hydrochlorid, davon können wie folgt hergestellt werden:

Zusammensetzung (für 1000 Lacktabletten)

| | |
|---|---|
| Wirkstoff | 400,0 g |
| Lactose | 100,0 g |
| Maisstärke | 70,0 g |
| Talk | 8,5 g |
| Calciumstearat | 1,5 g |
| Hydroxpropylmethylcellulose | 2,36 g |
| Schellack | 0,64 g |
| Wasser | q.s. |
| Methylenchlorid | q.s. |

Der Wirkstoff, die Lactose und 40 g der Maisstärke werden gemischt und mit einem Kleister, hergestellt aus 15 g Maisstärke und Wasser (unter Erwärmen), befeuchtet und granuliert. Das Granulat wird getrocknet, der Rest der Maisstärke, der Talk und das Calciumstearat zugegeben und mit dem Granulat vermischt. Das Gemisch wird zu Tabletten mg) verpresst und diese mit einer Lösung der Hydroxypropyl-methylcellulose und des Schellacks in Methylenchlorid lackiert; Endgewicht der Lacktablette: 580 mg.

Formulierungsbeispiel 3: Gelatinesteckkapseln, enthaltend 500 mg Wirkstoff, z.B. 3-Aminopropyl-(cyclohexylmethyl)phosphinsäure oder ein Salz, z.B. das Hydrochlorid, davon, können z.B. folgendermassen hergestellt werden:

Zusammensetzung (für 1000 Kapseln)

| | |
|---|---|
| Wirkstoff | 500,0 g |
| Lactose | 250,0 g |
| mikrokristalline Zellulose | 30,0 g |
| Natriumlaurylsulfat | 2,0 g |
| Magnesiumstearat | 8,0 g |

Das Natriumlaurylsulfat wird durch ein Sieb mit einer Machenweite von 0,2 mm zu dem lyophilisierten Wirkstoff hinzugesiebt. Beide Komponenten werden innig vermischt. Dann wird zunächst die Lactose durch ein Sieb mit einer Maschenweite von 0,6 mm und dann die mikrokristalline Zellulose durch ein Sieb mit einer Maschenweite von 0,9 mm hinzugesiebt. Daraufhin wird erneut 10 Minuten innig gemischt. Zuletzt

wird das Magnesiumstearat durch ein Sieb mit einer Maschenweite von 0,8 mm hinzugesiebt. Nach 3-minütigem weiteren Mischen werden je 790 mg der erhaltenen Formulierung in Gelatinesteckkapseln passender Grösse abgefüllt.

Formulierungsbeispiel 4: Eine 5%-ige Injektions- oder Infusionslösung von 3-Aminopropyl-(cyclohexylmethyl)phosphinsäure oder eines Salzes, z.B. des Hydrochlorides, davon, kann beispielsweise folgendermassen hergestellt werden:

Zusammensetzung (für 1000 bzw. 400 Ampullen)

| | |
|---|---|
| Wirkstoff | 125,0 g |
| Natriumchlorid | 22,5 g |
| Phosphatpuffer pH = 7.4 | 300,0 g |
| entmineralisiertes Wasser | ad 2500,0 ml |

Der Wirkstoff und das Natriumchlorid werden in 1000 ml Wasser gelöst und durch ein Mikrofilter filtriert. Man versetzt mit der Pufferlösung und füllt mit Wasser auf 2500 ml auf. Zur Herstellung von Dosiseinheitsformen werden je 1,0 oder 2,5 ml in Glasampullen abgefüllt, die dann je 50 bzw. 125 mg Wirkstoff enthalten.

Formulierungsbeispiel 5: In analoger Weise wie in den vorstehenden Formulierungsbeispielen 1 bis 4 beschrieben kann man auch pharmazeutische Präparate, enthaltend eine Verbindung gemäss einem der Herstellungsbeispiele 1 bis 25 oder eine an sich bekannte Verbindung mit $GABA_B$-antagonistischen Eigenschaften, beispielsweise eine der erfindungsgemäss zur Verwendung als antiepileptischen Arzneimittelwirkstoff vorgeschlagene Verbindung der Formel I, insbesondere
3-Aminopropyl(n-butyl)phosphinsäure,
3-Aminopropyl(diäthoxymethyl)phosphinsäure,
3-Aminopropyl(benzyl)phosphinsäure,
3-Aminopropyl(1,1-difluorbutyl)phosphinsäure,
3-Amino-2-hydroxy-propyl(cyclohexylmethyl)phosphinsäure,
3-(p-Chlorbenzylamino)-2(R)-hydroxy-propyl(benzyl)phosphinsäure,
3-Amino-2(S)-hydroxy-propyl(cyclohexylmethyl)phosphinsäure,
3-Amino-2(S)-hydroxy-propyl(benzyl)phosphinsäure,
3-(p-Chlorbenzylamino)propyl(diäthoxymethyl)phosphinsäure,
3-(p-Chlorbenzylamino)-2(S)-hydroxy-propyl(benzyl)phosphinsäure,
3-(p-Chlorbenzylamino)-2-hydroxy-propyl(n-butyl)phosphinsäure,
3-(3,4-Dichlorbenzylamino)-2(S)-hydroxy-propyl(benzyl)phosphinsäure,
3-(3,4-Dichlorbenzylamino)-2(S)-hydroxy-propyl(diäthoxymethyl)phosphinsäure und
3-(p-Chlorbenzylamino)propyl(cyclohexylmethyl)phosphinsäure
oder jeweils ein pharmazeutisch verwendbares Salz davon herstellen.

Pharmakologisches Beispiel 1:

Hemmung der "Spike and Wave"-Entladungen an epileptischen Ratten

Methode: Die Wirkung von GABAB-Antagonisten wurde an männlichen Wistar-Ratten (300-400 g) der Strasbourg-Kolonie geprüft. Tiere der 16. oder 17. Generation des Stammes, in dem 100% der Versuchstiere "Spike and Wave"-Entladungen zeigen (epileptischen Ratten) bzw. Tiere der 16. oder 17. Generation des Stammes, in dem 0% der Versuchstiere "Spike and Wave"-Entladungen zeigen (Kontrollstamm) wurden verwendet. Die Ratten erhielten Wasser und Nahrung ad libidum und der Hell/Dunkel Zyklus betrug 12/12 h. Die Ratten wurden mit Pentobarbital (40 mg/kg i.p.) anaesthesiert und mit 4 rostfreien Stahlelektroden (je zwei im Frontal- und Parietal-Cortex) bilateral implantiert. Die Ratten wurden frühestens eine Woche nach der Operation für Experimente verwendet.

Die freibeweglichen Ratten wurden in einem Plexiglaskäfig (17x17x28 cm) untergebracht (eine Ratte pro Käfig) und das EEG nach 15-minütiger Anpassung an die neue Umgebung während 20 Minuten registriert (Referenzperiode). Man erhält so die Basislinie für die jeweilige Versuchsreihe. Anschliessend wurde ein GABAB-Antagonist verabreichet und das EEG während 120 bis 180 Minuten registiert. Die Ratten wurden während dieser Periode andauernd beobachtet und durch sanfte manuelle Berührung vom Einschlafen

abgehalten.

Versuchsanordnung: Die jeweilige Testsubstanz wurde in 0,9%-iger Natriumchloridlösung gelöst und in Dosen von 50, 100, 200 und 400 mg/kg i.p. injiziert. Alternativ kann man die jeweilige Testsubstanz auch einem Polyäthylenglykol, wie Tween® 80, (2 Tropfen/ 10 ml Suspension der Testsubstanz in 0,9%-iger Natriumchloridlösung; die Suspension wird bei 40° 10 min mittels Ultraschall homogenisiert, Injektionsvolumen: 2 ml/kg) i.p. oder per Magensonde p.o. appliziert werden. Schwerlösliche Testsubstanzen werden vorteilhaft in einem Gemisch von Dimethylsulfoxid (nicht mehr als 10 Vol-%) und 0,9%-iger Natriumchloridlösung oder in 45 %-iger wässriger Hydroxypropyl-β-cyclodextrinlösung gelöst appliziert. Jedes Tier wird mit allen Dosen in einer randomisierten Reihenfolge behandelt. Die Zeit zwischen 2 Behandlungen soll mindestens 5 Tage betragen. Erfasst werden die Mittelwerte ± SEM der aufsummierten Gesamtdauer der "Spike and Wave"-Entladungen von 6 Ratten über 20 Minuten.

Auswertung der Daten:

Die aufsummierte Gesamtdauer der Spike and Wave-Entladungen (in Sekunden) wird für jede 20-Minutenperiode der EEG- Registrierung gemessen. Vergleiche zwischen Behandlungs- und Referenzperiode werden mit Hilfe der nonparametrischen Varianzanalyze der betreffenden Gruppen durchgeführt (Friedman Test). Kontrolle und behandelte Gruppen werden erst dann mit Hilfe des Wilcoxon-Test verglichen, wenn mit Hilfe des Friedmann-Testes ein signifikanter Unterschied zwischen beiden Gruppen gefunden wird. Die dosisabhängige Abnahme der "Spike and Wave"-Entladungen ist in den folgenden Tabellen gezeigt. Die Signifikanz der Unterschiede der mit Sternchen markierten Werte zur Dosis 0 im Wilcoxon-Test entspricht mindestens $p<0,005$.

3-Aminopropyl(diäthoxymethyl)phosphinsäure (i.p.):

| DOSEN (mg/kg) | 0 | 50 | 100 | 200 | 400 |
|---|---|---|---|---|---|
| PERIODEN (min) | | | | | |
| 0-20 | 322±51 | 322±53 | 251±73 | 260±44 | 264±54 |
| 20-40 | 362±58 | 281±58 | 193±65 | 134±22 | 105±37 |
| 40-60 | 393±46 | 133±40* | 66±44* | 57±26* | 47±20* |
| 60-80 | 336±37 | 198±28* | 74±59* | 27±16* | 52±44* |
| 80-100 | 337±38 | 195±32 | 195±38 | 46±21* | 42±28* |
| 100-120 | 377±51 | 182±43 | 127±57 | 43±12* | 22±9* |
| BASISLINIE | 419±33 | 377±82 | 462±74 | 452±60 | 511±53 |

3-Aminopropyl(diäthoxymethyl)phosphinsäure (p.o.):

| DOSEN (mg/kg) | 0 | 300 | 500 |
|---|---|---|---|
| PERIODEN (min) | | | |
| 0-20 | 263±36 | 911±31* | 123±58 |
| 20-40 | 391±60 | 182±92 | 20±10* |
| 40-60 | 374±86 | 227±127 | 2±1* |
| 60-80 | 369±58 | 122±67 | 7±5* |
| 80-100 | 364±44 | 85±35* | 0±0* |
| 100-120 | 347±58 | 184±86* | 2±1* |
| 120-140 | 391±60 | 239±132* | 2±2* |
| 140-160 | 364±44 | 234±117* | 1±1* |
| BASISLINIE | 386±52 | 356±39 | 372±64 |

3-Aminopropyl(n-butyl)phosphinsäure (i.p.):

| DOSEN (mg/kg) / PERIODEN (min) | 0 | 50 | 100 | 200 | 400 |
|---|---|---|---|---|---|
| 0-20 | 321±48 | 219±55 | 213±25 | 192±73 | 49±21* |
| 20-40 | 353±59 | 232±45 | 58±18* | 73±21* | 13±6* |
| 40-60 | 299±30 | 137±34* | 59'18* | 40'19* | 5'2* |
| 60-80 | 327±61 | 151±34* | 76±30* | 47±22* | 2±1* |
| 80-100 | 312±55 | 227±53 | 33±12* | 32±30* | 1±1* |
| 100-120 | 282±39 | 258±39 | 107±40 | 95±45 | 0±0* |
| 120-140 | 197±51 | 226±41 | 135±36 | 42±25 | 2±1* |
| 140-160 | 219±52 | 292±39 | 92±21 | 82±44 | 1±1* |
| 160-180 | 270±44 | 309±32 | 131±29 | 58±21* | 2±1* |
| BASISLINIE | 369±60 | 340±60 | 435±48 | 430±58 | 425±74 |

3-Aminopropyl(n-butyl)phosphinsäure (p.o.):

| DOSEN (mg/kg) / PERIODEN (min) | 0 | 300 | 500 |
|---|---|---|---|
| 0-20 | 263±36 | 61±23* | 143±45 |
| 20-40 | 391±60 | 65±42* | 134±38 |
| 40-60 | 374±86 | 108±55 | 98±26* |
| 60-80 | 369±58 | 102±59 | 63±16* |
| 80-100 | 364±44 | 73±42* | 36±19* |
| 100-120 | 347±58 | 44±18* | 23±15* |
| 120-140 | 391±60 | 26±11* | 9±19* |
| 140-160 | 364±44 | 0±0* | 0±0* |
| BASISLINIE | 384±52 | 231±30 | 285±53 |

3-Aminopropyl(cyclohexylmethyl)phosphinsäure (i.p.):

| DOSEN (mg/kg) / PERIODEN (min) | 0 | 25 | 50 | 100 | 200 | 400 |
|---|---|---|---|---|---|---|
| 0-20 | 333±39 | 163±19 | 63±20* | 133±37* | 61±18* | 38±23* |
| 20-40 | 385±48 | 45±15* | 17±7* | 15±3* | 3±3* | 0±0* |
| 40-60 | 312±50 | 14±5* | 1±1* | 12±3* | 1±1* | 0±0* |
| 60-80 | 392±51 | 29±8* | 11±5* | 3±2* | 0±0* | 0±0* |
| 80-100 | 392±41 | 41±6* | 0±0* | 32±7* | 2±2* | 0±0* |
| 100-120 | 262±30 | 18±8* | 45±22* | 12±5* | 3±2* | 1±1* |
| BASISLINIE | 383±38 | 235±26 | 361±75 | 371±66 | 463±55 | 380±77 |

3-Aminopropyl(cyclohexylmethyl)phosphinsäure (p.o.):

| DOSEN (mg/kg) | 0 | 300 | 500 |
|---|---|---|---|
| PERIODEN (min) | | | |
| 0-20 | 263±36 | 124±53 | 10±6* |
| 20-40 | 391±60 | 157±78 | 0±0* |
| 40-60 | 374±86 | 162±71 | 0±0* |
| 60-80 | 369±58 | 75±43 | 0±0* |
| 80-100 | 364±44 | 116±77 | 0±0* |
| 100-120 | 347±58 | 21±21* | 0±0* |
| 120-140 | 391±60 | 54±38* | 0±0* |
| 140-160 | 364±44 | 47±34* | 0±0* |
| BASISLINIE | 386±52 | 337±37 | 426±74 |

3-Aminopropyl(benzyl)phosphinsäure (i.p.):

| DOSEN (mg/kg) | 0 | 50 | 100 | 200 | 400 |
|---|---|---|---|---|---|
| PERIODEN (min) | | | | | |
| 0-20 | 292±57 | 223±57 | 186±43 | 12±11* | 7±4* |
| 20-40 | 306±82 | 68±30* | 39±26* | 2±1* | 0±0* |
| 40-60 | 268±15 | 114±32* | 39±28* | 1±1* | 0±0* |
| 60-80 | 326±69 | 112±34* | 52±30* | 2±2* | 0±0* |
| 80-100 | 326±73 | 140±43 | 36±13* | 2±1* | 0±0* |
| 100-120 | 232±55 | 162±50 | 27±17* | 0±0* | 0±0* |
| BASISLINIE | 343±68 | 393±75 | 318±49 | 307±34 | 415±73 |

3-Amino-2-hydoxy-propyl(cyclohexylmethyl)phosphinsäure (i.p.):

| DOSEN (mg/kg) | 0 | 25 | 50 | 100 | 200 | 400 |
|---|---|---|---|---|---|---|
| PERIODEN (min) | | | | | | |
| 0-20 | 294±37 | 240±67 | 192±50 | 98±43* | 7±7* | 7±5* |
| 20-40 | 227±33 | 78±47* | 82±44* | 41±29* | 0±0* | 0±0* |
| 40-60 | 276±34 | 27±8* | 21±9* | 40±39* | 1±1* | 1±1* |
| 60-80 | 281±40 | 35±20* | 8±3* | 16±16* | 1±1* | 3±3* |
| 80-100 | 299±14 | 6±2* | 15±5 | 16±16* | 0±0* | 5±5* |
| 100-120 | 293±14 | 29±14* | 13±5 | 17±17* | 0±0* | 5±5* |
| BASISLINE | 312±36 | 305±65 | 445±74 | 315±60 | 292±26 | 310±50 |

3-Amino-2-hydoxy-propyl(cyclohexylmethyl)phosphinsäure (p.o.):

| DOSEN (mg/kg) | 0 | 300 | 500 |
|---|---|---|---|
| PERIODEN (min) | | | |
| 0-20 | 263±36 | 176±61 | 105±29* |
| 20-40 | 391±60 | 103±42* | 149±43 |
| 40-60 | 374±86 | 95±33* | 117±26* |
| 60-80 | 369±58 | 73±22* | 104±26* |
| 80-100 | 364±44 | 46±14* | 56±30* |
| 100-120 | 347±58 | 19±12* | 32±12* |
| 120-140 | 391±60 | 73±55* | 40±13* |
| 140-160 | 364±44 | 4±4* | 5±4* |
| BASISLINIE | 386±52 | 220±43 | 288±46 |

3-Aminopropyl(cyclopropylmethyl)phosphinsäure (i.p.):

| DOSEN (mg(kg) | 0 | 100 | 200 | 400 |
|---|---|---|---|---|
| PERIODEN (min) | | | | |
| 0-20 | 332±78 | 292±47 | 338±71 | 199±75 |
| 20-40 | 321±47 | 252±24 | 108±54* | 60±34* |
| 40-60 | 334±62 | 107±18* | 100±72* | 7±4* |
| 60-80 | 358±68 | 185±51* | 19±12* | 5±3* |
| 80-100 | 272±37 | 117±30* | 90±46* | 2±2* |
| 100-120 | 339±37 | 151±40* | 54±27* | 4±1* |
| BASISLINIE | 372±64 | 392±77 | 447±66 | 352±12 |

3-Aminopropyl(1,1-difluorbutyl)phosphinsäure (i.p.):

| DOSEN /mg/kg) | 0 | 100 | 200 |
|---|---|---|---|
| PERIODEN (min) | | | |
| 0-20 | 267±39 | 145±57 | 219±61 |
| 20-40 | 266±38 | 63±27* | 71±33* |
| 40-60 | 273±40 | 56±27* | 47±18* |
| 60-80 | 263±37 | 99±46* | 16±11* |
| 80-100 | 264±35 | 87±47* | 14±13* |
| 100-120 | 308±41 | 36±23* | 18±10* |
| BASISLINIE | 307±42 | 304±72 | 297±46 |

3-Aminopropyl(1-hydroxybenzyl)phosphinsäure (i.p.):

| DOSEN (mg/kg) | 0 | 100 | 200 | 400 |
|---|---|---|---|---|
| PERIODEN (min) | | | | |
| 0-20 | 251±56 | 235±74 | 187±80 | 49±17* |
| 20-40 | 273±60 | 222±55 | 78±40* | 16±6* |
| 40-60 | 306±70 | 88±20* | 44±21* | 1±1* |
| 60-80 | 254±48 | 167±63 | 12±6* | 0±0* |
| 80-100 | 262±54 | 130±48 | 20±10* | 9±4* |
| 100-120 | 280±57 | 95±25* | 14±10 | 7±2* |
| BASISLINIE | 306±45 | 320±34 | 298±36 | 248±23 |

3-Amino-2(S)hydroxy-propyl(cyclohexylmethyl)phosphinsäure (i.p.):

| DOSEN (mg/kg) | 0 | 25 | 100 |
|---|---|---|---|
| PERIODEN (min) | | | |
| 0-20 | 286±47 | 206±48 | 240±60 |
| 20-40 | 310±64 | 97±19* | 12±5* |
| 40-60 | 342±69 | 49±23* | 6±4* |
| 60-80 | 285±47 | 23±14* | 2±1* |
| 80-100 | 292±54 | 4±3* | 4±3* |
| 100-120 | 301±49 | 9±5* | 3±2* |
| BASISLINIE | 341±43 | 402±58 | 548±38 |

3-(3,4-Dichlorbenzylamino)propyl(diäthoxymethyl)phosphinsäure (i.p):

| DOSEN (mg/kg) | 0 | 100 | 200 | 400 |
|---|---|---|---|---|
| PERIODEN (min) | | | | |
| 0-20 | 321±50 | 119±49 | 148±65 | 310±73 |
| 20-40 | 292±92 | 151±33 | 166±92 | 45±25* |
| 40-60 | 363±96 | 270±68 | 161±103 | 75±44* |
| 60-80 | 313±45 | 151±27 | 200±112 | 100±71* |
| 80-100 | 326±52 | 261±60 | 183±110 | 58±363 |
| 100-120 | 328±72 | 205±40 | 218±58 | 76±48* |
| BASISLINIE | 348±58 | 271±64 | 420±48 | 424±55 |

Pharmakologisches Beispiel 2:

Hemmung der durch "grand mal"-Antiepileptika induzierten "Spike and Wave"-Entladungen an epileptischen Ratten

Methode und Versuchsanordnung

Methode und Versuchsanordnung entsprechen derjenigen des pharmakologischen Beispiels 1.

3-Aminopropyl(diäthoxymethyl)phosphinsäure: Antagonismus der durch Carbamazepin induzierten "Spike and Wave"-Entladungen.
3-Aminopropyl(diäthoxymethyl)phosphinsäure wurde zum Zeitpunkt 0 und 20 mg/kg Carbamazepin zum Zeitpunkt 40 jeweils i.p. appliziert.

| DOSEN (mg/kg) | 0 | 200 | 400 |
| --- | --- | --- | --- |
| PERIODEN (min) | | | |
| 0-20 | 289±43 | 353±53 | 115±43 |
| 20-40 | 324±46 | 178±37 | 93±71* |
| 40-60 | 636±43 | 173±78* | 23±14* |
| 60-80 | 669±14 | 70±64* | 29±29* |
| 80-100 | 675±75 | 18±17* | 9±9* |
| BASISLINIE | 337±58 | 357±30 | 310±69 |

3-Aminopropyl(diäthoxymethyl)phosphinsäure: Antagonismus der durch Phenytoin induzierten "Spike and Wave"-Entladungen.
3-Aminopropyl(diäthoxymethyl)phosphinsäure wurde zum Zeitpunkt 0 und 20 mg/kg Phenytoin zum Zeitpunkt 40 jeweils i.p. appliziert.

| DOSEN (mg/kg) | 0 | 200 | 400 |
| --- | --- | --- | --- |
| PERIODEN (min) | | | |
| 0-20 | 382±62 | 394±87 | 139±61 |
| 20-40 | 330±58 | 172±64 | 48±40 |
| 40-60 | 555±24 | 124±85* | 31±31* |
| 60-80 | 658±27 | 239±70* | 56±53* |
| 80-100 | 562±76 | 95±37* | 42±39* |
| BASISLINIE | 371±23 | 430±61 | 497±68 |

3-Aminopropyl(diäthoxymethyl)phosphinsäure: Antagonismus der durch Vigabatrin® induzierten "Spike and Wave"-Entladungen. 3-Aminopropyl(diäthoxymethyl)phosphinsäure wurde zum Zeitpunkt 0 und 20 mg/kg Vigabatrin® zum Zeitpunkt 160 jeweils i.p. appliziert.

| DOSEN (mg/kg) | 0 | 200 | 400 |
| --- | --- | --- | --- |
| PERIODEN (min) | | | |
| 240-260 | 875±63 | 213±52* | 44±13* |
| 260-280 | 938±65 | 249±57* | 28±8* |
| BASISLINIE | 408±68 | 310±34 | 363±44 |

**Patentansprüche**

**Patentansprüche für folgende Vertragsstaaten : AT, BE, CH, DE, DK, FR, GB, IT, LI, LU, NL, SE**

1. Neue araliphatisch N-substituierte Aminoalkanphosphinsäuren der Formel I

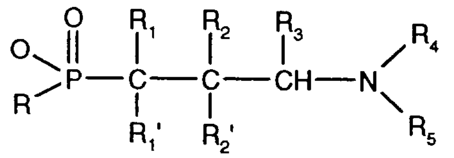

(I),

worin $R_1$, $R_1$', $R_2$' und $R_3$ Wasserstoff bedeuten, $R_2$ Wasserstoff oder Hydroxy darstellt, $R_4$ einen im Phenyl- bzw. Naphthylteil durch $C_1$-$C_4$-Alkyl, $C_1$-$C_4$-Alkoxy, Hydroxy und/oder Halogen mono-, di- oder trisubstituierten Phenyl-$C_1$-$C_4$-alkyl-, Diphenyl-$C_1$-$C_4$-alkyl- oder Naphthyl-$C_1$-$C_4$-alkylrest oder einen unsubstituierten oder im Thienyl-, Furyl- bzw. Pyridylteil durch Halogen substituierten Thienyl-, Furyl- oder Pyridyl-$C_1$-$C_4$-alkylrest bedeutet, $R_5$ Wasserstoff, $C_1$-$C_4$-Alkyl oder eine Gruppe $R_4$ darstellt und R $C_2$-$C_4$-Alkyl, $C_2$-$C_4$-Alkenyl, $C_2$-$C_7$-Alkinyl, Oxo-$C_2$-$C_7$-alkyl, Hydroxy-$C_2$-$C_7$-alkyl oder Dihydroxy-$C_2$-$C_7$-alkyl, $\alpha$-Hydroxy-$C_3$-$C_5$-alkenyl, Mono-, Di- oder Trifluor-$C_2$-$C_5$-alkyl, Mono-, Di- oder Trifluor-$C_2$-$C_5$-alkenyl, Mono-, Di- oder Trifluor-$\alpha$-hydroxy-$C_2$-$C_5$-alkyl, Mono-, Di- oder Trifluor-$\alpha$-hydroxy-$C_2$-$C_5$-alkenyl, $C_1$-$C_4$-Alkoxy-$C_1$-$C_4$-alkyl, Di-$C_1$-$C_4$-alkoxy-$C_1$-$C_4$-alkyl, $C_1$-$C_4$-Alkoxy $C_1$-$C_4$-Alkoxy-$C_2$-$C_7$-(hydroxy)-alkyl, $C_1$-$C_4$-Alkoxy-$C_2$-$C_5$-(halo)-alkyl, $C_1$-$C_4$-$C_1$-$C_4$-alkyl, Di-$C_1$-$C_4$-alkylthio-$C_1$-$C_4$-alkyl, $C_3$-$C_8$-Cycloalkyl, $\alpha$-Hydroxy-$C_3$-$C_6$-cycloalkyl, Oxa- oder Thia-$C_3$-$C_6$-cycloalkyl, 1,3-Dioxa- oder 1,3-Dithia-$C_3$-$C_8$-cycloalkyl, $C_3$-$C_8$-Cycloalkyl-$C_1$-$C_4$-alkyl, $C_3$-$C_8$-Cycloalkenyl-$C_1$-$C_4$-alkyl, Mono-, Di- oder Trihydroxy-$C_3$-$C_8$-cycloalkenyl, $C_3$-$C_6$-Cycloalkyl-(hydroxy)-$C_1$-$C_4$-alkyl, 1-($C_1$-$C_4$-Alkylthio-$C_3$-$C_6$-cycloalkyl)-1-hydroxy)-$C_1$-$C_4$-alkyl, unsubstituiertes oder durch $C_1$-$C_4$-Alkyl, $C_1$-$C_4$-Alkoxy, Halogen, Hydroxy und/oder Trifluormethyl mono-, di- oder trisubstituiertes Mono- oder Diphenyl-$C_1$-$C_4$-alkyl, Naphthyl-$C_1$-$C_4$-alkyl 1 oder unsubstituiertes oder durch Halogen substituiertes Thienyl-, Furyl- oder Pyridyl-$C_1$-$C_4$-alkyl bedeutet, und ihre Salze.

2. Verbindungen gemäß Anspruch 1 der Formel I, worin $R_1$, $R_1$', $R_2$' und $R_3$ Wasserstoff bedeuten, $R_2$ Wasserstoff oder Hydroxy darstellt, $R_4$ einen durch $C_1$-$C_4$-Alkyl, $C_1$-$C_4$-Alkoxy, Hydroxy und/oder Halogen substituierten Phenyl- oder Naphthyl-$C_1$-$C_4$-alkylrest oder einen unsubstituierten oder durch durch Halogen substituierten Thienyl-, Furyl- oder Pyridyl-$C_1$-$C_4$alkylrest bedeutet, $R_5$ Wasserstoff, Niederalkyl oder eine Gruppe $R_4$ darstellt und R $C_2$-$C_4$-Alkyl, $C_2$-$C_4$-Alkenyl, $C_2$-$C_7$-Alkinyl, Oxo-$C_2$-$C_7$-alkyl, Hydroxy-$C_2$-$C_7$-alkyl oder Dihydroxy-$C_2$-$C_7$-alkyl, $\alpha$-Hydroxy-$C_3$-$C_5$-alkenyl. Mono-, Di- oder Mono-, Di- oder Trifluor-$C_2$-$C_5$-alkyl, Mono-, Di- oder Trifluor-$C_2$-$C_5$-alkenyl, Mono-, Di- oder Trifluor-$\alpha$-hydroxy-$C_2$-$C_5$-alkyl, Mono-, Di- oder Trifluor-$\alpha$-hydroxy-$C_2$-$C_5$-alkenyl, $C_1$-$C_4$-Alkoxy-$C_1$-$C_4$-alkyl, Di-$C_1$-$C_4$-alkoxy-$C_1$-$C_4$-alkyl, $C_1$-$C_4$-Alkoxy-$C_2$-$C_7$-(hydroxy)-alkyl,, $C_1$-$C_4$-Alkoxy-$C_2$-$C_5$-(halo)-alkyl, $C_1$-$C_4$-Alkylthio-$C_1$-$C_4$-alkyl, Di-$C_1$-$C_4$-alkylthio-$C_1$-$C_4$-alkyl, $C_3$-$C_8$-Cycloalkyl, $\alpha$-Hydroxy-$C_3$-$C_6$-cycloalkyl, Oxa- oder Thia-$C_3$-$C_6$-cycloalkyl, 1,3-Dioxa- oder 1,3-Dithia-$C_3$-$C_8$-cycloalkyl, $C_3$-$C_6$-Cycloalkyl-(hydroxy)-$C_1$-$C_4$-alkyl, 1-($C_1$-$C_4$-Alkylthio-$C_3$-$C_6$-cycloalkyl)-1-hydroxy)-$C_1$-$C_4$-alkyl oder unsubstituiertes oder durch $C_1$-$C_4$-Alkyl, $C_1$-$C_4$-Alkoxy, Hydroxy und/oder Halogen substituierten Phenyl-$C_1$-$C_4$-alkyl oder Naphthyl-$C_1$-$C_4$-alkyl bedeutet, und ihre Salze.

3. Verbindungen gemäß Anspruch 1 der Formel I, worin R $C_3$-$C_7$-Alkyl, $\alpha,\alpha$-Di-$C_1$-$C_4$-alkoxy-$C_1$-$C_4$-alkyl, $C_3$-$C_6$-Cycloalkyl-$C_1$-$C_4$-alkyl, $C_3$-$C_6$-Cycloalkenyl-$C_1$-$C_4$-alkyl, Mono-, Di- oder Trihydroxy-$C_3$-$C_6$-cycloalkyl-$C_1$-$C_4$-alkyl oder unsubstituiertes oder durch $C_1$-$C_4$-Alkyl, $C_1$-$C_4$-Alkoxy, Hydroxy und/oder Halogen mono-, di- oder trisubstituiertes Benzyl bedeutet, $R_2$ Wasserstoff oder Hydroxy darstellt, $R_1$, $R_1$', $R_2$' und $R_3$ Wasserstoff bedeuten, $R_4$ durch $C_1$-$C_4$-Alkyl, $C_1$-$C_4$-Alkoxy und/oder Halogen mono-, di- oder trisubstituiertes Phenyl- oder Diphenyl-$C_1$-$C_4$-alkyl oder unsubstituiertes oder durch Halogen der Atomnummer bis und mit 35 monosubstituiertes Naphthyl-$C_1$-$C_4$-alkyl darstellt und $R_5$ Wasserstoff, $C_1$-$C_4$-Alkyl oder eine Gruppe $R_4$ bedeutet, und ihre Salze.

4. Verbindungen gemäß Anspruch 1 der Formel I, worin $R_1$, $R_1$', $R_2$' und $R_3$ Wasserstoff bedeuten, $R_2$ Wasserstoff oder Hydroxy darstellt, $R_4$ durch $C_1$-$C_4$-Alkyl oder $C_1$-$C_4$-Alkoxy monosubstituiertes oder durch Halogen der Atomnummer bis und mit 35 mono- oder disubstituiertes Phenyl-$C_1$-$C_4$-alkyl oder unsubstituiertes oder durch Halogen der Atomnummer bis und mit 35 monosubstituiertes Naphthyl-,

Thienyl-, Furyl- oder Pyridyl-$C_1$-$C_4$-alkyl darstellt, $R_5$ Wasserstoff, $C_1$-$C_4$-Alkyl oder eine Gruppe $R_4$ bedeutet und R $C_3$-$C_7$-Alkyl $\alpha,\alpha$-Di-$C_1$-$C_4$-alkoxy-$C_1$-$C_4$-alkyl, $C_3$-$C_6$-Cycloalkyl-$C_1$-$C_4$-alkyl oder unsubstituiertes oder durch $C_1$-$C_4$-Alkyl, $C_1$-$C_4$-Alkoxy oder Halogen der Atomnummer bis und mit 35 substituiertes Benzyl bedeutet, und ihre Salze.

**5.** Verbindungen gemäß Anspruch 1 der Formel I, worin $R_1$, $R_1'$, $R_2'$ und $R_3$ Wasserstoff bedeuten, $R_2$ Wasserstoff oder Hydroxy darstellt, $R_4$ durch Halogen der Atomnummer bis und mit 35 mono- oder disubstituiertes $\alpha$-Phenyl-$C_1$-$C_4$-alkyl oder unsubstituiertes $\alpha$-Naphthyl-$C_1$-$C_4$-alkyl, $\alpha$-Thienyl-$C_1$-$C_4$-alkyl, $\alpha$-Furyl-$C_1$-$C_4$-alkyl oder $\alpha$-Pyridyl-$C_1$-$C_4$-alkyl darstellt, $R_5$ Wasserstoff, $C_1$-$C_4$-Alkyl oder eine Gruppe $R_4$ bedeutet und R $C_3$-$C_5$-Alkyl, $\alpha,\alpha$-Di-$C_1$-$C_4$-alkoxymethyl, $C_3$-$C_6$-Cycloalkyl-$C_1$-$C_4$-alkyl, $\alpha$-($C_3$-$C_6$-Cycloalkenyl)-$C_1$-$C_4$-alkyl, $\alpha$-(Mono-, Di- oder Trihydroxy-$C_3$-$C_6$-cycloalkyl)-$C_1$-$C_4$-alkyl oder Benzyl bedeutet, und ihre Salze.

**6.** Verbindungen gemäß Anspruch 1 der Formel I, worin $R_1$, $R_1'$, $R_2'$ und $R_3$ Wasserstoff bedeuten, $R_2$ Wasserstoff oder Hydroxy darstellt, $R_4$ durch Halogen der Atomnummer bis und mit 35 mono- oder disubstituiertes Phenyl-$C_1$-$C_4$-alkyl oder unsubstituiertes Naphthyl-, Thienyl-, Furyl- oder Pyridyl-$C_1$-$C_4$-alkyl darstellt, $R_5$ Wasserstoff, $C_1$-$C_4$-Alkyl oder eine Gruppe $R_4$ bedeutet und R $C_3$-$C_5$-Alkyl, $\alpha,\alpha$-Di-$C_1$-$C_4$-alkoxymethyl, $C_3$-$C_6$-Cycloalkyl-$C_1$-$C_4$-alkyl oder Benzyl bedeutet, und ihre Salze.

**7.** 3-(p-Chlorbenzylamino)propyl(diäthoxymethyl)phosphinsäure gemäß Anspruch 1 oder ein Salz davon.

**8.** 3-(p-Chlorbenzylamino)-2(S)-hydroxy-propyl(benzyl)phosphinsäure gemäß Anspruch 1 oder ein Salz davon.

**9.** 3-(p-Chlorbenzylamino)propyl(n-butyl)phosphinsäure gemäß Anspruch 1 oder ein Salz davon.

**10.** 3-(p-Chlorbenzylamino)propyl(cyclohexylmethyl)phosphinsäure gemäß Anspruch 1 oder ein Salz davon.

**11.** 3-(p-Chlorbenzylamino)-2(R)-hydroxy-propyl(benzyl)phosphinsäure gemäß Anspruch 1 oder ein Salz davon.

**12.** 3-(3,4-Dichlorbenzylamino)propyl(diäthoxymethyl)phosphinsäure gemäß Anspruch 1 oder ein Salz davon.

**13.** 3-[1-(p-Chlorphenyl)äthylamino]propyl(diäthoxymethyl)phosphinsäure gemäß Anspruch 1 oder ein Salz davon.

**14.** 3-(Naphthyl-1-ylmethylamino)propyl(diäthoxymethyl)phosphinsäure gemäß Anspruch 1 oder ein Salz davon.

**15.** 3-(3,4-Dichlorbenzylamino)propyl(cyclohexylmethyl)phosphinsäure gemäß Anspruch 1 oder ein Salz davon.

**16.** 3-(Pyrid-2-ylmethylamino)-2(S)-hydroxy-propyl(benzyl)phosphinsäure gemäß Anspruch 1 oder ein Salz davon.

**17.** 3-(p-Chlorbenzylaminoyl)-2(S)-hydroxy-propyl(cyclohexylmethyl)phosphinsäure gemäß Anspruch 1 oder ein Salz davon.

**18.** 3-(3,4-Dichlorbenzylaminoyl)-2(S)-hydroxy-propyl(benzyl)phosphinsäure gemäß Anspruch 1 oder ein Salz davon.

**19.** 3-(p-Fluorbenzylamino)propyl(diäthoxymethyl)phosphinsäure gemäß Anspruch 1 oder ein Salz davon.

**20.** 3-(4-Chlor-3-trifluomethyl-benzylamino)propyl(diäthoxymethyl)phosphinsäure gemäß Anspruch 1 oder ein Salz davon.

**21.** 3-(m-Chlorbenzylamino)propyl(diäthoxymethyl)phosphinsäure gemäß Anspruch 1 oder ein Salz davon.

**22.** 3-(3,4-Dichlorbenzylamino)-2(S)-hydroxy-propyl(cyclohexylmethyl)phosphinsäure gemäß Anspruch 1 oder ein Salz davon.

**23.** 3-[1-(3,4-Dichlorphenyl)äthylamino]propyl(diäthoxymethyl)phosphinsäure gemäß Anspruch 1 oder ein Salz davon.

**24.** 3-[1-(p-Chlorphenyl)äthylamino]-2(S)hydroxy-propyl(benzyl)phosphinsäure gemäß Anspruch 1 oder ein Salz davon.

**25.** 3-(p-Jodbenzylamino)propyl(diäthoxymethyl)phosphinsäure gemäß Anspruch 1 oder ein Salz davon.

**26.** 3-(2,4-Dichlorbenzylamino)propyl(diäthoxymethyl)phosphinsäure gemäß Anspruch 1 oder ein Salz davon.

**27.** 3-(o-Chlorbenzylamino)propyl(diäthoxymethyl)phosphinsäure gemäß Anspruch 1 oder ein Salz davon.

**28.** 3-(3,4-Dichlorbenzylamino)propyl(cyclopropylmethyl)phosphinsäure gemäß Anspruch 1 oder ein Salz davon.

**29.** 3-(p-Chlorbenzylamino)propyl(tetrahydrofuran-2-yl)phosphinsäure gemäß Anspruch 1 oder ein Salz davon.

**30.** 3-[N-(p-Chlorbenzyl)-N-methyl-amino]propyl(diäthoxymethyl)phosphinsäure gemäß Anspruch 1 oder ein Salz davon.

**31.** 3-(p-Chlorbenzylamino)-2(S)-hydroxy-propyl(p-methylbenzyl)phosphinsäure gemäß Anspruch 1 oder ein Salz davon.

**32.** 3-(3,4-Dichlorbenzylamino)-2(S)-hydroxy-propyl(p-methylbenzyl)phosphinsäure gemäß Anspruch 1 oder ein Salz davon.

**33.** 3-(p-Chlorbenzylamino)-2(S)-hydroxy-propyl(p-chlorbenzyl)phosphinsäure gemäß Anspruch 1 oder ein Salz davon.

**34.** 3-(3,4-Dichlorbenzylamino)-2(S)hydroxy-propyl(p-chlorbenzyl)phosphinsäure gemäß Anspruch 1 oder ein Salz davon.

**35.** 3-(p-Chlorbenzylamino)-2(S)-hydroxy-propyl(diäthoxymethyl)phosphinsäure;
3-[1-(p-Chlorphenyl)äthylamino]-2(S)-hydroxy-propyl(diäthoxymethyl)phosphinsäure;
3-(3,4-Dichlorbenzylamino)-2(S)-hydroxy-propyl(diäthoxymethyl)phosphinsäure;
3-[1-(3,4-Dichlorphenyl)äthylamino]-2(S)-hydroxy-propyl(diäthoxymethyl)phosphinsäure;
3-[N-(p-Chlorbenzyl)-N-methyl-amino]-2(S)-hydroxy-propyl(benzyl)phosphinsäure;
3-(p-Chlorbenzylamino)-2(S)-hydroxy-propyl(p-methoxybenzyl)phosphinsäure;
3-(p-Chlorbenzylamino)-2(S)-hydroxy-propyl(3,4-dimethoxybenzyl)phosphinsäure;
3-(3,4-Dichlorbenzylamino)-2(S)-hydroxy-propyl(3,4-dimethoxybenzyl)phosphinsäure;
3-[2-(p-Chlorphenyl)prop-2-ylamino]propyl(diäthoxymethyl)phosphinsäure;
3-[2-(3,4-Dichlorphenyl)prop-2-ylamino]propyl(diäthoxymethyl)phosphinsäure;
3-[2-(p-Chlorphenyl)prop-2-ylamino]-2(S)-hydroxy-propyl(benzyl)phosphinsäure;
3-[2-(3,4-Dichlorphenyl)prop-2-ylamino)-2(S)-hydroxy-propyl(benzyl)phosphinsäure;
3-(3,4-Dichlorbenzylamino)-2(S)-hydroxy-propyl(3,4,5-trihydroxycyclohexlmethyl)phosphinsäure;
3-(4-Chlor-3-methoxy-benzylamino)propyl(diäthoxymethyl)phosphinsäure;
3-(4-Chlor-3-methoxy-benzylamino)-2(S)-hydroxy-propyl(benzyl)phosphinsäure;
3-(2-Phenyläthylamino)propyl(diäthoxymethyl)phosphinsäure;
3-(2-Phenyläthylamino)-2(S)-hydroxy-propyl(diäthoxymethyl)phosphinsäure;
3-(p-Chlorbenzylamino-2(S)-hydroxy-propyl(4-methoxycyclohexyl)phosphinsäure,
3-(3,4-Dichlor-6-jod-benzylamino)-2(S)-hydroxy-propyl(benzyl) phosphinsäure;
3-(3,4-Dichlor-6-jod-benzylamino)propyl(diäthoxymethyl)phosphinsäure;
3-(4-Chlor-3-jod-benzylamino)propyl(diäthoxymethyl)phosphinsäure;

3-(3-Chlor-4-jod-benzylamino)propyl(diäthoxymethyl)phosphinsäure;
3-(4-Chlor-3-jod-benzylamino)-2(S)-hydroxy-propyl(benzyl)phosphinsäure;
3-(3-Chlor-4-jod-benzylamino)-2(S)-hydroxy-propyl(benzyl)phosphinsäure;
3-Di(p-chlorbenzyl)aminopropyl(diäthoxymethyl)phosphinsäure;
3-Di(3,4-dichlorbenzyl)amino-2(S)-hydroxy-propyl(benzyl)phosp hinsäure;
3-(m-Chlorbenzylamino)-2(S)-hydroxy-propyl(benzyl)phosphinsäure;
3-(3,4-Dichlorbenzyl)amino-2(S)-hydroxy-propyl(cis-4,5-dihydroxycyclohexylmethyl)phosphinsäure;
3-(3,4-Dichlorbenzyl)amino-2(S)-hydroxy-propyl(cyclohex-3-enylmethyl)phosphinsäure;
3-[1-(3,4-Dichlorphenyl)äthylamino]-2(S)-hydroxy-propyl(cyclohex-3-enylmethyl)phosphinsäure oder
3-[1-(3,4-Dichlorphenyl)äthylamino]-2(S)-hydroxy-propyl(cis-4,5-dihydroxycyclohexylmethyl)-phosphinsäure
gemäß Anspruch 1 oder jeweils ein Salz davon.

**36.** Eine Verbindung der Formel I

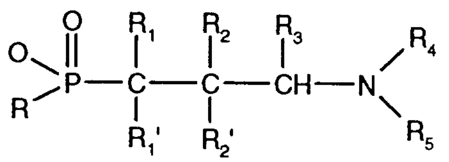

(I),

worin $R_1$, $R_1'$, $R_2'$ und $R_3$ Wasserstoffbedeuten, $R_2$ Wasserstoff oder Hydroxy darstellt, $R_4$ einen unsubstituierten oder im Phenyl- bzw. Naphthylteil durch $C_1$-$C_4$-Alkyl, $C_1$-$C_4$-Alkoxy, Hydroxy und/oder Halogen mono-, di- oder trisubstituierten Phenyl-$C_1$-$C_4$-alkyl-, Diphenyl-$C_1$-$C_4$-alkyl- oder Naphthyl-$C_1$-$C_4$-alkylrest oder einen unsubstituierten oder im Thienyl-, Furyl- bzw. Pyridylteil durch Halogen substituierten Thienyl-, Furyl- oder Pyridyl-$C_1$-$C_4$-alkylrest bedeutet, $R_5$ Wasserstoff, $C_1$-$C_4$-Alkyl oder eine Gruppe $R_4$ darstellt und R $C_2$-$C_4$-Alkyl, $C_2$-$C_4$-Alkenyl, $C_2$-$C_7$-Alkinyl, Oxo-$C_2$-$C_7$-alkyl, Hydroxy-$C_2$-$C_7$-alkyl oder Dihydroxy-$C_2$-$C_7$-alkyl, $\alpha$-Hydroxy-$C_3$-$C_5$-alkenyl, Mono-, Di- oder Trifluor-$C_2$-$C_5$-alkyl, Mono-, Di- oder Trifluor-$C_2$-$C_5$-alkenyl, Mono-, Di- oder Trifluor-$\alpha$-hydroxy-$C_2$-$C_5$-alkyl, Mono-, Di- oder Trifluor-$\alpha$-hydroxy-$C_2$-$C_5$-alkenyl, $C_1$-$C_4$-Alkoxy-$C_1$-$C_4$-alkyl, Di-$C_1$-$C_4$-alkoxy-$C_1$-$C_4$-alkyl, $C_1$-$C_4$-Alkoxy $C_1$-$C_4$-Alkoxy-$C_2$-$C_7$-(hydroxy)-alkyl, $C_1$-$C_4$-Alkoxy-$C_2$-$C_5$-(halo)-alkyl, $C_1$-$C_4$-Alkylthio-$C_1$-$C_4$-alkyl, Di-$C_1$-$C_4$-alkylthio-$C_1$-$C_4$-alkyl, $C_3$-$C_8$-Cycloalkyl, $\alpha$-Hydroxy-$C_3$-$C_6$-cycloalkyl, Oxa- oder Thia-$C_3$-$C_6$-cycloalkyl, 1,3-Dioxa- oder 1,3-Dithia-$C_3$-$C_8$-cycloalkyl, $C_3$-$C_8$-Cycloalkyl-$C_1$-$C_4$-alkyl, $C_3$-$C_8$-Cycloalkenyl-$C_1$-$C_4$-alkyl, Mono-, Di- oder Trihydroxy-$C_3$-$C_8$-cycloalkenyl, $C_3$-$C_6$-Cycloalkyl-(hydroxy)-$C_1$-$C_4$-alkyl, 1-($C_1$-$C_4$-Alkylthio-$C_3$-$C_6$-cycloalkyl)-1-hydroxy)-$C_1$-$C_4$-alkyl, unsubstituiertes oder durch $C_1$-$C_4$-Alkyl, $C_1$-$C_4$-Alkoxy, Halogen, Hydroxy und/oder Trifluormethyl mono-, di- oder trisubstituiertes Mono- oder Diphenyl-$C_1$-$C_4$-alkyl, Naphthyl-$C_1$-$C_4$-alkyl 1 oder unsubstituiertes oder durch Halogen substituiertes Thienyl-, Furyl- oder Pyridyl-$C_1$-$C_4$-alkyl bedeutet, in freier Form oder in pharmazeutisch verwendbarer Salzform zur Anwendung in einem Verfahren zur therapeutischen Behandlung des menschlichen oder tierischen Körpers.

**37.** Verbindungen gemäß einem der Ansprüche 2, 4 und 6 bis 11 zur Anwendung gemäß Anspruch 36.

**38.** Verbindungen gemäß einem der Ansprüche 1, 3, 5 und 12 bis 35 zur Anwendung Anwendung gemäß Anspruch 36.

**39.** Pharmazeutische Präparate, als pharmazeutischen Wirkstoff enthaltend eine Verbindung gemäß Anspruch 36 in freier Form oder in pharmazeutisch verwendbarer Salzform.

**40.** Pharmazeutische Präparate gemäß Anspruch 36, als pharmazeutischen Wirkstoff enthaltend eine Verbindung gemäß einem der Ansprüche 2, 4 und 6 bis 11 in freier Form oder in pharmazeutisch verwendbarer Salzform.

**41.** Pharmazeutische Präparate gemäß Anspruch 36, als pharmazeutischen Wirkstoff enthaltend eine Verbindung gemäß einem der Ansprüche 1, 3, 5 und 12 bis 35 in freier Form oder in pharmazeutisch verwendbarer Salzform.

**42.** Pharmazeutische Präparate gemäß einem der Ansprüche 39 bis 41 für die Behandlung von Epilepsien des "petit mal"-Formenkreises sowie zur Unterdrückung von "petit mal"-ähnlichen Zuständen, die bei der Behandlung mit Phenytoin, Carbamazepin, Vigabatrin® und Antiepileptika mit gleichem oder ähnlichen Wirkungsprofil auftreten können.

**43.** Verfahren zur Herstellung von neuen araliphatisch N-substituierten Aminoalkanphosphinsäuren gemäß Anspruch 1, dadurch gekennzeichnet, dass man in einer Verbindung der Formel II

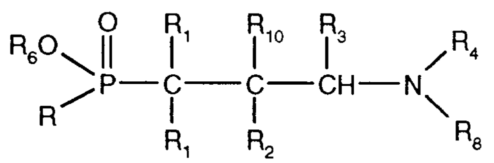

(II),

worin $R_1$', $R_2$' und $R_3$ Wasserstoffbedeuten, $R_6$ für eine Hydroxyschutzgruppe, $R_8$ für eine Gruppe $R_5$ oder für eine Aminoschutzgruppe und $R_{10}$ für Wasserstoff oder geschütztes Hydroxy steht, wobei R, $R_1$, $R_4$ und $R_5$ die angegebene Bedeutung haben, oder in einem Salz davon die Hydroxygruppen durch Ersatz der Hydroxyschutzgruppe $R_6$ durch Wasserstoff freisetzt und gegebenenfalls die Aminoschutzgruppe $R_8$ abspaltet und gegebenenfalls die Hydroxygruppen $R_2$ aus der geschützten Hydroxygruppe $R_{10}$ freisetzt und gewünschtenfalls eine erhaltene Verbindung in eine andere Verbindung der Formel I überführt, ein verfahrensgemäß erhältliches Isomerengemisch in die Komponenten auftrennt und das jeweils bevorzugte Isomere abtrennt und/oder eine verfahrensgemäß erhältliche freie Verbindung in ein Salz oder eine verfahrensgemäß erhältliches Salz in die entsprechende freie Verbindung überführt.

**44.** Verwendung von Verbindungen mit $GABA_B$-antagonistischen Eigenschaften zur Herstellung eines pharmazeutischen Präparates für die Behandlung von Epilepsien des "petit mal"-Formenkreises oder zur Unterdrückung von "petit mal"-ähnlichen Zuständen, die bei der Behandlung mit Phenytoin, Carbamazepin, Vigabatrin® und Antiepileptika mit gleichem oder ähnlichen Wirkungsprofil auftreten können.

**45.** Verwendung gemäß Anspruch 44 zur Herstellung eines für die Behandlung von Epilepsien des "petit mal"-Formenkreises.

**46.** Verwendung gemäß Anpruch 44 zur Herstellung von pharmazeutischen Präparaten, als pharmazeutischen Wirkstoff enthaltend eine Verbindung der Formel I

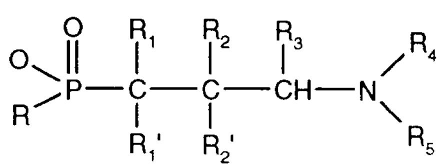

(I),

worin einer der Reste $R_1$, $R_2$ und $R_3$ Wasserstoff $C_1$-$C_7$-Aalkyl, $C_2$-$C_7$-Aalkenyl, $C_2$-$C_7$-Alkinyl, $C_3$-$C_8$-Cycloalkyl, $C_3$-$C_8$-Cycloalkenyl-$C_1$-$C_4$-alkyl, Mono-, Di- oder Trihydroxy-$C_3$-$C_8$-cycloalkyl-$C_1$-$C_4$-alkyl, unsubstituiertes oder im Phenyl- bzw. Naphthylteil durch $C_1$-$C_4$-Alkyl, $C_1$-$C_4$-Alkoxy, Halogen und/oder Trifluormethyl ein- oder mehrfach substituiertes Phenyl- oder Naphthyl-$C_1$-$C_4$-alkyl, Diphenyl-$C_1$-$C_4$-alkyl oder Naphthyl-$C_1$-$C_4$-ralkyl, unsubstituiertes oder durch Halogen, $C_1$-$C_4$-Alkyl, $C_1$-$C_4$-Alkoxy und/oder Trifluormethyl substituiertes Phenyl oder Naphthyl, ein anderer Wasserstoff oder im Falle von $R_1$ oder $R_2$ Hydroxy oder im Falle von $R_1$ Halogen oder im Falle von $R_2$ gemeinsam mit $R_2$' Oxo bedeutet und der verbleibende Wasserstoff bedeutet, $R_1$' Wasserstoff oder Halogen ist, $R_2$' Wasserstoff, Hydroxy oder gemeinsam mit $R_2$ Oxo darstellt, $R_4$ und $R_5$ Wasserstoff darstellen oder $R_4$ unsubstituiertes oder im Phenyl-, Naphthyl-, Thienyl-, Furyl- oder Pyridylteil $C_1$-$C_4$-Alkyl, $C_1$-$C_4$-Alkoxy, Halogen und/oder Trifluormethyl ein- oder mehrfach substituiertes Phenyl-$C_1$-$C_4$-alkyl, Diphenyl-$C_1$-$C_4$-alkyl, Naphthyl-$C_1$-$C_4$-alkyl, Thienyl-$C_1$-$C_4$-alkyl, Furyl-$C_1$-$C_4$-alkyl oder Pyridyl-$C_1$-$C_4$-alkyl bedeutet und $R_5$ für Wasserstoff, , $C_1$-$C_7$-Aalkyl, $C_2$-$C_7$-Aalkenyl, $C_2$-$C_7$-Alkinyl oder eine Gruppe $R_4$ steht und R $C_2$-$C_7$-Alkyl, , $C_2$-$C_7$-Aalkenyl, $C_2$-$C_7$-Alkinyl, $C_3$-$C_8$-Cycloalkyl, $C_3$-$C_8$-Cycloalkyl-$C_1$-$C_4$-alkyl, $C_3$-$C_8$-

Cycloalkenyl-$C_1$-$C_4$-alkyl,, Mono-, Di- oder Trihydroxy-$C_3$-$C_8$-cycloalkyl-$C_1$-$C_4$-alkyl, $C_3$-$C_8$-Cycloalkyl-(hydroxy)-$C_1$-$C_4$-alkyl, ($C_1$-$C_4$-Alkylthio)cycloalkyl(hydroxy)-$C_1$-$C_4$-alkyl, unsubstituiertes oder im Phenyl- bzw. Naphthylteil durch $C_1$-$C_4$-Alkyl, $C_1$-$C_4$-Alkoxy, Halogen und/oder Trifluormethyl ein- oder mehrfach substituiertes Phenyl- oder Naphthyl-$C_1$-$C_4$-niederalkyl, unsubstituiertes oder durch Halogen, $C_1$-$C_4$-Alkyl, $C_1$-$C_4$-Alkoxy und/oder Trifluormethyl substituiertes Phenyl oder Naphthyl oder, sofern $R_1$ Wasserstoff oder Hydroxy, $R_2$ einen wie vorstehend definierten aromatischen Rest und $R_1'$, $R_2'$ sowie $R_3$ Wasserstoff darstellen, Methyl bedeutet, oder ein pharmazeutisch verwendbares Salz davon.

**47.** Verwendung gemäß Anpruch 44 zur Herstellung von pharmazeutischen Präparaten, als pharmazeutischen Wirkstoff enthaltend eine Verbindung der Formel I, worin einer der Reste $R_1$, $R_2$ und $R_3$ Wasserstoff, $C_1$-$C_4$-Alkyl, $C_3$-$C_6$-Cycloalkyl, unsubstituiertes oder im Phenyl- bzw. Naphthylteil durch $C_1$-$C_4$-Alkyl, $C_1$-$C_4$-Alkoxy, Halogen und/oder Trifluormethyl ein- oder mehrfach substituiertes Phenyl-$C_1$-$C_4$-alkyl, unsubstituiertes oder im Phenyl- bzw. Naphthylteil durch $C_1$-$C_4$-Alkyl, $C_1$-$C_4$-Alkoxy, Halogen und/oder Trifluormethyl ein- oder mehrfach substituiertes Phenyl, ein anderer Wasserstoff oder im Falle von $R_1$ oder $R_2$ Hydroxy und der verbleibende Wasserstoffbedeutet, $R_1'$ Wasserstoff ist, $R_2'$ Wasserstoff, Hydroxy oder gemeinsam mit $R_2$ Oxo darstellt, $R_4$ und $R_5$ Wasserstoff darstellen oder $R_4$ einen unsubstituierten oder im Phenylteil durch $C_1$-$C_4$-Alkyl, $C_1$-$C_4$-Alkoxy, Halogen und/oder Trifluormethyl ein- oder mehrfach substituierten Phenyl-$C_1$-$C_4$-alkylrest bedeutet und $R_5$ für Wasserstoff, $C_1$-$C_4$-Alkyl oder eine Gruppe $R_4$ steht und R $C_2$-$C_7$-Alkyl, $\alpha,\alpha$-Di-$C_1$-$C_4$-alkoxy-$C_2$-$C_7$-alkyl, $\alpha,\alpha$-Dihalogen-$C_2$-$C_7$-alkyl, $C_3$-$C_6$-Cycloalkyl, $C_3$-$C_6$-Cycloalkyl-$C_1$-$C_4$-alkyl, $C_3$-$C_8$-Cycloalkenyl-$C_1$-$C_4$-alkyl, Mono-, Di- oder Trihydroxy-$C_3$-$C_8$-cycloalkyl-$C_1$-$C_4$-alkyl, Benzyl oder, sofern $R_2$ Halogenphenyl und $R_1$, $R_1'$, $R_2'$ sowie $R_3$ Wasserstoff darstellen, Methyl bedeutet, oder ein pharmazeutisch verwendbares Salz davon.

**48.** Verwendung gemäß Anpruch **44** zur Herstellung von pharmazeutischen Präparaten, als pharmazeutischen Wirkstoff enthaltend eine Verbindung der Formel I, worin

a) $R_1$, $R_1'$, $R_2'$, $R_3$, $R_4$ und $R_5$ für Wasserstoff stehen, $R_2$ $C_1$-$C_4$-Alkyl, $C_3$-$C_6$-Cycloalkyl, Benzyl, Phenyl, Halogenphenyl oder Naphthyl und $R_2'$ Wasserstoff oder Hydroxy oder $R_2$ Hydroxy und $R_2'$ Wasserstoff bzw. $R_2$ und $R_2'$ gemeinsam eine Oxogruppe bedeuten, oder

b) $R_1$ für Hydroxy, $C_1$-$C_4$-Alkyl, $C_3$-$C_6$-Cycloalkyl, Benzyl, Phenyl, Halogenphenyl oder Naphthyl steht und $R_1'$, $R_2$, $R_3$, $R_4$ und $R_5$ Wasserstoff bedeuten oder

c) $R_1$ für Halogen steht, wobei $R_1'$ Wasserstoff oder Halogen und $R_2$, $R_2'$, $R_3$, $R_4$ und $R_5$ Wasserstoff bedeuten und

R $C_2$-$C_7$-Alkyl, $\alpha,\alpha$-Di-$C_1$-$C_4$-alkoxy-$C_2$-$C_7$-alkyl, $\alpha,\alpha$-Dihalogen-$C_2$-$C_7$-alkyl, $C_3$-$C_6$-Cycloalkyl, $C_3$-$C_6$-Cycloalkyl-$C_1$-$C_4$-alkyl, Benzyl oder, sofern oder, sofern $R_2$ Halogenphenyl oder Naphthyl und $R_1$, $R_1'$, $R_2'$ sowie $R_3$ Wasserstoff darstellen, Methyl bedeutet, oder ein pharmazeutisch verwendbares Salz davon.

**49.** Verwendung gemäß Anpruch 44 zur Herstellung von pharmazeutischen Präparaten, als pharmazeutischen Wirkstoff enthaltend eine Verbindung der Formel I, worin $R_1$, $R_1'$, $R_2'$ und $R_3$ Wasserstoff bedeuten, $R_2$ für Wasserstoff oder Hydroxy steht, $R_4$ und $R_5$ Wasserstoff darstellen oder $R_4$ einen unsubstituierten oder im Phenylteil durch $C_1$-$C_4$-Alkyl, $C_1$-$C_4$-Alkoxy, Halogen und/oder Trifluormethyl ein- oder mehrfach substituierten Phenyl-$C_1$-$C_4$-alkylrest bedeutet und $R_5$ für Wasserstoff oder $C_1$-$C_4$-Alkyl steht und R $C_2$-$C_7$-Alkyl, $\alpha,\alpha$-Di-$C_1$-$C_4$-alkoxy-$C_1$-$C_4$-alkyl, $\alpha,\alpha$-Dihalogen-$C_2$-$C_7$-alkyl, $C_3$-$C_6$-Cycloalkyl, $C_3$-$C_6$-Cycloalkyl-$C_1$-$C_4$-alkyl, a-($C_3$-$C_6$-Cycloalkenyl)-$C_1$-$C_4$-alkyl, a-(Mono-, Di- oder Trihydroxy-$C_3$-$C_6$-cycloalkyl)-$C_1$-$C_4$-alkyl oder einen unsubstituierten oder im Phenylteil durch $C_1$-$C_4$-Alkyl, $C_1$-$C_4$-Alkoxy, Halogen und/oder Trifluormethyl ein- oder mehrfach substituierten Phenyl-$C_1$-$C_4$-alkylrest bedeutet, oder ein pharmazeutisch verwendbares Salz davon.

**50.** Verwendung gemäß Anpruch 45 zur Herstellung von pharmazeutischen Präparaten, als pharmazeutischen Wirkstoff enthaltend eine Verbindung der Formel I, worin $R_1$, $R_1'$, $R_2'$, $R_3$, $R_4$ und $R_5$ Wasserstoff bedeuten, $R_2$ für Wasserstoff oder Hydroxy steht und R $C_2$-$C_7$-Alkyl, $\alpha,\alpha,$-Di-$C_1$-$C_4$-alkoxy-$C_1$-$C_4$-alkyl, $\alpha,\alpha,$-Dihalogen-$C_2$-$C_7$-alkyl, $C_3$-$C_6$-Cycloalkyl, $C_3$-$C_6$-Cycloalkyl-$C_1$-$C_4$-alkyl oder Benzyl bedeutet, oder ein pharmazeutisch verwendbares Salz davon.

**51.** Verwendung gemäß Anpruch 44 zur Herstellung von pharmazeutischen Präparaten, als pharmazeutischen Wirkstoff enthaltend 3-Aminopropyl(cyclohexylmethyl)phosphinsäure oder ein pharmazeutisch verwendbares Salz davon.

**52.** Verwendung gemäß Anpruch 44 zur Herstellung von pharmazeutischen Präparaten, als pharmazeutischen Wirkstoffenthaltend 3-Aminopropyl(n-butyl)phosphinsäure oder ein pharmazeutisch verwendbares Salz davon.

**53.** Verwendung gemäß Anpruch 44 zur Herstellung von pharmazeutischen Präparaten, als pharmazeutischen Wirkstoff enthaltend 3-Aminopropyl(diäthoxymethyl)phosphinsäure oder ein pharmazeutisch verwendbares Salz davon.

**54.** Verwendung gemäß Anpruch 44 zur Herstellung von pharmazeutischen Präparaten, als pharmazeutischen Wirkstoff enthaltend 3-Aminopropyl(benzyl)phosphinsäure oder ein pharmazeutisch verwendbares Salz davon.

**55.** Verwendung gemäß Anpruch 44 zur Herstellung von pharmazeutischen Präparaten, als pharmazeutischen Wirkstoff enthaltend 3-Aminopropyl(1,1-difluorbutyl)phosphinsäure oder ein pharmazeutisch verwendbares Salz davon.

**56.** Verwendung gemäß Anpruch 44 zur Herstellung von pharmazeutischen Präparaten, als pharmazeutischen Wirkstoff enthaltend 3-Amino-2-hydroxy-propyl(cyclohexylmethyl)phosphinsäure oder ein pharmazeutisch verwendbares Salz davon.

**57.** Verwendung gemäß Anpruch 44 zur Herstellung von pharmazeutischen Präparaten, als pharmazeutischen Wirkstoff enthaltend 3-Aminopropyl(cyclohexylmethyl)phosphinsäure oder ein pharmazeutisch verwendbares Salz davon.

**58.** Verwendung gemäß Anpruch 44 zur Herstellung von pharmazeutischen Präparaten, als pharmazeutischen Wirkstoff enthaltend 3-Amino-2-(p-chlorphenyl)-propyl(methyl)phosphinsäure oder ein pharmazeutisch verwendbares Salz davon.

**59.** Verwendung gemäß Anpruch 44 zur Herstellung von pharmazeutischen Präparaten, als pharmazeutischen Wirkstoff 3-Aminopropyl(n-butyl)phosphinsäure oder ein pharmazeutisch verwendbares Salz davon.

**60.** Verwendung gemäß Anpruch 44 zur Herstellung von pharmazeutischen Präparaten, als pharmazeutischen Wirkstoff enthaltend 3-Aminopropyl(diäthoxymethyl)phosphinsäure oder ein pharmazeutisch verwendbares Salz davon.

**61.** Verwendung gemäß Anpruch 44 zur Herstellung von pharmazeutischen Präparaten, als pharmazeutischen Wirkstoff enthaltend 3-Aminopropyl(benzyl)phosphinsäure oder ein pharmazeutisch verwendbares Salz davon.

**62.** Verwendung gemäß Anpruch 44 zur Herstellung von pharmazeutischen Präparaten, als pharmazeutischen Wirkstoff enthaltend 3-Aminopropyl(1,1-difluorbutyl)phosphinsäure oder ein pharmazeutisch verwendbares Salz davon.

**63.** Verwendung gemäß Anpruch 44 zur Herstellung von pharmazeutischen Präparaten, als pharmazeutischen Wirkstoff enthaltend 3-Amino-2-hydroxy-propyl(cyclohexylmethyl)phosphinsäure oder ein pharmazeutisch verwendbares Salz davon.

**64.** Verwendung gemäß Anpruch 44 zur Herstellung von pharmazeutischen Präparaten, als pharmazeutischen Wirkstoff enthaltend 3-Amino-2(R)-hydroxy-propyl(cyclohexylmethyl)phosphinsäure oder ein pharmazeutisch verwendbares Salz davon.

**Patentansprüche für folgende Vertragsstaaten : ES, GR**

**1.** Verfahren zur Herstellung von neuen araliphatisch N-substituierten Aminoalkanphosphinsäuren der Formel I

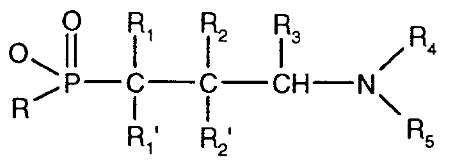

(I),

worin $R_1$, $R_1'$, $R_2'$ und $R_3$ Wasserstoff bedeuten, $R_2$Wasserstoff oder Hydroxy darstellt, $R_4$ einen im Phenyl- bzw. Naphthylteil durch $C_1$-$C_4$-Alkyl, $C_1$-$C_4$-Alkoxy, Hydroxy und/oder Halogen mono-, di- oder trisubstituierten Phenyl-$C_1$-$C_4$-alkyl-, Diphenyl-$C_1$-$C_4$-alkyl- oder Naphthyl-$C_1$-$C_4$-alkylrest oder einen unsubstituierten oder im Thienyl-, Furyl- bzw. Pyridylteil durch Halogen substituierten Thienyl-, Furyl- oder Pyridyl-$C_1$-$C_4$-alkylrest bedeutet, $R_5$ Wasserstoff, $C_1$-$C_4$-Alkyl oder eine Gruppe $R_4$ darstellt und R $C_2$-$C_4$-Alkyl, $C_2$-$C_4$-Alkenyl, $C_2$-$C_7$-Alkinyl, Oxo-$C_2$-$C_7$-alkyl, Hydroxy-$C_2$-$C_7$-alkyl oder Dihydroxy-$C_2$-$C_7$-alkyl, $\alpha$-Hydroxy-$C_3$-$C_5$-alkenyl, Mono-, Di- oder Trifluor-$C_2$-$C_5$-alkyl, Mono-, Di- oder Trifluor-$C_2$-$C_5$-alkenyl, Mono-, Di- oder Trifluor-$\alpha$-hydroxy-$C_2$-$C_5$-alkyl, Mono-, Di- oder Trifluor-$\alpha$-hydroxy-$C_2$-$C_5$-alkenyl, $C_1$-$C_4$-Alkoxy-$C_1$-$C_4$-alkyl, Di-$C_1$-$C_4$-alkoxy-$C_1$-$C_4$-alkyl, $C_1$-$C_4$-Alkoxy $C_1$-$C_4$-Alkoxy-$C_2$-$C_7$-(hydroxy)-alkyl, $C_1$-$C_4$-Alkoxy-$C_2$-$C_5$-(halo)-alkyl, $C_1$-$C_4$-Alkylthio-$C_1$-$C_4$-alkyl, Di-$C_1$-$C_4$-alkylthio-$C_1$-$C_4$-alkyl, $C_3$-$C_8$-Cycloalkyl, $\alpha$-Hydroxy-$C_3$-$C_6$-cycloalkyl, Oxa- oder Thia-$C_3$-$C_6$-cycloalkyl, 1,3-Dioxa- oder 1,3-Dithia-$C_3$-$C_8$-cycloalkyl, $C_3$-$C_8$-Cycloalkyl-$C_1$-$C_4$-alkyl, $C_3$-$C_8$-Cycloalkenyl-$C_1$-$C_4$-alkyl, Mono-, Di- oder Trihydroxy-$C_3$-$C_8$-cycloalkenyl, $C_3$-$C_6$-Cycloalkyl-(hydroxy)-$C_1$-$C_4$-alkyl, 1-($C_1$-$C_4$-Alkylthio-$C_3$-$C_6$-cycloalkyl)-1-hydroxy)-$C_1$-$C_4$-alkyl, unsubstituiertes oder durch $C_1$-$C_4$-Alkyl, $C_1$-$C_4$-Alkoxy, Halogen, Hydroxy und/oder Trifluormethyl mono-, di- oder trisubstituiertes Mono- oder Diphenyl-$C_1$-$C_4$-alkyl, Naphthyl-$C_1$-$C_4$-alkyl 1 oder unsubstituiertes oder durch Halogen substituiertes Thienyl-, Furyl- oder Pyridyl-$C_1$-$C_4$-alkyl bedeutet, und ihrer Salze, dadurch gekennzeichnet, dass man in einer Verbindung der Formel II

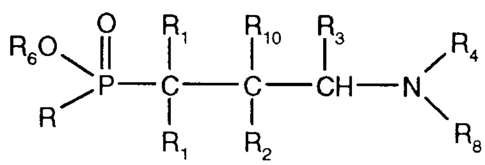

(II),

worin $R_1$, $R_1'$, $R_2'$ und $R_3$ Wasserstoffbedeuten, $R_6$ für eine Hydroxyschutzgruppe, $R_8$ für eine Gruppe $R_5$ oder für eine Aminoschutzgruppe und $R_{10}$ für Wasserstoff oder geschütztes Hydroxy steht, wobei R, $R_1$, $R_4$ und $R_5$ die angegebene Bedeutung haben, oder in einem Salz davon die Hydroxygruppen durch Ersatz der Hydroxyschutzgruppe $R_6$durch Wasserstoff freisetzt und gegebenenfalls die Aminoschutzgruppe $R_8$ abspaltet und gegebenenfalls die Hydroxygruppen $R_2$ aus der geschützten Hydroxygruppe $R_{10}$ freisetzt und gewünschtenfalls eine erhaltene Verbindung in eine andere Verbindung der Formel I überführt, ein verfahrensgemäß erhältliches Isomerengemisch in die Komponenten auftrennt und das jeweils bevorzugte Isomere abtrennt und/oder eine verfahrensgemäß erhältliche freie Verbindung in ein Salz oder eine verfahrensgemäß erhältliches Salz in die entsprechende freie Verbindung überführt.

2. Verfahren gemäß Anspruch 1 zur Herstellung von Verbindungen der Formel I, worin worin $R_1$, $R_1'$, $R_2'$ und $R_3$ Wasserstoff bedeuten, $R_2$ Wasserstoff oder Hydroxy darstellt, $R_4$ einen durch $C_1$-$C_4$-Alkyl, $C_1$-$C_4$-Alkoxy, Hydroxy und/oder Halogen substituierten Phenyl- oder Naphthyl-$C_1$-$C_4$-alkylrest oder einen unsubstituierten oder durch durch Halogen substituierten Thienyl-, Furyl- oder Pyridyl-$C_1$-$C_4$-alkylrest bedeutet, $R_5$ Wasserstoff, Niederalkyl oder eine Gruppe $R_4$ darstellt und R $C_2$-$C_4$-Alkyl, $C_2$-$C_4$-Alkenyl, $C_2$-$C_7$-Alkinyl, Oxo-$C_2$-$C_7$-alkyl Hydroxy-$C_2$-$C_7$-alkyl oder Dihydroxy-$C_2$-$C_7$-alkyl, $\alpha$-Hydroxy-$C_3$-$C_5$-alkenyl. Mono-, Di- oder Mono-, Di- oder Trifluor-$C_2$-$C_5$-alkyl, Mono-, Di- oder Trifluor-$C_2$-$C_5$-alkenyl, Mono-, di- oder Trifluor-$\alpha$-hydroxy-$C_2$-$C_5$-alkyl, Mono-, Di- oder Trifluor-$\alpha$-hydroxy-$C_2$-$C_5$-alkenyl, $C_1$-$C_4$-Alkoxy-$C_1$-$C_4$-alkyl, Di-$C_1$-$C_4$-alkoxy-$C_1$-$C_4$-alkyl, $C_1$-$C_4$-Alkoxy-$C_2$-$C_7$-(hydroxy)-alkyl, , $C_1$-$C_4$-Alkoxy-$C_2$-$C_5$-(halo)-alkyl, $C_1$-$C_4$-Alkylthio-$C_1$-$C_4$-alkyl, Di-$C_1$-$C_4$-alkylthio-$C_1$-$C_4$-alkyl, $C_3$-$C_8$-Cycloalkyl, $\alpha$-Hydroxy-$C_3$-$C_6$-cycloalkyl, Oxa- oder Thia-$C_3$-$C_6$-cycloalkyl, 1,3-Dioxa- oder 1,3-Dithia-$C_3$-$C_8$-cycloalkyl, $C_3$-$C_6$-Cycloalkyl-(hydroxy)-$C_1$-$C_4$-alkyl, 1-($C_1$-$C_4$-Alkylthio-$C_3$-$C_6$-cycloalkyl)-1-hydroxy)-$C_1$-$C_4$-alkyl oder unsubstituiertes oder durch $C_1$-$C_4$-Alkyl, $C_1$-$C_4$-Alkoxy, Hydroxy und/oder Halogen substituierten Phenyl-$C_1$-$C_4$-alkyl oder Naphthyl-$C_1$-$C_4$-alkyl bedeutet, und ihrer Salze.

**3.** Verfahren gemäß Anspruch 1 zur Herstellung von Verbindungen der Formel 1, worin worin R $C_3$-$C_7$-Alkyl, α,α-Di-$C_1$-$C_4$-alkoxy-$C_1$-$C_4$-alkyl, $C_3$-$C_6$-Cycloalkyl-$C_1$-$C_4$-alkyl, $C_3$-$C_6$-Cycloalkenyl-$C_1$-$C_4$-alkyl, Mono-, Di- oder Trihydroxy-$C_3$-$C_6$-cycloalkyl-$C_1$-$C_4$-alkyl oder unsubstituiertes oder durch $C_1$-$C_4$-Alkyl, $C_1$-$C_4$-Alkoxy, Hydroxy und/oder Halogen mono-, di- oder trisubstituiertes Benzyl bedeutet, $R_2$ Wasserstoff oder Hydroxy darstellt, $R_1$, $R_1$', $R_2$' und $R_3$ Wasserstoff bedeuten, $R_4$ durch $C_1$-$C_4$-Alkyl, $C_1$-$C_4$-Alkoxy und/oder Halogen mono-, di- oder trisubstituiertes Phenyl- oder Diphenyl-$C_1$-$C_4$-alkyl oder unsubstituiertes oder durch Halogen der Atomnummer bis und mit 35 monosubstituiertes Naphthyl-$C_1$-$C_4$-alkyl darstellt und $R_5$ Wasserstoff, $C_1$-$C_4$-Alkyl oder eine Gruppe $R_4$ bedeutet, und ihrer Salze.

**4.** Verfahren gemäß Anspruch 1 zur Herstellung von Verbindungen der Formel I, worin worin $R_1$, $R_1$', $R_2$' und $R_3$ Wasserstoff bedeuten, $R_2$ Wasserstoff oder Hydroxy darstellt, $R_4$ durch $C_1$-$C_4$-Alkyl oder $C_1$-$C_4$-Alkoxy monosubstituiertes oder durch Halogen der Atomnummer bis und mit 35 mono- oder disubstituiertes Phenyl-$C_1$-$C_4$-alkyl oder unsubstituiertes oder durch Halogen der Atomnummer bis und mit 35 monosubstituiertes Naphthyl-, Thienyl-, Furyl- oder Pyridyl-$C_1$-$C_4$-alkyl darstellt, $R_5$ Wasserstoff, $C_1$-$C_4$-Alkyl oder eine Gruppe $R_4$ bedeutet und R $C_3$-$C_7$-Alkyl α,α-Di-$C_1$-$C_4$-alkoxy-$C_1$-$C_4$-alkyl, $C_3$-$C_6$-Cycloalkyl-$C_1$-$C_4$-alkyl oder unsubstituiertes oder durch $C_1$-$C_4$-Alkyl, $C_1$-$C_4$-Alkoxy oder Halogen der Atomnummer bis und mit 35 substituiertes Benzyl bedeutet, und ihrer Salze.

**5.** Verfahren gemäß Anspruch 1 zur Herstellung von Verbindungen der Formel I, worin worin $R_1$, $R_1$', $R_2$' und $R_3$ Wasserstoff bedeuten, $R_2$Wasserstoff oder Hydroxy darstellt, $R_4$ durch Halogen der Atomnummer bis und mit 35 mono- oder disubstituiertes α-Phenyl-$C_1$-$C_4$-alkyl oder unsubstituiertes α-Naphthyl-$C_1$-$C_4$-alkyl, α-Thienyl-$C_1$-$C_4$-alkyl, α-Furyl-$C_1$-$C_4$-alkyl oder α-Pyridyl-$C_1$-$C_4$-alkyl darstellt, $R_5$ Wasserstoff, $C_1$-$C_4$-Alkyl oder eine Gruppe $R_4$ bedeutet und R $C_3$-$C_5$-Alkyl, α,α-Di-$C_1$-$C_4$-alkoxymethyl, $C_3$-$C_6$-Cycloalkyl-$C_1$-$C_4$-alkyl, α-($C_3$-$C_6$-Cycloalkenyl)-$C_1$-$C_4$-alkyl, α -(Mono-, Di- oder Trihydroxy-$C_3$-$C_6$-cycloalkyl)-$C_1$-$C_4$-alkyl oder Benzyl bedeutet, und ihrer Salze.

**6.** Verfahren gemäß Anspruch 1 zur Herstellung von Verbindungen der Formel I, worin worin $R_1$, $R_1$', $R_2$' und $R_3$ Wasserstoff bedeuten, $R_2$Wasserstoff oder Hydroxy darstellt, $R_4$ durch Halogen der Atomnummer bis und mit 35 mono- oder disubstituiertes Phenyl-$C_1$-$C_4$-alkyl oder unsubstituiertes Naphthyl-, Thienyl-, Furyl- oder Pyridyl-$C_1$-$C_4$-alkyl darstellt, $R_5$Wasserstoff, $C_1$-$C_4$-Alkyl oder eine Gruppe $R_4$ bedeutet und R $C_3$-$C_5$-Alkyl, α,α-Di-$C_1$-$C_4$-alkoxymethyl, $C_3$-$C_6$-Cycloalkyl-$C_1$-$C_4$-alkyl oder Benzyl bedeutet, und ihrer Salze.

**7.** Verfahren gemäß Anspruch 1 zur Herstellung von 3-(p-Chlorbenzylamino)propyl(diäthoxymethyl)-phosphinsäure oder eines ihrer Salze.

**8.** Verfahren gemäß Anspruch 1 zur Herstellung von 3-(p-Chlorbenzylamino)-2(S)-hydroxy-propyl(benzyl)-phosphinsäure oder eines ihrer Salze.

**9.** Verfahren gemäß Anspruch 1 zur Herstellung von 3-(p-Chlorbenzylamino)propyl(n-butyl)phosphinsäure oder eines ihrer Salze.

**10.** Verfahren gemäß Anspruch 1 zur Herstellung von 3-(p-Chlorbenzylamino)propyl(cyclohexylmethyl)-phosphinsäure oder eines ihrer Salze.

**11.** Verfahren gemäß Anspruch 1 zur Herstellung von 3-(p-Chlorbenzylamino)-2(R)-hydroxy-propyl(benzyl)-phosphinsäure oder eines ihrer Salze.

**12.** Verfahren gemäß Anspruch 1 zur Herstellung von 3-(3,4-Dichlorbenzylamino)propyl(diäthoxymethyl)-phosphinsäure oder eines ihrer Salze.

**13.** Verfahren gemäß Anspruch 1 zur Herstellung von 3-[1-(p-Chlorphenyl)äthylamino]propyl-(diäthoxymethyl)phosphinsäure oder eines ihrer Salze.

**14.** Verfahren gemäß Anspruch 1 zur Herstellung von 3-(Naphthyl-1-ylmethylamino)propyl(diäthoxymethyl)-phosphinsäure oder eines ihrer Salze.

**15.** Verfahren gemäß Anspruch 1 zur Herstellung von 3-(3,4-Dichlorbenzylamino)propyl(cyclohexylmethyl)-phosphinsäure oder eines ihrer Salze.

**16.** Verfahren gemäß Anspruch 1 zur Herstellung von 3-(Pyrid-2-ylmethylamino)-2(S)-hydroxy-propyl-(benzyl)phosphinsäure oder eines ihrer Salze.

**17.** Verfahren gemäß Anspruch 1 zur Herstellung von 3-(p-Chlorbenzylaminoyl)-2(S)-hydroxy-propyl-(cyclohexylmethyl)phosphinsäure oder eines ihrer Salze.

**18.** Verfahren gemäß Anspruch 1 zur Herstellung von 3-(3,4-Dichlorbenzylaminoyl)-2(S)-hydroxy-propyl-(benzyl)phosphinsäure oder eines ihrer Salze.

**19.** Verfahren gemäß Anspruch 1 zur Herstellung von 3-(p-Fluorbenzylamino)propyl(diäthoxymethyl)-phosphinsäure oder eines ihrer Salze.

**20.** Verfahren gemäß Anspruch 1 zur Herstellung von 3-(4-Chlor-3-trifluormethyl-benzylamino)propyl-(diäthoxymethyl)phosphinsäure oder eines ihrer Salze.

**21.** Verfahren gemäß Anspruch 1 zur Herstellung von 3-(m-Chlorbenzylamino)propyl(diäthoxymethyl)-phosphinsäure oder eines ihrer Salze.

**22.** Verfahren gemäß Anspruch 1 zur Herstellung von 3-(3,4-Dichlorbenzylamino)-2(S)-hydroxy-propyl-(cyclohexylmethyl)phosphinsäure oder eines ihrer Salze.

**23.** Verfahren gemäß Anspruch 1 zur Herstellung von 3-[1-(3,4-Dichlorphenyl)äthylamino]propyl-(diäthoxymethyl)phosphinsäure oder eines ihrer Salze.

**24.** Verfahren gemäß Anspruch 1 zur Herstellung von 3-[1-(p-Chlorphenyl)aminoäthyl]-2(S)-hydroxy-propyl-(benzyl)phosphinsäure oder eines ihrer Salze.

**25.** Verfahren gemäß Anspruch 1 zur Herstellung von 3-(p-Jodbenzylamino)propyl(diäthoxymethyl)-phosphinsäure oder eines ihrer Salze.

**26.** Verfahren gemäß Anspruch 1 zur Herstellung von 3-(2,4-Dichlorbenzylamino)propyl(diäthoxymethyl)-phosphinsäure oder eines ihrer Salze.

**27.** Verfahren gemäß Anspruch 1 zur Herstellung von 3-(o-Chlorbenzylamino)propyl(diäthoxymethyl)-phosphinsäure oder eines ihrer Salze.

**28.** Verfahren gemäß Anspruch 1 zur Herstellung von 3-(3,4-Dichlorbenzylamino)propyl(cyclopropylmethyl)-phosphinsäure oder eines ihrer Salze.

**29.** Verfahren gemäß Anspruch 1 zur Herstellung von 3-(p-Chlorbenzylamino)propyl(tetrahydrofuran-2-yl)-phosphinsäure oder eines ihrer Salze.

**30.** Verfahren gemäß Anspruch 1 zur Herstellung von 3-[N-(p-Chlorbenzyl)-N-methyl-amino]propyl-(diäthoxymethyl)phosphinsäure oder eines ihrer Salze.

**31.** Verfahren gemäß Anspruch 1 zur Herstellung von 3-(p-Chlorbenzylamino)-2(S)-hydroxy-propyl(p-methylbenzyl)phosphinsäure oder eines ihrer Salze.

**32.** Verfahren gemäß Anspruch 1 zur Herstellung von 3-(3,4-Dichlorbenzylamino)-2(S)-hydroxy-propyl(p-methylbenzyl)phosphinsäure oder eines ihrer Salze.

**33.** Verfahren gemäß Anspruch 1 zur Herstellung von 3-(p-Chlorbenzylamino)-2(S)-hydroxy-propyl(p-chlorbenzyl)phosphinsäure oder eines ihrer Salze.

**34.** Verfahren gemäß Anspruch 1 zur Herstellung von 3-(3,4-Dichlorbenzylamino)-2(S)-hydroxy-propyl(p-chlorbenzyl)phosphinsäure oder eines ihrer Salze.

**35.** Verfahren gemäß Anspruch 1 zur Herstellung von
3-(p-Chlorbenzylamino)-2(S)-hydroxy-propyl(diäthoxymethyl)phosphinsäure;
3-[1-(p-Chlorphenyl)äthylamino]-2(S)-hydroxy-propyl(diäthoxymethyl)phosphinsäure;
3-(3,4-Dichlorbenzylamino)-2(S)-hydroxy-propyl(diäthoxymethyl)phosphinsäure;
3-[1-(3,4-Dichlorphenyl)äthylamino]-2(S)-hydroxy-propyl(diäthoxymethyl)phosphinsäure;
3-[N-(p-Chlorbenzyl)-N-methyl-amino]-2(S)-hydroxy-propyl(benzyl)phosphinsäure;
3-(p-Chlorbenzylamino)-2(S)-hydroxy-propyl(p-methoxybenzyl)phosphinsäure;
3-(p-Chlorbenzylamino)-2(S)-hydroxy-propyl(3,4-dimethoxybenzyl)phosphinsäure;
3-(3,4-Dichlorbenzylamino)-2(S)-hydroxy-propyl(3,4-dimethoxybenzyl)phosphinsäure;
3-[2-(p-Chlorphenyl)prop-2-ylamino]propyl(diäthoxymethyl)phosphinsäure;
3-[2-(3,4-Dichlorphenyl)prop-2-ylamino]propyl(diäthoxymethyl)phosphinsäure;
3-[2-(p-Chlorphenyl)prop-2-ylamino]-2(S)-hydroxy-propyl(benzyl)phosphinsäure;
3-[2-(3,4-Dichlorphenyl)prop-2-ylamino)-2(S)-hydroxy-propyl(benzyl)phosphinsäure;
3-(3,4-Dichlorbenzylamino)-2(S)-hydroxy-propyl(3,4,5-trihydroxycyclohexylmethyl)phosphinsäure;
3-(4-Chlor-3-methoxy-benzylamino)propyl(diäthoxymethyl)phosphinsäure;
3-(4-Chlor-3-methoxy-benzylamino)-2(S)-hydroxy-propyl(benzyl)phosphinsäure;
3-(2-Phenyläthylamino)propyl(diäthoxymethyl)phosphinsäure;
3-(2-Phenyläthylamino)-2(S)-hydroxy-propyl(diäthoxymethyl)phosphinsäure;
3-(p-Chlorbenzylamino-2(S)-hydroxy-propyl(4-methoxycyclohexyl)phosphinsäure;
3-(3,4-Dichlor-6-jod-benzylamino)-2(S)-hydroxy-propyl(benzyl) phosphinsäure;
3-(3,4-Dichlor-6-jod-benzylamino)propyl(diäthoxymethyl)phosph insäure;
3-(4-Chlor-3-jod-benzylamino)propyl(diäthoxymethyl)phosphinsäure;
3-(3-Chlor-4-jod-benzylamino)propyl(diäthoxymethyl)phosphinsäure;
3-(4-Chlor-3-jod-benzylamino)-2(S)-hydroxy-propyl(benzyl)phosphinsäure;
3-(3-Chlor-4-jod-benzylamino)-2(S)-hydroxy-propyl(benzyl)phosphinsäure;
3-Di(p-chlorbenzyl)aminopropyl(diäthoxymethyl)phosphinsäure;
3-Di(3,4-dichlorbenzyl)amino-2(S)-hydroxy-propyl(benzyl)phosp hinsäure;
3-(m-Chlorbenzylamino)-2(S)-hydroxy-propyl(benzyl)phosphinsäure;
3-(3,4-Dichlorbenzyl)amino-2(S)-hydroxy-propyl(cis-4,5-dihydr oxycyclohexylmethyl)phosphinsäure;
3-(3,4-Dichlorbenzyl)amino-2(S)-hydroxy-propyl(cyclohex-3-eny lmethyl)phosphinsäure;
3-[1-(3,4-Dichlorphenyl)äthylamino]-2(S)-hydroxy-propyl(cyclo hex-3-enylmethyl)phosphinsäure oder
3-[1-(3,4-Dichlorphenyl)äthylamino]-2(S)-hydroxy-propyl(cis-4,5-dihydroxycyclohexylmethyl)-phosphinsäure oder jeweils eines Salzes davon.

**36.** Verfahren zur Herstellung von pharmazeutischen Präparaten, dadurch gekennzeichnet, dass man eine Verbindung der Formel I

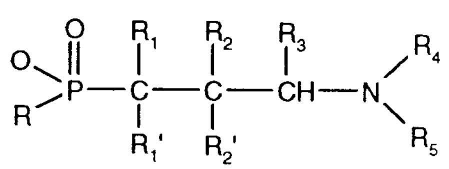

(I),

worin $R_1$, $R_1'$, $R_2'$ und $R_3$ Wasserstoffbedeuten, $R_2$Wasserstoff oder Hydroxy darstellt, $R_4$ einen unsubstituierten oder im Phenyl- bzw. Naphthylteil durch $C_1$-$C_4$-Alkyl, $C_1$-$C_4$-Alkoxy, Hydroxy und/oder Halogen mono-, di- oder trisubstituierten Phenyl-$C_1$-$C_4$-alkyl-, Diphenyl-$C_1$-$C_4$-alkyl- oder Naphthyl-$C_1$-$C_4$-alkylrest oder einen unsubstituierten oder im Thienyl-, Furyl- bzw. Pyridylteil durch Halogen substituierten Thienyl-, Furyl- oder Pyridyl-$C_1$-$C_4$-alkylrest bedeutet, $R_5$ Wasserstoff, $C_1$-$C_4$-Alkyl oder eine Gruppe $R_4$ darstellt und R $C_2$-$C_4$-Alkyl, $C_2$-$C_4$-Alkenyl, $C_2$-$C_7$-Alkinyl, Oxo-$C_2$-$C_7$-alkyl, Hydroxy-$C_2$-$C_7$-alkyl oder Dihydroxy-$C_2$-$C_7$-alkyl, $\alpha$-Hydroxy-$C_3$-$C_5$-alkenyl, Mono-, Di- oder Trifluor-$C_2$-$C_5$-alkyl, Mono-, Di- oder Trifluor-$C_2$-$C_5$-alkenyl, Mono-, Di- oder Trifluor-$\alpha$-hydroxy-$C_2$-$C_5$-alkyl, Mono-, Di- oder Trifluor-$\alpha$-hydroxy-$C_2$-$C_5$-alkenyl, $C_1$-$C_4$-Alkoxy-$C_1$-$C_4$-alkyl, Di-$C_1$-$C_4$-alkoxy-$C_1$-$C_4$-alkyl, $C_1$-$C_4$-Alkoxy $C_1$-$C_4$-Alkoxy-$C_2$-$C_7$-(hydroxy)-alkyl, $C_1$-$C_4$-Alkoxy-$C_2$-$C_5$-(halo)-alkyl, $C_1$-$C_4$-Alkylthio-$C_1$-$C_4$-alkyl, Di-$C_1$-$C_4$-alkylthio-$C_1$-$C_4$-alkyl, $C_3$-$C_8$-Cycloalkyl, $\alpha$-Hydroxy-$C_3$-$C_6$-cycloalkyl, Oxa- oder Thia-$C_3$-$C_6$-cyclo-

alkyl, 1,3-Dioxa- oder 1,3-Dithia-$C_3$-$C_8$-cycloalkyl, $C_3$-$C_8$-Cycloalkyl-$C_1$-$C_4$-alkyl, $C_3$-$C_8$-Cycloalkenyl-$C_1$-$C_4$-alkyl, Mono-, Di- oder Trihydroxy-$C_3$-$C_8$-cycloalkenyl, $C_3$-$C_6$-Cycloalkyl-(hydroxy)-$C_1$-$C_4$-alkyl, 1-($C_1$-$C_4$-Alkylthio-$C_3$-$C_6$-cycloalkyl)-1-hydroxy)-$C_1$-$C_4$-alkyl, unsubstituiertes oder durch $C_1$-$C_4$-Alkyl, $C_1$-$C_4$-Alkoxy, Halogen, Hydroxy und/oder Trifluormethyl mono-, di- oder trisubstituiertes Mono- oder Diphenyl-$C_1$-$C_4$-alkyl, Naphthyl-$C_1$-$C_4$-alkyl 1 oder unsubstituiertes oder durch Halogen substituiertes Thienyl-, Furyl- oder Pyridyl-$C_1$-$C_4$-alkyl bedeutet, in freier Form oder in pharmazeutisch verwendbarer Salzform mit üblichen pharmazeutischen Hilfsstoffen vermischt.

**37.** Verfahren gemäß Anpruch 36 zur Herstellung von pharmazeutischen Präparaten, dadurch gekennzeichnet, dass man eine Verbindung gemäß einem der Ansprüche 1 bis 35 in freier Form oder in pharmazeutisch verwendbarer Salzform mit üblichen pharmazeutischen Hilfsstoffen vermischt.

**38.** Verfahren gemäß Anpruch 36 oder 37 zur Herstellung von pharmazeutischen Präparaten, bestimmt für die Behandlung von Epilepsien des "petit mal"-Formenkreises sowie zur Unterdrückung von "petit mal"-ähnlichen Zuständen die bei der Behandlung mit Phenytoin, Carbamazepin, Vigabatrin® und Antiepileptika mit gleichem oder ähnlichen Wirkungsprofil auftreten können.

**Claims**

**Claims for the following Contracting States : AT, BE, CH, DE, DK, FR, GB, IT, LI, LU, NL, SE**

**1.** A novel araliphatically N-substituted aminoalkanephosphinic acid of formula I

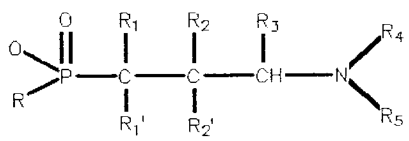

(I),

wherein $R_1$, $R_1'$, $R_2$ and $R_3$ are hydrogen, $R_2$ is hydrogen or hydroxy, $R_4$ is a phenyl-$C_1$-$C_4$alkyl, diphenyl-$C_1$-$C_4$alkyl or naphthyl-$C_1$-$C_4$alkyl radical each of which is mono-, di- or tri-substituted in the phenyl or naphthyl moiety by $C_1$-$C_4$alkyl, $C_1$-$C_4$alkoxy, hydroxy and/or by halogen, or is a thienyl-, furyl- or pyridyl-$C_1$-$C_4$alkyl radical each of which is unsubstituted or halo-substituted in the thienyl, furyl or pyridyl moiety, $R_5$ is hydrogen, $C_1$-$C_4$alkyl or a group $R_4$, and A is $C_2$-$C_4$alkyl, $C_2$-$C_4$alkenyl, $C_2$-$C_7$alkynyl, oxo-$C_2$-$C_7$alkyl, hydroxy-$C_2$-$C_7$alkyl or dihydroxy-$C_2$-$C_7$alkyl, $\alpha$-hydroxy-$C_3$-$C_5$alkenyl, mono-, di- or tri-fluoro-$C_2$-$C_5$alkyl, mono-, di- or tri-fluoro-$C_2$-$C_5$alkenyl, mono-, di- or tri-fluoro-$\alpha$-hydroxy-$C_2$-$C_5$alkyl, mono-, di- or tri-fluoro-$\alpha$-hydroxy-$C_2$-$C_5$alkenyl, $C_1$-$C_4$alkoxy-$C_1$-$C_4$alkyl, di-$C_1$-$C_4$alkoxy-$C_1$-$C_4$alkyl, $C_1$-$C_4$alkoxy $C_1$-$C_4$alkoxy-$C_2$-$C_7$(hydroxy)alkyl, $C_1$-$C_4$alkoxy-$C_2$-$C_5$(halo)alkyl, $C_1$-$C_4$- $C_1$-$C_4$alkyl, di-$C_1$-$C_4$alkylthio-$C_1$-$C_4$alkyl, $C_3$-$C_8$cycloalkyl, $\alpha$-hydroxy-$C_3$-$C_6$cycloalkyl, oxa- or thia-$C_3$-$C_6$cycloalkyl,1,3-dioxa- or 1,3-dithia-$C_3$-$C_8$cycloalkyl, $C_3$-$C_8$cycloalkyl-$C_1$-$C_4$alkyl, $C_3$-$C_8$cycloalkenyl-$C_1$-$C_4$alkyl, mono-, di- or tri-hydroxy-$C_3$-$C_8$cycloalkenyl, $C_3$-$C_6$cycloalkyl-(hydroxy)-$C_1$-$C_4$alkyl, 1-($C_1$-$C_4$alkylthio-$C_3$-$C_6$cycloalkyl)-1-(hydroxy)-$C_1$-$C_4$alkyl, or mono- or di-phenyl-$C_1$-$C_4$alkyl or naphthyl-$C_1$-$C_4$alkyl each of which is unsubstituted or mono-, di- or tri-substituted by $C_1$-$C_4$alkyl, $C_1$-$C_4$alkoxy, halogen, hydroxy and/or by trifluoromethyl, or unsubstituted or halo-substituted thienyl-, furyl- or pyridyl-$C_1$-$C_4$alkyl, or a salt thereof.

**2.** A compound according to claim 1 of formula I, wherein $R_1$, $R_1'$, $R_2'$ and $R_3$ are hydrogen, $R_2$ is hydrogen or hydroxy, $R_4$ is a phenyl- or naphthyl-$C_1$-$C_4$alkyl radical each of which is substituted by $C_1$-$C_4$alkyl, $C_1$-$C_4$alkoxy, hydroxy and/or by halogen, or an unsubstituted or halo-substituted thienyl-, furyl- or pyridyl-$C_1$-$C_4$alkyl radical, $R_5$ is hydrogen, lower alkyl or a group $R_4$, and R is $C_2$-$C_4$alkyl, $C_2$-$C_4$alkenyl, $C_2$-$C_7$alkynyl, oxo-$C_2$-$C_7$alkyl, hydroxy-$C_2$-$C_7$alkyl or di-hydroxy-$C_2$-$C_7$alkyl, $\alpha$-hydroxy-$C_3$-$C_5$alkenyl, mono-, di- or mono-, di- or tri-fluoro-$C_2$-$C_5$alkyl, mono-, di- or tri-fluoro-$C_2$-$C_5$alkenyl, mono-, di- or tri-fluoro-$\alpha$-hydroxy-$C_2$-$C_5$alkyl, mono-, di- or tri-fluoro-$\alpha$-hydroxy-$C_2$-$C_5$alkenyl, $C_1$-$C_4$alkoxy-$C_1$-$C_4$alkyl, di-$C_1$-$C_4$alkoxy-$C_1$-$C_4$alkyl, $C_1$-$C_4$alkoxy-$C_2$-$C_7$(hydroxy)alkyl, $C_1$-$C_4$alkoxy-$C_2$-$C_5$(halo)alkyl, $C_1$-$C_4$alkylthio-$C_1$-$C_4$alkyl, di-$C_1$-$C_4$alkylthio-$C_1$-$C_4$alkyl, $C_3$-$C_8$cycloalkyl, $\alpha$-hydroxy-$C_3$-$C_6$cycloalkyl, oxa- or thia-$C_3$-$C_6$cycloalkyl,1,3-dioxa- or 1,3-dithia-$C_3$-$C_8$cycloalkyl, $C_3$-$C_6$cycloalkyl-(hydroxy)-$C_1$-$C_4$alkyl, 1-($C_1$-$C_4$alkylthio-$C_3$-$C_6$cycloalkyl)-1-(hydroxy)-$C_1$-$C_4$alkyl, or phenyl-$C_1$-$C_4$alkyl or naphthyl-$C_1$-$C_4$alkyl each of which is unsubstituted or substituted by $C_1$-$C_4$alkyl, $C_1$-$C_4$alkoxy, hydroxy and/or by halogen, or a salt thereof.

**3.** A compound according to claim 1 of formula I, wherein R is $C_3$-$C_7$alkyl, $\alpha,\alpha$-di-$C_1$-$C_4$alkoxy-$C_1$-$C_4$alkyl, $C_3$-$C_6$cycloalkyl-$C_1$-$C_4$alkyl,$C_3$-$C_6$cycloalkenyl-$C_1$-$C_4$alkyl, mono-, di- or tri-hydroxy-$C_3$-$C_6$cycloalkyl-$C_1$-$C_4$alkyl, or benzyl that is unsubstituted or mono-, di- or tri-substituted by $C_1$-$C_4$alkyl, $C_1$-$C_4$alkoxy, hydroxy and/or by halogen, $R_2$ is hydrogen or hydroxy, $R_1$, $R_1$', $R_2$' and $R_3$ are hydrogen, $R_4$ is phenyl- or diphenyl-$C_1$-$C_4$alkyl each of which is mono-, di- or tri-substituted by $C_1$-$C_4$alkyl, $C_1$-$C_4$alkoxy and/or by halogen, or is naphthyl-$C_1$-$C_4$alkyl that is unsubstituted or monosubstituted by halogen having an atomic number of up to and including 35, and $R_5$ is hydrogen, $C_1$-$C_4$alkyl or a group $R_4$, or a salt thereof.

**4.** A compound according to claim 1 of formula I, wherein $R_1$, $R_1$', $R_2$' and $R_3$ are hydrogen, $R_2$ is hydrogen or hydroxy, $R_4$ is phenyl-$C_1$-$C_4$alkyl that is monosubstituted by $C_1$-$C_4$alkyl or $C_1$-$C_4$alkoxy or mono- or di-substituted by halogen having an atomic number of up to and including 35, or is naphthyl-, thienyl-, furyl- or pyridyl-$C_1$-$C_4$alkyl each of which is unsubstituted or monosubstituted by halogen having an atomic number of up to and including 35, $R_5$ is hydrogen, $C_1$-$C_4$alkyl or a group $R_4$, and R is $C_3$-$C_7$alkyl, $\alpha,\alpha$-di-$C_1$-$C_4$alkoxy-$C_1$-$C_4$alkyl, $C_3$-$C_6$cycloalkyl-$C_1$-$C_4$alkyl, or benzyl that is unsubstituted or substituted by $C_1$-$C_4$alkyl, $C_1$-$C_4$alkoxy or by halogen having an atomic number of up to and including 35, or a salt thereof.

**5.** A compound according to claim 1 of formula I, wherein $R_1$, $R_1$', $R_2$' and $R_3$ are hydrogen, $R_2$ is hydrogen or hydroxy, $R_4$ is $\alpha$-phenyl-$C_1$-$C_4$alkyl that is mono- or di-substituted by halogen having an atomic number of up to and including 35, or is unsubstituted $\alpha$-naphthyl-$C_1$-$C_4$alkyl, $\alpha$-thienyl-$C_1$-$C_4$alkyl, $\alpha$-furyl-$C_1$-$C_4$alkyl or $\alpha$-pyridyl-$C_1$-$C_4$alkyl, $R_5$ is hydrogen, $C_1$-$C_4$alkyl or a group $R_4$, and R is $C_3$-$C_5$alkyl, $\alpha,\alpha$-di-$C_1$-$C_4$-alkoxymethyl, $C_3$-$C_6$cycloalkyl-$C_1$-$C_4$alkyl, $\alpha$-($C_3$-$C_6$cycloalkenyl)-$C_1$-$C_4$alkyl, $\alpha$-(mono-, di- or tri-hydroxy-$C_3$-$C_6$cycloalkyl)-$C_1$-$C_4$alkyl or benzyl, or a salt thereof.

**6.** A compound according to claim 1 of formula I, wherein $R_1$, $R_1$', $R_2$' and $R_3$ are hydrogen, $R_2$ is hydrogen or hydroxy, $R_4$ is phenyl-$C_1$-$C_4$alkyl that is mono- or di-substituted by halogen having an atomic number of up to and including 35, or is unsubstituted naphthyl-, thienyl-, furyl- or pyridyl-$C_1$-$C_4$alkyl, $R_5$ is hydrogen, $C_1$-$C_4$alkyl or a group $R_4$, and R is $C_3$-$C_5$alkyl, $\alpha,\alpha$-di-$C_1$-$C_4$alkoxymethyl, $C_3$-$C_6$cycloalkyl-$C_1$-$C_4$alkyl or benzyl, or a salt thereof.

**7.** 3-(p-Chlorobenzylamino)propyl(diethoxymethyl)phosphinic acid according to claim 1, or a salt thereof.

**8.** 3-(p-Chlorobenzylamino)-2(S)-hydroxypropyl(benzyl)phosphinic acid according to claim 1, or a salt thereof.

**9.** 3-(p-Chlorobenzylamino)propyl(n-butyl)phosphinic acid according to claim 1, or a salt thereof.

**10.** 3-(p-Chlorobenzylamino)propyl(cyclohexylmethyl)phosphinic acid according to claim 1, or a salt thereof.

**11.** 3-(p-Chlorobenzylamino)-2(R)-hydroxypropyl(benzyl)phosphinic acid according to claim 1, or a salt thereof.

**12.** 3-(3,4-Dichlorobenzylamino)propyl(diethoxymethyl)phosphinic acid according to claim 1, or a salt thereof.

**13.** 3-[1-(p-Chlorophenyl)ethylamino]propyl(diethoxymethyl)phosphinic acid according to claim 1, or a salt thereof.

**14.** 3-(Naphth-1-ylmethylamino)propyl(diethoxymethyl)phosphinic acid according to claim 1, or a salt thereof.

**15.** 3-(3,4-Dichlorobenzylamino)propyl(cyclohexylmethyl)phosphinic acid according to claim 1, or a salt thereof.

**16.** 3-(Pyrid-2-ylmethylamino)-2(S)-hydroxypropyl(benzyl)phosphinic acid according to claim 1, or a salt thereof.

**17.** 3-(p-Chlorobenzylaminoyl)-2(S)-hydroxypropyl(cyclohexylmethyl)phosphinic acid according to claim 1, or a salt thereof.

**18.** 3-(3,4-Dichlorobenzylaminoyl)-2(S)-hydroxypropyl(benzyl)phosphinic acid according to claim 1, or a salt thereof.

**19.** 3-(p-Fluorobenzylamino)propyl(diethoxymethyl)phosphinic acid according to claim 1, or a salt thereof.

**20.** 3-(4-Chloro-3-trifluoromethylbenzylamino)propyl(diethoxymethyl)phosphinic acid according to claim 1, or a salt thereof.

**21.** 3-(m-Chlorobenzylamino)propyl(diethoxymethyl)phosphinic acid according to claim 1, or a salt thereof.

**22.** 3-(3,4-Dichlorobenzylamino)-2(S)-hydroxypropyl(cyclohexylmethyl)phosphinic acid according to claim 1, or a salt thereof.

**23.** 3-[1-(3,4-Dichlorophenyl)ethylamino]propyl(diethoxymethyl)phosphinic acid according to claim 1, or a salt thereof.

**24.** 3-[1-(p-Chlorophenyl)ethylamino]-2(S)-hydroxypropyl(benzyl)phosphinic acid according to claim 1, or a salt thereof.

**25.** 3-(p-Iodobenzylamino)propyl(diethoxymethyl)phosphinic acid according to claim 1, or a salt thereof.

**26.** 3-(2,4-Dichlorobenzylamino)propyl(diethoxymethyl)phosphinic acid according to claim 1, or a salt thereof.

**27.** 3-(o-Chlorobenzylamino)propyl(diethoxymethyl)phosphinic acid according to claim 1, or a salt thereof.

**28.** 3-(3,4-Dichlorobenzylamino)propyl(cyclopropylmethyl)phosphinic acid according to claim 1, or a salt thereof.

**29.** 3-(p-Chlorobenzylamino)propyl(tetrahydrofuran-2-yl)phosphinic acid according to claim 1, or a salt thereof.

**30.** 3-[N-(p-Chlorobenzyl)-N-methylamino]propyl(diethoxymethyl)phosphinic acid according to claim 1, or a salt thereof.

**31.** 3-(p-Chlorobenzylamino)-2(S)-hydroxypropyl(p-methylbenzyl)phosphinic acid according to claim 1, or a salt thereof.

**32.** 3-(3,4-Dichlorobenzylamino)-2(S)-hydroxypropyl(p-methylbenzyl)phosphinic acid according to claim 1, or a salt thereof.

**33.** 3-(p-Chlorobenzylamino)-2(S)-hydroxypropyl(p-chlorobenzyl)phosphinic acid according to claim 1, or a salt thereof.

**34.** 3-(3,4-Dichlorobenzylamino)-2(S)-hydroxypropyl(p-chlorobenzyl)phosphinic acid according to claim 1, or a salt thereof.

**35.** 3-(p-Chlorobenzylamino)-2(S)-hydroxypropyl(diethoxymethyl)phosphinic acid;
3-[1-(p-chlorophenyl)ethylamino]-2(S)-hydroxypropyl(diethoxymethyl)phosphinic acid;
3-(3,4-dichlorobenzylamino)-2(S)-hydroxypropyl(diethoxymethyl)phosphinic acid;
3-[1-(3,4-dichlorophenyl)ethylamino]-2(S)-hydroxypropyl(diethoxymethyl)phosphinic acid;
3-[N-(p-chlorobenzyl)-N-methylamino]-2(S)-hydroxypropyl(benzyl)phosphinic acid;
3-(p-chlorobenzylamino)-2(S)-hydroxypropyl(p-methoxybenzyl)phosphinicacid;
3-(p-chlorobenzylamino)-2(S)-hydroxypropyl(3,4-dimethoxybenzyl)phosphinic acid;
3-(3,4-dichlorobenzylamino)-2(S)-hydroxypropyl(3,4-dimethoxybenzyl)phosphinic acid;

3-[2-(p-chlorophenyl)prop-2-ylamino]propyl(diethoxymethyl)phosphinic acid;
3-[2-(3,4-dichlorophenyl)prop-2-ylamino]propyl(diethoxymethyl)phosphinic acid;
3-[2-(p-chlorophenyl)prop-2-ylamino]-2(S)-hydroxypropyl(benzyl)phosphinic acid;
3-[2-(3,4-dichlorophenyl)prop-2-ylamino]-2(S)-hydroxypropyl(benzyl)phosphinic acid;
3-(3,4-dichlorobenzylamino)-2(S)-hydroxypropyl(3,4,5-trihydroxycyclohexylmethyl)phosphinic acid;
3-(4-chloro-3-methoxybenzylamino)propyl(diethoxymethyl)phosphinic acid;
3-(4-chloro-3-methoxybenzylamino)-2(S)-hydroxypropyl(benzyl)phosphinic acid;
3-(2-phenylethylamino)propyl(diethoxymethyl)phosphinic acid;
3-(2-phenylethylamino)-2(S)-hydroxypropyl(diethoxymethyl)phosphinic acid;
3-(p-chlorobenzylamino)-2(S)-hydroxypropyl(4-methoxycyclohexyl)phosphinic acid;
3-(3,4-dichloro-6-iodobenzylamino)-2(S)-hydroxypropyl(benzyl)phosphinic acid;
3-(3,4-dichloro-6-iodobenzylamino)propyl(diethoxymethyl)phosphinic acid;
3-(4-chloro-3-iodobenzylamino)propyl(diethoxymethyl)phosphinic acid;
3-(3-chloro-4-iodobenzylamino)propyl(diethoxymethyl)phosphinic acid;
3-(4-chloro-3-iodobenzylamino)-2(S)-hydroxypropyl(benzyl)phosphinicacid;
3-(3-chloro-4-iodobenzylamino)-2(S)-hydroxypropyl(benzyl)phosphinicacid;
3-di(p-chlorobenzyl)aminopropyl(diethoxymethyl)phosphinic acid;
3-di(3,4-dichlorobenzyl)amino-2(S)-hydroxypropyl(benzyl)phosphinic acid;
3-(m-chlorobenzylamino)-2(S)-hydroxypropyl(benzyl)phosphinic acid;
3-(3,4-dichlorobenzyl)amino-2(S)-hydroxypropyl(cis-4,5-dihydroxycyclohexylmethyl)phosphinic acid;
3-(3,4-dichlorobenzyl)amino-2(S)-hydroxypropyl(cyclohex-3-enylmethyl)phosphinic acid;
3-[1-(3,4-dichlorophenyl)ethylamino]-2(S)-hydroxypropyl(cyclohex-3-enylmethyl)phosphinic acid; or
3-[1-(3,4-dichlorophenyl)ethylamino]-2(S)-hydroxypropyl(cis-4,5-dihydroxycyclohexylmethyl)phosphinic acid
according to claim 1, or a salt of any one of such compounds.

**36.** A compound of formula I

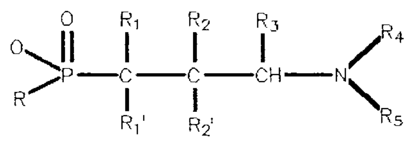

(I),

wherein $R_1$, $R_1'$, $R_2'$ and $R_3$ are hydrogen, $R_2$ is hydrogen or hydroxy, $R_4$ is a phenyl-$C_1$-$C_4$alkyl, diphenyl-$C_1$-$C_4$alkyl or naphthyl-$C_1$-$C_4$alkyl radical each of which is unsubstituted or mono-, di- or tri-substituted in the phenyl or naphthyl moiety by $C_1$-$C_4$alkyl, $C_1$-$C_4$alkoxy, hydroxy and/or by halogen, or is a thienyl-, furyl- or pyridyl-$C_1$-$C_4$alkyl radical each of which is unsubstituted or halo-substituted in the thienyl, furyl or pyridyl moiety, $R_5$ is hydrogen, $C_1$-$C_4$alkyl or a group $R_4$, and R is $C_2$-$C_4$alkyl, $C_2$-$C_4$alkenyl, $C_2$-$C_7$alkynyl, oxo-$C_2$-$C_7$alkyl, hydroxy-$C_2$-$C_7$alkyl or dihydroxy-$C_2$-$C_7$alkyl, $\alpha$-hydroxy-$C_3$-$C_5$alkenyl, mono-, di- or tri-fluoro-$C_2$-$C_5$alkyl, mono-, di- or tri-fluoro-$C_2$-$C_5$alkenyl, mono-, di- or tri-fluoro-$\alpha$-hydroxy-$C_2$-$C_5$alkyl, mono-, di- or tri-fluoro-$\alpha$-hydroxy-$C_2$-$C_5$alkenyl, $C_1$-$C_4$alkoxy-$C_1$-$C_4$alkyl, di-$C_1$-$C_4$alkoxy-$C_1$-$C_4$alkyl, $C_1$-$C_4$alkoxy $C_1$-$C_4$alkoxy-$C_2$-$C_7$(hydroxy)alkyl, $C_1$-$C_4$alkoxy-$C_2$-$C_5$(halo)-alkyl, $C_1$-$C_4$alkylthio-$C_1$-$C_4$alkyl, di-$C_1$-$C_4$alkylthio-$C_1$-$C_4$alkyl, $C_3$-$C_8$cycloalkyl, $\alpha$-hydroxy-$C_3$-$C_6$cycloalkyl, oxa- or thia-$C_3$-$C_6$cycloalkyl, 1,3-dioxa- or 1,3-dithia-$C_3$-$C_8$cycloalkyl, $C_3$-$C_8$cycloalkyl-$C_1$-$C_4$alkyl,$C_3$-$C_8$cycloalkenyl-$C_1$-$C_4$alkyl, mono-, di- or tri-hydroxy-$C_3$-$C_8$cycloalkenyl, $C_3$-$C_6$cycloalkyl-(hydroxy)$C_1$-$C_4$alkyl, 1-($C_1$-$C_4$alkylthio-$C_3$-$C_6$cycloalkyl)-1-(hydroxy)$C_1$-$C_4$alkyl, or mono- or di-phenyl-$C_1$-$C_4$alkyl or naphthyl-$C_1$-$C_4$alkyl each of which is unsubstituted or mono-, di- or tri-substituted by $C_1$-$C_4$alkyl, $C_1$-$C_4$alkoxy, halogen, hydroxy and/or by trifluoromethyl, or unsubstituted or halo-substituted thienyl-, furyl- or pyridyl-$C_1$-$C_4$alkyl, in free form or in the form of a pharmaceutically acceptable salt, for use in a method of therapeutically treating the human or animal body.

**37.** A compound according to any one of claims 2, 4 and 6 to 11 for use according to claim 36.

**38.** A compound according to any one of claims 1, 3, 5 and 12 to 35 for use according to claim 36.

**39.** A pharmaceutical composition comprising as pharmaceutical active ingredient a compound according to claim 36 in free form or in the form of a pharmaceutically acceptable salt.

**40.** A pharmaceutical composition according to claim 36 comprising as pharmaceutical active ingredient a compound according to any one of claims 2, 4 and 6 to 11 in free form or in the form of a pharmaceutically acceptable salt.

**41.** A pharmaceutical composition according to claim 36 comprising as pharmaceutical active ingredient a compound according to any one of claims 1, 3, 5 and 12 to 35 in free form or in the form of a pharmaceutically acceptable salt.

**42.** A pharmaceutical composition according to any one of claims 39 to 41 for the treatment of epilepsies of the "petit mal" type and for suppressing "petit mal"-type conditions which may arise in the case of treatment with phenytoin, carbamazepine, Vigabatrin® and anti-epileptics having the same or a similar profile of action.

**43.** A process for the preparation of a novel araliphatically N-substituted aminoalkanephosphinic acid according to claim 1, which process comprises, in a compound of formula II

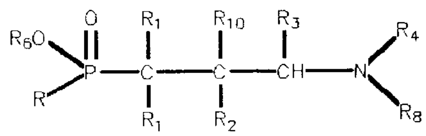

(II),

wherein $R_1'$, $R_2'$ and $R_3$ are hydrogen, $R_6$ is a hydroxy-protecting group, $R_8$ is a group $R_5$ or an amino-protecting group and $R_{10}$ is hydrogen or protected hydroxy, and R, $R_1$, $R_4$ and $R_5$ are as defined above, or in a salt thereof, freeing the hydroxy groups by replacing the hydroxy-protecting group $R_6$ by hydrogen and, where appropriate, removing the amino-protecting group $R_8$ and, where appropriate, freeing the hydroxy groups $R_2$ from the protected hydroxy group $R_{10}$, and, if desired, converting a resulting compound into a different compound of formula I, separating a mixture of isomers obtainable in accordance with the process into the components and separating the preferred isomer, and/or converting a free compound obtainable in accordance with the process into a salt or converting a salt obtainable in accordance with the process into the corresponding free compound.

**44.** The use of a compound having $GABA_B$-antagonistic properties for the preparation of a pharmaceutical composition for the treatment of epilepsies of the "petit mal" type or for suppressing "petit mal"-type conditions which may arise in the case of treatment with phenytoin, carbamazepine, Vigabatrin® and anti-epileptics having the same or a similar profile of action.

**45.** Use according to claim 44 for the preparation of a pharmaceutical composition for the treatment of epilepsies of the "petit mal" type.

**46.** Use according to claim 44 for the preparation of a pharmaceutical composition comprising as pharmaceutical active ingredient a compound of formula I

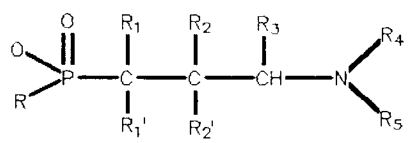

(I),

wherein one of the radicals $R_1$, $R_2$ and $R_3$ is hydrogen, $C_1$-$C_7$alkyl, $C_2$-$C_7$alkenyl, $C_2$-$C_7$alkynyl, $C_3$-$C_8$cycloalkyl, $C_3$-$C_8$cycloalkenyl-$C_1$-$C_4$alkyl, mono-, di- or tri-hydroxy-$C_3$-$C_8$cycloalkyl-$C_1$-$C_4$alkyl, or phenyl- or naphthyl-$C_1$-$C_4$alkyl, diphenyl-$C_1$-$C_4$alkyl or naphthyl-$C_1$-$C_4$alkyl each of which is unsubstituted or mono- or poly-substituted in the phenyl or naphthyl moiety by $C_1$-$C_4$alkyl, $C_1$-$C_4$alkoxy, halogen and/or by trifluoromethyl, or phenyl or naphthyl each of which is unsubstituted or substituted by halogen, $C_1$-$C_4$alkyl, $C_1$-$C_4$alkoxy and/or by trifluoromethyl, another radical is hydrogen or, in the case of $R_1$ or $R_2$, hydroxy or, in the case of $R_1$, halogen or, in the case of $R_2$ together with $R_2'$, oxo, and the remaining radical is hydrogen, $R_1'$ is hydrogen or halogen, $R_2'$ is hydrogen, hydroxy or, together with $R_2$, is oxo, $R_4$ and $R_5$ are hydrogen, or $R_4$ is phenyl-$C_1$-$C_4$alkyl, diphenyl-$C_1$-$C_4$alkyl,

naphthyl-$C_1$-$C_4$alkyl, thienyl-$C_1$-$C_4$alkyl, furyl-$C_1$-$C_4$alkyl or pyridyl-$C_1$-$C_4$alkyl each of which is unsubstituted or mono- or poly-substituted in the phenyl, naphthyl, thienyl, furyl or pyridyl moiety by $C_1$-$C_4$alkyl, $C_1$-$C_4$alkoxy, halogen and/or by trifluoromethyl, and $R_5$ is hydrogen, $C_1$-$C_7$alkyl, $C_2$-$C_7$alkenyl, $C_2$-$C_7$alkynyl or a group $R_4$, and R is $C_2$-$C_7$alkyl, $C_2$-$C_7$alkenyl, $C_2$-$C_7$alkynyl, $C_3$-$C_8$cycloalkyl, $C_3$-$C_8$cycloalkyl-$C_1$-$C_4$alkyl, $C_3$-$C_8$cycloalkenyl-$C_1$-$C_4$alkyl, mono-, di- or tri-hydroxy-$C_3$-$C_8$cycloalkyl-$C_1$-$C_4$alkyl, $C_3$-$C_8$cycloalkyl-(hydroxy)-$C_1$-$C_4$alkyl, ($C_1$-$C_4$alkylthio)cycloalkyl(hydroxy)-$C_1$-$C_4$alkyl, or phenyl- or naphthyl-$C_1$-$C_4$-lower alkyl each of which is unsubstituted or mono- or poly-substituted in the phenyl or naphthyl moiety by $C_1$-$C_4$alkyl, $C_1$-$C_4$alkoxy, halogen and/or by trifluoromethyl, or phenyl or naphthyl each of which is unsubstituted or substituted by halogen, $C_1$-$C_4$alkyl, $C_1$-$C_4$alkoxy and/or by trifluoromethyl, or, when $R_1$ is hydrogen or hydroxy, $R_2$ is an aromatic radical as defined above and $R_1'$, $R_2'$ and $R_3$ are hydrogen, R is methyl, or a pharmaceutically acceptable salt thereof.

**47.** Use according to claim 44 for the preparation of a pharmaceutical composition comprising as pharmaceutical active ingredient a compound of formula I wherein one of the radicals $R_1$, $R_2$ and $R_3$ is hydrogen, $C_1$-$C_4$alkyl, $C_3$-$C_6$cycloalkyl, phenyl-$C_1$-$C_4$alkyl that is unsubstituted or mono- or poly-substituted in the phenyl or naphthyl moiety by $C_1$-$C_4$alkyl, $C_1$-$C_4$alkoxy, halogen and/or by trifluoromethyl, or is phenyl that is unsubstituted or mono- or poly-substituted in the phenyl or naphthyl moiety by $C_1$-$C_4$alkyl, $C_1$-$C_4$alkoxy, halogen and/or by trifluoromethyl, another radical is hydrogen or, in the case of $R_1$ or $R_2$, hydroxy, and the remaining radical is hydrogen, $R_1'$ is hydrogen, $R_2'$ is hydrogen, hydroxy or, together with $R_2$, is oxo, $R_4$ and $R_5$ are hydrogen, or $R_4$ is a phenyl-$C_1$-$C_4$alkyl radical that is unsubstituted or mono- or poly-substituted in the phenyl moiety by $C_1$-$C_4$alkyl, $C_1$-$C_4$alkoxy, halogen and/or by trifluoromethyl, and $R_5$ is hydrogen, $C_1$-$C_4$alkyl or a group $R_4$, and R is $C_2$-$C_7$alkyl, $\alpha,\alpha$-di-$C_1$-$C_4$alkoxy-$C_2$-$C_7$alkyl, $\alpha,\alpha$-dihalo-$C_2$-$C_7$alkyl, $C_3$-$C_6$cycloalkyl, $C_3$-$C_6$cycloalkyl-$C_1$-$C_4$alkyl, $C_3$-$C_8$cycloalkenyl-$C_1$-$C_4$alkyl, mono-, di- or tri-hydroxy-$C_3$-$C_8$cycloalkyl-$C_1$-$C_4$alkyl, benzyl or, when $R_2$ is halophenyl and $R_1$, $R_1'$, $R_2'$ and $R_3$ are hydrogen, R is methyl, or a pharmaceutically acceptable salt thereof.

**48.** Use according to claim 44 for the preparation of a pharmaceutical composition comprising as pharmaceutical active ingredient a compound of formula I wherein

a) $R_1$, $R_1'$, $R_2'$, $R_3$, $R_4$ and $R_5$ are hydrogen, $R_2$ is $C_1$-$C_4$alkyl, $C_3$-$C_6$cycloalkyl, benzyl, phenyl, halophenyl or naphthyl and $R_2'$ is hydrogen or hydroxy, or $R_2$ is hydroxy and $R_2'$ is hydrogen, or $R_2$ and $R_2'$ together form an oxo group, or

b) $R_1$ is hydroxy, $C_1$-$C_4$alkyl, $C_3$-$C_6$cycloalkyl, benzyl, phenyl, halophenyl or naphthyl, and $R_1'$, $R_2$, $R_3$, $R_4$ and $R_5$ are hydrogen, or

c) $R_1$ is halogen, $R_1'$ is hydrogen or halogen and $R_2$, $R_2'$, $R_3$, $R_4$ and $R_5$ are hydrogen, and

R is $C_2$-$C_7$alkyl, $\alpha,\alpha$-di-$C_1$-$C_4$alkoxy-$C_2$-$C_7$alkyl, $\alpha,\alpha$,-dihalo-$C_2$-$C_7$alkyl, $C_3$-$C_6$cycloalkyl, $C_3$-$C_6$cycloalkyl-$C_1$-$C_4$alkyl, benzyl or, when $R_2$ is halophenyl or naphthyl and $R_1$, $R_1'$, $R_2'$ and $R_3$ are hydrogen, R is methyl, or a pharmaceutically acceptable salt thereof.

**49.** Use according to claim 44 for the preparation of a pharmaceutical composition comprising as pharmaceutical active ingredient a compound of formula I wherein $R_1$, $R_1'$, $R_2'$ and $R_3$ are hydrogen, $R_2$ is hydrogen or hydroxy, $R_4$ and $R_5$ are hydrogen, or $R_4$ is a phenyl-$C_1$-$C_4$alkyl radical that is unsubstituted or mono- or poly-substituted in the phenyl moiety by $C_1$-$C_4$alkyl, $C_1$-$C_4$alkoxy, halogen and/or by tri-fluoromethyl, and $R_5$ is hydrogen or $C_1$-$C_4$alkyl, and R is $C_2$-$C_7$alkyl, $\alpha,\alpha$-di-$C_1$-$C_3$alkoxy-$C_1$-$C_4$alkyl, $\alpha,\alpha$-dihalo-$C_2$-$C_7$alkyl, $C_3$-$C_6$cycloalkyl, $C_3$-$C_6$cycloalkyl-$C_1$-$C_4$alkyl, a-($C_3$-$C_6$cycloalkenyl)-$C_1$-$C_4$alkyl, a-(mono-, di- or tri-hydroxy-$C_3$-$C_6$cycloalkyl)-$C_1$-$C_4$alkyl, or is a phenyl-$C_1$-$C_4$alkyl radical that is unsubstituted or mono- or poly-substituted in the phenyl moiety by $C_1$-$C_4$alkyl, $C_1$-$C_4$alkoxy, halogen and/or by trifluoromethyl, or a pharmaceutically acceptable salt thereof.

**50.** Use according to claim 45 for the preparation of a pharmaceutical composition comprising as pharmaceutical active ingredient a compound of formula I wherein $R_1$, $R_1'$, $R_2'$, $R_3$, $R_4$ and $R_5$ are hydrogen, $R_2$ is hydrogen or hydroxy and R is $C_2$-$C_7$alkyl, $\alpha,\alpha$-di-$C_1$-$C_4$alkoxy-$C_1$-$C_4$alkyl, $\alpha,\alpha$-dihalo-$C_2$-$C_7$alkyl, $C_3$-$C_6$cycloalkyl, $C_3$-$C_6$cycloalkyl-$C_1$-$C_4$alkyl or benzyl, or a pharmaceutically acceptable salt thereof.

**51.** Use according to claim 44 for the preparation of a pharmaceutical composition comprising as pharmaceutical active ingredient 3-aminopropyl(cyclohexylmethyl)phosphinic acid or a pharmaceutically acceptable salt thereof.

**52.** Use according to claim 44 for the preparation of a pharmaceutical composition comprising as pharmaceutical active ingredient 3-aminopropyl(n-butyl)phosphinic acid or a pharmaceutically acceptable salt thereof.

**53.** Use according to claim 44 for the preparation of a pharmaceutical composition comprising as pharmaceutical active ingredient 3-aminopropyl(diethoxymethyl)phosphinic acid or a pharmaceutically acceptable salt thereof.

**54.** Use according to claim 44 for the preparation of a pharmaceutical composition comprising as pharmaceutical active ingredient 3-aminopropyl(benzyl)phosphinic acid or a pharmaceutically acceptable salt thereof.

**55.** Use according to claim 44 for the preparation of a pharmaceutical composition comprising as pharmaceutical active ingredient 3-aminopropyl(1,1-difluorobutyl)phosphinic acid or a pharmaceutically acceptable salt thereof.

**56.** Use according to claim 44 for the preparation of a pharmaceutical composition comprising as pharmaceutical active ingredient 3-amino-2-hydroxypropyl(cyclohexylmethyl)phosphinic acid or a pharmaceutically acceptable salt thereof.

**57.** Use according to claim 44 for the preparation of a pharmaceutical composition comprising as pharmaceutical active ingredient 3-aminopropyl(cyclohexylmethyl)phosphinic acid or a pharmaceutically acceptable salt thereof.

**58.** Use according to claim 44 for the preparation of a pharmaceutical composition comprising as pharmaceutical active ingredient 3-amino-2-(p-chlorophenyl)propyl(methyl)phosphinic acid or a pharmaceutically acceptable salt thereof.

**59.** Use according to claim 44 for the preparation of a pharmaceutical composition comprising as pharmaceutical active ingredient 3-aminopropyl(n-butyl)phosphinic acid or a pharmaceutically acceptable salt thereof.

**60.** Use according to claim 44 for the preparation of a pharmaceutical composition comprising as pharmaceutical active ingredient 3-aminopropyl(diethoxymethyl)phosphinic acid or a pharmaceutically acceptable salt thereof.

**61.** Use according to claim 44 for the preparation of a pharmaceutical composition comprising as pharmaceutical active ingredient 3-aminopropyl(benzyl)phosphinic acid or a pharmaceutically acceptable salt thereof.

**62.** Use according to claim 44 for the preparation of a pharmaceutical composition comprising as pharmaceutical active ingredient 3-aminopropyl(1,1-difluorobutyl)phosphinic acid or a pharmaceutically acceptable salt thereof.

**63.** Use according to claim 44 for the preparation of a pharmaceutical composition comprising as pharmaceutical active ingredient 3-amino-2-hydroxypropyl(cyclohexylmethyl)phosphinic acid or a pharmaceutically acceptable salt thereof.

**64.** Use according to claim 44 for the preparation of a pharmaceutical composition comprising as pharmaceutical active ingredient 3-amino-2(R)-hydroxypropyl(cyclohexylmethyl)phosphinic acid or a pharmaceutically acceptable salt thereof.

**Claims for the following Contracting States : ES, GR**

**1.** A process for the preparation of a novel araliphatically N-substituted aminoalkanephosphinic acid of formula I

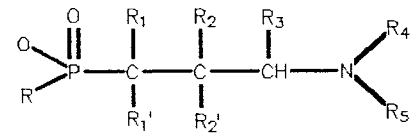

(I),

wherein $R_1$, $R_1'$, $R_2'$ and $R_3$ are hydrogen, $R_2$ is hydrogen or hydroxy, $R_4$ is a phenyl-$C_1$-$C_4$alkyl, diphenyl-$C_1$-$C_4$alkyl or naphthyl-$C_1$-$C_4$alkyl radical each of which is mono-, di- or tri-substituted in the phenyl or naphthyl moiety by $C_1$-$C_4$alkyl, $C_1$-$C_4$alkoxy, hydroxy and/or by halogen, or is a thienyl-, furyl- or pyridyl-$C_1$-$C_4$alkyl radical each of which is unsubstituted or halo-substituted in the thienyl, furyl or pyridyl moiety, $R_5$ is hydrogen, $C_1$-$C_4$alkyl or a group $R_4$, and R is $C_2$-$C_4$alkyl, $C_2$-$C_4$alkenyl, $C_2$-$C_7$alkynyl, oxo-$C_2$-$C_7$alkyl, hydroxy-$C_2$-$C_7$alkyl or dihydroxy-$C_2$-$C_7$alkyl, $\alpha$-hydroxy-$C_3$-$C_5$alkenyl, mono-, di- or tri-fluoro-$C_2$-$C_5$alkyl, mono-, di- or tri-fluoro-$C_2$-$C_5$alkenyl, mono-, di- or tri-fluoro-$\alpha$-hydroxy-$C_2$-$C_5$alkyl, mono-, di- or tri-fluoro-$\alpha$-hydroxy-$C_2$-$C_5$alkenyl, $C_1$-$C_4$alkoxy-$C_1$-$C_4$alkyl, di-$C_1$-$C_4$alkoxy-$C_1$-$C_4$alkyl, $C_1$-$C_4$alkoxy $C_1$-$C_4$alkoxy-$C_2$-$C_7$(hydroxy)alkyl, $C_1$-$C_4$alkoxy-$C_2$-$C_5$(halo)alkyl, $C_1$-$C_4$alkylthio-$C_1$-$C_4$alkyl, di-$C_1$-$C_4$alkylthio-$C_1$-$C_4$alkyl, $C_3$-$C_8$cycloalkyl, $\alpha$-hydroxy-$C_3$-$C_6$cycloalkyl, oxa- or thia-$C_3$-$C_6$cycloalkyl, 1,3-dioxa- or 1,3-dithia-$C_3$-$C_8$cycloalkyl, $C_3$-$C_8$cycloalkyl-$C_1$-$C_4$alkyl, $C_3$-$C_8$cycloalkenyl-$C_1$-$C_4$alkyl, mono-, di- or tri-hydroxy-$C_3$-$C_8$cycloalkenyl, $C_3$-$C_6$cycloalkyl-(hydroxy)-$C_1$-$C_4$alkyl, 1-($C_1$-$C_4$alkylthio-$C_3$-$C_6$cycloalkyl)-1-(hydroxy)-$C_1$-$C_4$alkyl, or mono- or di-phenyl-$C_1$-$C_4$alkyl or naphthyl-$C_1$-$C_4$alkyl each of which is unsubstituted or mono-, di- or tri-substituted by $C_1$-$C_4$alkyl, $C_1$-$C_4$alkoxy, halogen, hydroxy and/or by trifluoromethyl, or unsubstituted or halo-substituted thienyl-, furyl- or pyridyl-$C_1$-$C_4$alkyl, or a salt thereof, which process comprises, in a compound of formula II

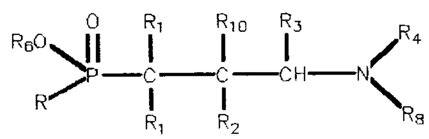

(II),

wherein $R_1$, $R_1'$, $R_2'$ and $R_3$ are hydrogen, $R_6$ is a hydroxy-protecting group, $R_8$ is a group $R_5$ or an amino-protecting group and $R_{10}$ is hydrogen or protected hydroxy, and R, $R_1$, $R_4$ and $R_5$ are as defined above, or in a salt thereof, freeing the hydroxy groups by replacing the hydroxy-protecting group $R_6$ by hydrogen and, where appropriate, removing the amino-protecting group $R_8$ and, where appropriate, freeing the hydroxy groups $R_2$ from the protected hydroxy group $R_{10}$, and, if desired, converting a resulting compound into a different compound of formula I, separating a mixture of isomers obtainable in accordance with the process into the components and separating the preferred isomer, and/or converting a free compound obtainable in accordance with the process into a salt or converting a salt obtainable in accordance with the process into the corresponding free compound.

**2.** A process according to claim 1 for the preparation of a compound of formula I wherein $R_1$, $R_1'$, $R_2'$ and $R_3$ are hydrogen, $R_2$ is hydrogen or hydroxy, $R_4$ is a phenyl- or naphthyl-$C_1$-$C_4$alkyl radical each of which is substituted by $C_1$-$C_4$alkyl, $C_1$-$C_4$alkoxy, hydroxy and/or by halogen, or an unsubstituted or halo-substituted thienyl-, furyl- or pyridyl-$C_1$-$C_4$alkyl radical, $R_5$ is hydrogen, lower alkyl or a group $R_4$, and R is $C_2$-$C_4$alkyl, $C_2$-$C_4$alkenyl, $C_2$-$C_7$alkynyl, oxo-$C_2$-$C_7$alkyl, hydroxy-$C_2$-$C_7$alkyl or dihydroxy-$C_2$-$C_7$alkyl, $\alpha$-hydroxy-$C_3$-$C_5$alkenyl, mono-, di- or mono-, di- or tri-fluoro-$C_2$-$C_5$alkyl, mono-, di- or tri-fluoro-$C_2$-$C_5$alkenyl, mono-, di- or tri-fluoro-$\alpha$-hydroxy-$C_2$-$C_5$alkyl, mono-, di- or tri-fluoro-$\alpha$-hydroxy-$C_2$-$C_5$alkenyl, $C_1$-$C_4$alkoxy-$C_1$-$C_4$alkyl, di-$C_1$-$C_4$alkoxy-$C_1$-$C_4$alkyl, $C_1$-$C_4$alkoxy-$C_2$-$C_7$(hydroxy)alkyl, $C_1$-$C_4$alkoxy-$C_2$-$C_5$(halo)alkyl, $C_1$-$C_4$alkylthio-$C_1$-$C_4$alkyl, di-$C_1$-$C_4$alkylthio-$C_1$-$C_4$alkyl, $C_3$-$C_8$cycloalkyl, $\alpha$-hydroxy-$C_3$-$C_6$cycloalkyl, oxa- or thia-$C_3$-$C_6$cycloalkyl, 1,3-dioxa- or 1,3-dithia-$C_3$-$C_8$cycloalkyl, $C_3$-$C_6$cycloalkyl-(hydroxy)-$C_1$-$C_4$alkyl, 1-($C_1$-$C_4$alkylthio-$C_3$-$C_6$cycloalkyl)-1-(hydroxy)-$C_1$-$C_4$alkyl, or phenyl-$C_1$-$C_4$alkyl or naphthyl-$C_1$-$C_4$alkyl each of which is unsubstituted or substituted by $C_1$-$C_4$alkyl, $C_1$-$C_4$alkoxy, hydroxy and/or by halogen, or a salt thereof.

**3.** A process according to claim 1 for the preparation of a compound of formula I wherein R is $C_3$-$C_7$alkyl, $\alpha,\alpha$-di-$C_1$-$C_4$alkoxy-$C_1$-$C_4$alkyl, $C_3$-$C_6$cycloalkyl-$C_1$-$C_4$alkyl, $C_3$-$C_6$cycloalkenyl-$C_1$-$C_4$alkyl, mono-, di- or tri-hydroxy-$C_3$-$C_6$cycloalkyl-$C_1$-$C_4$alkyl, or benzyl that is unsubstituted or mono-, di- or tri-substituted by $C_1$-$C_4$alkyl, $C_1$-$C_4$alkoxy, hydroxy and/or by halogen, $R_2$ is hydrogen or hydroxy, $R_1$, $R_1'$, $R_2'$ and $R_3$

are hydrogen, $R_4$ is phenyl- or diphenyl-$C_1$-$C_4$alkyl each of which is mono-, di- or tri-substituted by $C_1$-$C_4$alkyl, $C_1$-$C_4$alkoxy and/or by halogen, or is naphthyl-$C_1$-$C_4$alkyl that is unsubstituted or monosubstituted by halogen having an atomic number of up to and including 35, and $R_5$ is hydrogen, $C_1$-$C_4$alkyl or a group $R_4$, or a salt thereof.

**4.** A process according to claim 1 for the preparation of a compound of formula I wherein $R_1$, $R_1'$, $R_2'$ and $R_3$ are hydrogen, $R_2$ is hydrogen or hydroxy, $R_4$ is phenyl-$C_1$-$C_4$alkyl that is monosubstituted by $C_1$-$C_4$alkyl or $C_1$-$C_4$alkoxy or mono- or di-substituted by halogen having an atomic number of up to and including 35, or is naphthyl-, thienyl-, furyl- or pyridyl-$C_1$-$C_4$alkyl each of which is unsubstituted or mono-substituted by halogen having an atomic number of up to and including 35, $R_5$ is hydrogen, $C_1$-$C_4$alkyl or a group $R_4$, and R is $C_3$-$C_7$alkyl, $\alpha$,$\alpha$-di-$C_1$-$C_4$alkoxy-$C_1$-$C_4$alkyl, $C_3$-$C_6$cycloalkyl-$C_1$-$C_4$alkyl,or benzyl that is unsubstituted or substituted by $C_1$-$C_4$alkyl, $C_1$-$C_4$alkoxy or by halogen having an atomic number of up to and including 35, or a salt thereof.

**5.** A process according to claim 1 for the preparation of a compound of formula I wherein $R_1$, $R_1'$, $R_2'$ and $R_3$ are hydrogen, $R_2$ is hydrogen or hydroxy, $R_4$ is $\alpha$-phenyl-$C_1$-$C_4$alkyl that is mono- or di-substituted by halogen having an atomic number of up to and including 35, or is unsubstituted $\alpha$-naphthyl-$C_1$-$C_4$alkyl, $\alpha$-thienyl-$C_1$-$C_4$alkyl, $\alpha$-furyl-$C_1$-$C_4$alkyl or $\alpha$-pyridyl-$C_1$-$C_4$alkyl, $R_5$ is hydrogen, $C_1$-$C_4$alkyl or a group $R_4$, and R is $C_3$-$C_5$alkyl, $\alpha$,$\alpha$-di-$C_1$-$C_4$alkoxymethyl, $C_3$-$C_6$cycloalkyl-$C_1$-$C_4$alkyl, $\alpha$-($C_3$-$C_6$cycloalkenyl)-$C_1$-$C_4$alkyl, $\alpha$-(mono-, di- or tri-hydroxy-$C_3$-$C_6$cycloalkyl)-$C_1$-$C_4$alkyl or benzyl, or a salt thereof.

**6.** A process according to claim 1 for the preparation of a compound of formula I wherein $R_1$, $R_1'$, $R_2'$ and $R_3$ are hydrogen, $R_2$ is hydrogen or hydroxy, $R_4$ is phenyl-$C_1$-$C_4$alkyl that is mono- or di-substituted by halogen having an atomic number of up to and including 35, or is unsubstituted naphthyl-, thienyl-, furyl- or pyridyl-$C_1$-$C_4$alkyl, $R_5$ is hydrogen, $C_1$-$C_4$alkyl or a group $R_4$, and R is $C_3$-$C_5$alkyl, $\alpha$,$\alpha$-di-$C_1$-$C_4$alkoxymethyl, $C_3$-$C_6$cycloalkyl-$C_1$-$C_4$alkyl or benzyl, or a salt thereof.

**7.** A process according to claim 1 for the preparation of 3-(p-chlorobenzylamino)propyl(diethoxymethyl)-phosphinic acid or a salt thereof.

**8.** A process according to claim 1 for the preparation of 3-(p-chlorobenzylamino)-2(S)-hydroxypropyl-(benzyl)phosphinic acid or a salt thereof.

**9.** A process according to claim 1 for the preparation of 3-(p-chlorobenzylamino)propyl(n-butyl)phosphinic acid or a salt thereof.

**10.** A process according to claim 1 for the preparation of 3-(p-chlorobenzylamino)propyl(cyclohexylmethyl)-phosphinic acid or a salt thereof.

**11.** A process according to claim 1 for the preparation of 3-(p-chlorobenzylamino)-2(R)-hydroxypropyl-(benzyl)phosphinic acid or a salt thereof.

**12.** A process according to claim 1 for the preparation of 3-(3,4-dichlorobenzylamino)propyl-(diethoxymethyl)phosphinic acid or a salt thereof.

**13.** A process according to claim 1 for the preparation of 3-[1-(p-chlorophenyl)ethylamino]propyl-(diethoxymethyl)phosphinic acid or a salt thereof.

**14.** A process according to claim 1 for the preparation of 3-(naphth-1-ylmethylamino)propyl-(diethoxymethyl)phosphinic acid or a salt thereof.

**15.** A process according to claim 1 for the preparation of 3-(3,4-dichlorobenzylamino)propyl-(cyclohexylmethyl)phosphinic acid or a salt thereof.

**16.** A process according to claim 1 for the preparation of 3-(pyrid-2-ylmethylamino)-2(S)-hydroxypropyl-(benzyl)phosphinic acid or a salt thereof.

**17.** A process according to claim 1 for the preparation of 3-(p-chlorobenzylaminoyl)-2(S)-hydroxypropyl-(cyclohexylmethyl)phosphinic acid or a salt thereof.

**18.** A process according to claim 1 for the preparation of 3-(3,4-dichlorobenzylaminoyl)-2(S)-hydroxypropyl-(benzyl)phosphinic acid or a salt thereof.

**19.** A process according to claim 1 for the preparation of 3-(p-fluorobenzylamino)propyl(diethoxymethyl)-phosphinic acid or a salt thereof.

**20.** A process according to claim 1 for the preparation of 3-(4-chloro-3-trifluoromethylbenzylamino)propyl-(diethoxymethyl)phosphinic acid or a salt thereof.

**21.** A process according to claim 1 for the preparation of 3-(m-chlorobenzylamino)propyl(diethoxymethyl)-phosphinic acid or a salt thereof.

**22.** A process according to claim 1 for the preparation of 3-(3,4-dichlorobenzylamino)-2(S)-hydroxypropyl-(cyclohexylmethyl)phosphinic acid or a salt thereof.

**23.** A process according to claim 1 for the preparation of 3-[1-(3,4-dichlorophenyl)ethylamino]propyl-(diethoxymethyl)phosphinic acid or a salt thereof.

**24.** A process according to claim 1 for the preparation of 3-[1-(p-chlorophenyl)aminoethyl]-2(S)-hydroxypropyl(benzyl)phosphinic acid or a salt thereof.

**25.** A process according to claim 1 for the preparation of 3-(p-iodobenzylamino)propyl(diethoxymethyl)-phosphinic acid or a salt thereof.

**26.** A process according to claim 1 for the preparation of 3-(2,4-dichlorobenzylamino)propyl-(diethoxymethyl)phosphinic acid or a salt thereof.

**27.** A process according to claim 1 for the preparation of 3-(o-chlorobenzylamino)propyl(diethoxymethyl)-phosphinic acid or a salt thereof.

**28.** A process according to claim 1 for the preparation of 3-(3,4-dichlorobenzylamino)propyl-(cyclopropylmethyl)phosphinic acid or a salt thereof.

**29.** A process according to claim 1 for the preparation of 3-(p-chlorobenzylamino)propyl(tetrahydrofuran-2-yl)phosphinic acid or a salt thereof.

**30.** A process according to claim 1 for the preparation of 3-[N-(p-chlorobenzyl)-N-methylamino]propyl-(diethoxymethyl)phosphinic acid or a salt thereof.

**31.** A process according to claim 1 for the preparation of 3-(p-chlorobenzylamino)-2(S)-hydroxypropyl(p-methylbenzyl)phosphinic acid or a salt thereof.

**32.** A process according to claim 1 for the preparation of 3-(3,4-dichlorobenzylamino)-2(S)-hydroxypropyl(p-methylbenzyl)phosphinic acid or a salt thereof.

**33.** A process according to claim 1 for the preparation of 3-(p-chlorobenzylamino)-2(S)-hydroxypropyl(p-chlorobenzyl)phosphinic acid or a salt thereof.

**34.** A process according to claim 1 for the preparation of 3-(3,4-dichlorobenzylamino)-2(S)-hydroxypropyl(p-chlorobenzyl)phosphinic acid or a salt thereof.

**35.** A process according to claim 1 for the preparation of
3-(p-chlorobenzylamino)-2(S)-hydroxypropyl(diethoxymethyl)phosphinic acid;
3-[1-(p-chlorophenyl)ethylamino]-2(S)-hydroxypropyl(diethoxymethyl)phosphinic acid;
3-(3,4-dichlorobenzylamino)-2(S)-hydroxypropyl(diethoxymethyl)phosphinic acid;

3-[1(3,4-dichlorophenyl)ethylamino]-2(S)-hydroxypropyl(diethoxymethyl)phosphinic acid;
3-[N-(p-chlorobenzyl)-N-methylamino]-2(S)-hydroxypropyl(benzyl)phosphinic acid;
3-(p-chlorobenzylamino)-2(S)-hydroxypropyl(p-methoxybenzyl)phosphinicacid;
3-(p-chlorobenzylamino)-2(S)-hydroxypropyl(3,4-dimethoxybenzyl)phosphinic acid;
3-(3,4-dichlorobenzylamino)-2(S)-hydroxypropyl(3,4-dimethoxybenzyl)phosphinic acid;
3-[2-(p-chlorophenyl)prop-2-ylamino]propyl(diethoxymethyl)phosphinic acid;
3-[2-(3,4-dichlorophenyl)prop-2-ylamino]propyl(diethoxymethyl)phosphinic acid;
3-[2-(p-chlorophenyl)prop-2-ylamino]-2(S)-hydroxypropyl(benzyl)phosphinic acid;
3-[2-(3,4-dichlorophenyl)prop-2-ylamino]-2(S)-hydroxypropyl(benzyl)phosphinic acid;
3-(3,4-dichlorobenzylamino)-2(S)-hydroxypropyl(3,4,5-tri-hydroxycyclohexylmethyl)phosphinic acid;
3-(4-chloro-3-methoxybenzylamino)propyl(diethoxymethyl)phosphinic acid;
3-(4-chloro-3-methoxybenzylamino)-2(S)-hydroxypropyl(benzyl)phosphinic acid;
3-(2-phenylethylamino)propyl(diethoxymethyl)phosphinic acid;
3-(2-phenylethylamino)-2(S)-hydroxypropyl(diethoxymethyl)phosphinic acid;
3-(p-chlorobenzylamino)-2(S)-hydroxypropyl(4-methoxycyclohexyl)phosphinic acid;
3-(3,4-dichloro-6-iodobenzylamino)-2(S)-hydroxypropyl(benzyl)phosphinic acid;
3-(3,4-dichloro-6-iodobenzylamino)propyl(diethoxymethyl)phosphinic acid;
3-(4-chloro-3-iodobenzylamino)propyl(diethoxymethyl)phosphinic acid;
3-(3-chloro-4-iodobenzylamino)propyl(diethoxymethyl)phosphinic acid;
3-(4-chloro-3-iodobenzylamino)-2(S)-hydroxypropyl(benzyl)phosphinicacid;
3-(3-chloro-4-iodobenzylamino)-2(S)-hydroxypropyl(benzyl)phosphinicacid;
3-di(p-chlorobenzyl)aminopropyl(diethoxymethyl)phosphinic acid;
3-di(3,4-dichlorobenzyl)amino-2(S)-hydroxypropyl(benzyl)phosphinic acid;
3-(m-chlorobenzylamino)-2(S)-hydroxypropyl(benzyl)phosphinic acid;
3-(3,4-dichlorobenzyl)amino-2(S)-hydroxypropyl(cis-4,5-di-hydroxycyclohexylmethyl)phosphinic acid;
3-(3,4-dichlorobenzyl)amino-2(S)-hydroxypropyl(cyclohex-3-enylmethyl)phosphinic acid;
3-[1-(3,4-dichlorophenyl)ethylamino]-2(S)-hydroxypropyl(cyclohex-3-enylmethyl)phosphinic acid; or
3-[1-(3,4-dichlorophenyl)ethylamino]-2(S)-hydroxypropyl(cis-4,5-dihydroxycyclohexylmethyl)phosphinic acid
or of a salt of any one of such compounds.

**36.** A process for the preparation of a pharmaceutical composition, which comprises mixing with customary pharmaceutical excipients a compound of formula I

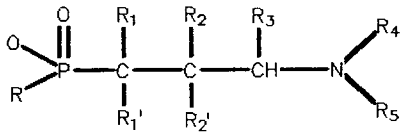

(I),

wherein $R_1$, $R_1'$, $R_2'$ and $R_3$ are hydrogen, $R_2$ is hydrogen or hydroxy, $R_4$ is a phenyl-$C_1$-$C_4$alkyl, diphenyl-$C_1$-$C_4$alkyl or naphthyl-$C_1$-$C_4$alkyl radical each of which is unsubstituted or mono-, di- or tri-substituted in the phenyl or naphthyl moiety by $C_1$-$C_4$alkyl, $C_1$-$C_4$alkoxy, hydroxy and/or by halogen, or is a thienyl-, furyl- or pyridyl-$C_1$-$C_4$alkyl radical each of which is unsubstituted or halo-substituted in the thienyl, furyl or pyridyl moiety, $R_5$ is hydrogen, $C_1$-$C_4$alkyl or a group $R_4$, and R is $C_2$-$C_4$alkyl, $C_2$-$C_4$alkenyl, $C_2$-$C_7$alkynyl, oxo-$C_2$-$C_7$alkyl, hydroxy-$C_2$-$C_7$alkyl or dihydroxy-$C_2$-$C_7$alkyl, $\alpha$-hydroxy-$C_3$-$C_5$alkenyl, mono-, di- or tri-fluoro-$C_2$-$C_5$alkyl, mono-, di- or tri-fluoro-$C_2$-$C_5$alkenyl, mono-, di- or tri-fluoro-$\alpha$-hydroxy-$C_2$-$C_5$alkyl, mono-, di- or tri-fluoro-$\alpha$-hydroxy-$C_2$-$C_5$alkenyl, $C_1$-$C_4$alkoxy-$C_1$-$C_4$alkyl, di-$C_1$-$C_4$alkoxy-$C_1$-$C_4$alkyl, $C_1$-$C_4$alkoxy $C_1$-$C_4$alkoxy-$C_2$-$C_7$(hydroxy)alkyl, $C_1$-$C_4$alkoxy-$C_2$-$C_5$(halo)-alkyl, $C_1$-$C_4$alkylthio-$C_1$-$C_4$alkyl, di-$C_1$-$C_4$alkylthio-$C_1$-$C_4$alkyl, $C_3$-$C_8$cycloalkyl, $\alpha$-hydroxy-$C_3$-$C_6$cycloalkyl, oxa- or thia-$C_3$-$C_6$cycloalkyl, 1,3-dioxa- or 1,3-dithia-$C_3$-$C_8$cycloalkyl, $C_3$-$C_8$cycloalkyl-$C_1$-$C_4$alkyl,$C_3$-$C_8$cycloalkenyl-$C_1$-$C_4$alkyl, mono-, di- or tri-hydroxy-$C_3$-$C_8$cycloalkenyl, $C_3$-$C_6$cycloalkyl-(hydroxy)$C_1$-$C_4$alkyl, 1-($C_1$-$C_4$alkylthio-$C_3$-$C_6$cycloalkyl)-1-(hydroxy)$C_1$-$C_4$alkyl, or mono- or di-phenyl-$C_1$-$C_4$alkyl or naphthyl-$C_1$-$C_4$alkyl each of which is unsubstituted or mono-, di- or tri-substituted by $C_1$-$C_4$alkyl, $C_1$-$C_4$alkoxy, halogen, hydroxy and/or by trifluoromethyl, or unsubstituted or halo-substituted thienyl-, furyl- or pyridyl-$C_1$-$C_4$alkyl, in free form or in the form of a pharmaceutically acceptable salt.

37. A process according to claim 36 for the preparation of a pharmaceutical composition, which comprises mixing a compound according to any one of claims 1 to 35, in free form or in the form of a pharmaceutically acceptable salt, with customary pharmaceutical excipients.

38. A process according to either claim 36 or claim 37 for the preparation of a pharmaceutical composition for the treatment of epilepsies of the "petit mal" type and for suppressing "petit mal"-type conditions which may arise in the case of treatment with phenytoin, carbamazepine, Vigabatrin® and anti-epileptics having the same or a similar profile of action.

**Revendications**
**Revendications pour les Etats contractants suivants : AT, BE, CH, DE, DK, FR, GB, IT, LI, LU, NL, SE**

1. Nouveaux acides aminoalcanephosphiniques araliphatiques N-substitués de formule I

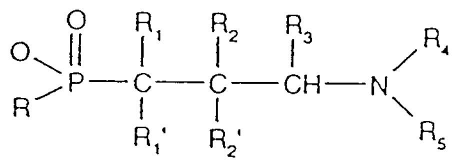

(I) ,

où $R_1$, $R_1'$, $R_2'$ et $R_3$ représentent l'hydrogène, $R_2$ représente l'hydrogène ou l'hydroxy, $R_4$ un reste phénylalkyle en $C_1$-$C_4$, diphénylalkyle en $C_1$-$C_4$ ou naphtylalkyle en $C_1$-$C_4$, mono-, di- ou trisubstitué dans la partie phényle ou naphtyle par un alkyle en $C_1$-$C_4$, un alcoxy en $C_1$-$C_4$, un hydroxy et/ou un halogène ou un reste thiényl-, furyl- ou pyridylalkyle en $C_1$-$C_4$ non substitué ou substitué dans la partie thiényle, furyle ou pyridyle par un halogène, $R_5$ représente l'hydrogène, un alkyle en $C_1$-$C_4$ ou un groupe $R_4$ et R représente un alkyle en $C_2$-$C_4$, un alcényle en $C_2$-$C_4$, un alcynyle en $C_2$-$C_7$, un oxoalkyle en $C_2$-$C_7$, un hydroxyalkyle en $C_2$-$C_7$ ou un dihydroxyalkyle en $C_2$-$C_7$, un $\alpha$-hydroxyalcényle en $C_3$-$C_5$, un mono-, di- ou trifluoroalkyle en $C_2$-$C_5$, un mono-, di- ou trifluoroalcényle en $C_2$-$C_5$, un mono-, di- ou trifluoro-$\alpha$-hydroxyalkyle en $C_2$-$C_5$, un mono-, di- ou trifluoro-$\alpha$-hydroxyalcényle en $C_2$-$C_5$, un alcoxy en $C_1$-$C_4$, alkyle en $C_1$-$C_4$, un dialcoxy en $C_1$-$C_4$, alkyle en $C_1$-$C_4$, un alcoxy en $C_1$-$C_4$, alcoxy en $C_1$-$C_4$(hydroxy)alkyle en $C_2$-$C_7$, un alcoxy en $C_1$-$C_4$(halo)alkyle en $C_2$-$C_5$, un alkylthio en $C_1$-$C_4$ alkyle en $C_1$-$C_4$, un dialkyle en $C_1$-$C_4$ thioalkyle en $C_1$-$C_4$, un cycloalkyle en $C_3$-$C_8$, un $\alpha$-hydroxycycloalkyle en $C_3$-$C_6$, un oxa- ou thiacycloalkyle en $C_3$-$C_6$, un 1,3-dioxa- ou 1,3-dithiacycloalkyle en $C_3$-$C_8$, un cycloalkyle en $C_3$-$C_8$ alkyle en $C_1$-$C_4$, un cycloalcényle en $C_3$-$C_8$alkyle en $C_1$-$C_4$, un mono-, di- ou trihydroxycycloalcényle en $C_3$-$C_8$, un cycloalkyle en $C_3$-$C_6$(hydroxy)alkyle en $C_1$-$C_4$, un 1-(alkyle en $C_1$-$C_4$ thiocycloalkyle en $C_3$-$C_6$)-1-hydroxyalkyle en $C_1$-$C_4$, un mono- ou diphénylalkyle en $C_1$-$C_4$ non substitué ou mono-, di- ou trisubstitué par un alkyle en $C_1$-$C_4$, un alcoxy en $C_1$-$C_4$, un halogène, un hydroxy et/ou le trifluorométhyle, un naphtylalkyle en $C_1$-$C_4$ ou un thiényl-, furyl- ou pyridylalkyle en $C_1$-$C_4$ non substitué ou substitué par un halogène et leurs sels.

2. Composés selon la revendication 1 de formule I où $R_1$, $R_1'$, $R_2'$ et $R_3$ représentent l'hydrogène, $R_2$ l'hydrogène ou l'hydroxy, $R_4$ un reste phényl- ou naphtylalkyle en $C_1$-$C_4$ substitué par un alkyle en $C_1$-$C_4$, un alcoxy en $C_1$-$C_4$, l'hydroxy et/ou un halogène ou un reste thiényl-, furyl- ou pyridylalkyle en $C_1$-$C_4$ non substitué ou substitué par un halogène, $R_5$ représente l'hydrogène, un alkyle inférieur ou un groupe $R_4$ et R représente un alkyle en $C_2$-$C_4$, un alcényle en $C_2$-$C_4$, un alcynyle en $C_2$-$C_7$, un oxoalkyle en $C_2$-$C_7$, un hydroxyalkyle en $C_2$-$C_7$ ou un dihydroxyalkyle en $C_2$-$C_7$, un $\alpha$-hydroxyalcényle en $C_3$-$C_5$, un mono-, di- ou trifluoroalkyle en $C_2$-$C_5$, un mono-, di- ou trifluoroalcényle en $C_2$-$C_5$, un mono-, di- ou trifluoro-$\alpha$-hydroxyalkyle en $C_2$-$C_5$, un mono-, di- ou trifluoro-$\alpha$-hydroxyalcényle en $C_2$-$C_5$, un alcoxy en $C_1$-$C_4$, alkyle en $C_1$-$C_4$, un dialcoxy en $C_1$-$C_4$ alkyle en $C_1$-$C_4$, un alcoxy en $C_1$-$C_4$ -(hydroxy)alkyle en $C_2$-$C_7$, un alcoxy en $C_1$-$C_4$ (halo)alkyle en $C_2$-$C_5$, un alkyle en $C_1$-$C_4$ thioalkyle en $C_1$-$C_4$, un dialkyle en $C_1$-$C_4$ thioalkyle en $C_1$-$C_4$, un cycloalkyle en $C_3$-$C_8$, un $\alpha$-hydroxycycloalkyle en $C_3$-$C_6$, un oxa- ou thiacycloalkyle en $C_3$-$C_6$, un 1,3-dioxa- ou 1,3-dithiacycloalkyle en $C_3$-$C_8$, un cycloalkyle en $C_3$-$C_6$ (hydroxy)alkyle en $C_1$-$C_4$, un 1-(alkyle en $C_1$-$C_4$ thiocycloalkyle en $C_3$-$C_6$)-1-hydroxyalkyle en $C_1$-$C_4$ ou un phénylalkyle en $C_1$-$C_4$ ou un naphtylalkyle en $C_1$-$C_4$ non substitué ou substitué par un alkyle en $C_1$-$C_4$, un alcoxy en $C_1$-$C_4$, un hydroxy et/ou un halogène et leurs sels.

**3.** Composés selon la revendication 1 de formule I où R représente un alkyle en $C_3$-$C_7$, un $\alpha,\alpha$-dialcoxy en $C_1$-$C_4$ alkyle en $C_1$-$C_4$, un cycloalkyle en $C_3$-$C_6$ alkyle en $C_1$-$C_4$, un cycloalcényle en $C_3$-$C_6$ alkyle en $C_1$-$C_4$, un mono-, di- ou trihydroxycycloalkyle en $C_3$-$C_6$ alkyle en $C_1$-$C_4$ ou un benzyle non substitué ou mono-, di- ou trisubstitué par un alkyle en $C_1$-$C_4$, un alcoxy en $C_1$-$C_4$, l'hydroxy et/ou un halogène, $R_2$ représente l'hydrogène ou un hydroxy, $R_1$, $R_1'$, $R_2'$ et $R_3$ représentent l'hydrogène, $R_4$ représente un phényl- ou diphénylalkyle en $C_1$-$C_4$ mono-, di- ou trisubstitué par un alkyle en $C_1$-$C_4$, un alcoxy en $C_1$-$C_4$ et/ou un halogène ou un naphtylalkyle en $C_1$-$C_4$ non substitué ou monosubstitué par un halogène ayant un numéro atomique allant jusqu'à 35 et $R_5$ représente l'hydrogène, un alkyle en $C_1$-$C_4$ ou un groupe $R_4$ et leurs sels.

**4.** Composés selon la revendication 1 de formule I où $R_1$, $R_1'$, $R_2'$ et $R_3$ représentent l'hydrogène $R_2$ représente l'hydrogène ou l'hydroxy, $R_4$ un phénylalkyle en $C_1$-$C_4$ monosubstitué par un alkyle en $C_1$-$C_4$ ou par un alcoxy en $C_1$-$C_4$ ou mono- ou disubstitué par un halogène ayant un numéro atomique allant jusqu'à 35 ou un naphtyl-, thiényl-, furyl- ou pyridylalkyle en $C_1$-$C_4$ non substitué ou monosubstitué par un halogène ayant un numéro atomique allant jusqu'à 35, $R_5$ représente l'hydrogène, un alkyle en $C_1$-$C_4$ ou un groupe $R_4$ et R représente un alkyle en $C_3$-$C_7$, un $\alpha,\alpha$-dialcoxy en $C_1$-$C_4$ alkyle en $C_1$-$C_4$, un cycloalkyle en $C_3$-$C_6$ alkyle en $C_1$-$C_4$ ou un benzyle non substitué ou substitué par un alkyle en $C_1$-$C_4$, un alcoxy en $C_1$-$C_4$ ou un halogène ayant un numéro atomique allant jusqu'à 35 et leurs sels.

**5.** Composés selon la revendication 1 de formule I où $R_1$, $R_1'$, $R_2'$ et $R_3$ représentent l'hydrogène, $R_2$l'hydrogène ou l'hydroxy, $R_4$ représente un $\alpha$-phénylalkyle en $C_1$-$C_4$ mono- ou disubstitué par un halogène ayant un numéro atomique allant jusqu'à 35 ou un -naphtylalkyle en $C_1$-$C_4$, un $\alpha$-thiénylalkyle en $C_1$-$C_4$, un $\alpha$-furylalkyle en $C_1$-$C_4$ ou un $\alpha$-pyridylalkyle en $C_1$-$C_4$ non substitué $R_5$ représente l'hydrogène, un alkyle en $C_1$-$C_4$ ou un groupe $R_4$ et R un alkyle en $C_3$-$C_5$, un $\alpha$, $\alpha$-dialcoxy en $C_1$-$C_4$ méthyle, un cycloalkyle en $C_3$-$C_6$ alkyle en $C_1$-$C_4$, un $\alpha$-(cycloalcényle en $C_3$-$C_6$)alkyle en $C_1$-$C_4$, un $\alpha$-(mono-, di- ou trihydroxycycloalkyle en $C_3$-$C_6$)alkyle en $C_1$-$C_4$ ou un benzyle et leurs sels.

**6.** Composés selon la revendication 1 de formule I où $R_1$, $R_1'$, $R_2'$ et $R_3$ représentent l'hydrogène, $R_2$ représente l'hydrogène ou l'hydroxy, $R_4$ un phénylalkyle en $C_1$-$C_4$ mono- ou disubstitué par un halogène ayant un numéro atomique allant jusqu'à 35 ou un naphtyl-, thiényl-, furyl- ou pyridylalkyle en $C_1$-$C_4$ non substitué, $R_5$ l'hydrogène, un alkyle en $C_1$-$C_4$ ou un groupe $R_4$ et R représente un alkyle en $C_3$-$C_5$, un $\alpha,\alpha$-dialcoxy en $C_1$-$C_4$ méthyle, un cycloalkyle en $C_3$-$C_6$ alkyle en $C_1$-$C_4$ ou un benzyle et leurs sels.

**7.** L'acide 3-(p-chlorobenzylamino)propyl(diéthoxyméthyl)phosphinique selon la revendication 1 ou l'un de ses sels.

**8.** L'acide 3-(p-chlorobenzylamino)-2(S)-hydroxypropyl(benzyl)phosphinique selon la revendication 1 ou l'un de ses sels.

**9.** L'acide 3-(p-chlorobenzylamino)propyl(n-butyl)phosphinique selon la revendication 1 ou l'un de ses sels.

**10.** L'acide 3-(p-chlorobenzylamino)propyl(cyclohexylméthyl)phosphinique selon la revendication 1 ou l'un de ses sels.

**11.** L'acide 3-(p-chlorobenzylamino)-2(R)-hydroxypropyl(benzyl)phosphinique selon la revendication 1 ou l'un de ses sels.

**12.** L'acide 3-(3,4-dichlorobenzylamino)propyl(diéthoxyméthyl)phosphinique selon la revendication 1 ou l'un de ses sels.

**13.** L'acide 3-[1-(p-chlorophényl)éthylamino]propyl(diéthoxyméthyl)phosphinique selon la revendication 1 ou l'un de ses sels.

**14.** L'acide 3-(naphtyl-1-ylméthylamino)propyl(diéthoxyméthyl)phosphinique selon la revendication 1 ou l'un de ses sels.

**15.** L'acide 3-(3,4-dichlorobenzylamino)propyl(cyclohexylméthyl)phosphinique selon la revendication 1 ou l'un de ses sels.

**16.** L'acide 3-(pyrid-2-ylméthylamino)-2(S)-hydroxypropyl(benzyl)phosphinique selon la revendication 1 ou l'un de ses sels.

**17.** L'acide 3-(p-chlorobenzylaminoyl)-2(S)-hydroxypropyl(cyclohexylméthyl)phosphinique selon la revendication 1 ou l'un de ses sels.

**18.** L'acide 3-(3,4-dichlorobenzylaminoyl)-2(S)-hydroxypropyl(benzyl)phosphinique selon la revendication 1 ou l'un de ses sels.

**19.** L'acide 3-(p-fluorobenzylamino)propyl(diéthoxyméthyl)phosphinique selon la revendication 1 ou l'un de ses sels.

**20.** L'acide 3-(4-chloro-3-trifluorométhylbenzylamino)propyl(diéthoxyméthyl)phosphinique selon la revendication 1 ou l'un de ses sels.

**21.** L'acide 3-(m-chlorobenzylamino)propyldiéthoxyméthyl)phosphinique selon la revendication 1 ou l'un de ses sels.

**22.** L'acide 3-(3,4-dichlorobenzylamino)-2(S)-hydroxypropyl(cyclohexylméthyl)phosphinique selon la revendication 1 ou l'un de ses sels.

**23.** L'acide 3-[1-(3,4-dichlorophényl)éthylamino]propyl(diéthoxyméthyl)phosphinique selon la revendication 1 ou l'un de ses sels.

**24.** L'acide 3-[1-(p-chlorophényl)éthylamino]-2(S)-hydroxypropyl(benzyl)phosphinique selon la revendication 1 ou l'un de ses sels.

**25.** L'acide 3-(p-iodobenzylamino)propyl(diéthoxyméthyl)phosphinique selon la revendication 1 ou l'un de ses sels.

**26.** L'acide 3-(2,4-dichlorobenzylamino)propyl(diéthoxyméthyl)phosphinique selon la revendication 1 ou l'un de ses sels.

**27.** L'acide 3-(o-chlorobenzylamino)propyl(diéthoxyméthyl)phosphinique selon la revendication 1 ou l'un de ses sels.

**28.** L'acide 3-(3,4-dichlorobenzylamino)propyl(cyclopropylméthyl)phosphinique selon la revendication 1 ou l'un de ses sels.

**29.** L'acide 3-(p-chlorobenzylamino)propyl(tétrahydrofuran-2-yl)phosphinique selon la revendication 1 ou l'un de ses sels.

**30.** L'acide 3-[N-(p-chlorobenzyl)-N-méthyl)amino]propyl(diéthoxyméthyl)phosphinique selon la revendication 1 ou l'un de ses sels.

**31.** L'acide 3-(p-chlorobenzylamino)-2(S)-hydroxypropyl(p-méthylbenzyl)phosphinique selon la revendication 1 ou l'un de ses sels.

**32.** L'acide 3-(3,4-dichlorobenzylamino)-2(S)-hydroxypropyl(p-méthylbenzyl)phosphinique selon la revendication 1 ou l'un de ses sels.

**33.** L'acide 3-(p-chlorobenzylamino)-2(S)-hydroxypropyl(p-chlorobenzyl)phosphinique selon la revendication 1 ou l'un de ses sels.

**34.** L'acide 3-(3,4-dichlorobenzylamino)-2(S)-hydroxypropyl(p-chlorobenzyl)phosphinique selon la revendication 1 ou l'un de ses sels.

**35.** L'acide 3-(p-chlorobenzylamino)-2(S)-hydroxypropyl(diéthoxyméthyl)phosphinique,
l'acide 3-[1-(p-chlorophényl)éthylamino]-2(S)-hydroxypropyl(diéthoxyméthyl)phosphinique,
l'acide 3-(3,4-dichlorobenzylamino)-2(S)-hydroxypropyl(diéthoxyméthyl)phosphinique,
l'acide 3-[1-(3,4-dichlorophényl)éthylamino]-2(S)-hydroxypropyl(diéthoxyméthyl)phosphinique,
l'acide 3-[N-(p-chlorobenzyl)-N-méthylamino]-2(S)-hydroxypropyl(benzyl)phosphinique,
l'acide 3-(p-chlorobenzylamino)-2(S)-hydroxypropyl(p-méthoxybenzyl)phosphinique,
l'acide 3-(p-chlorobenzylamino)-2(S)-hydroxypropyl(3,4-diméthoxybenzyl)phosphinique,
l'acide 3-(3,4-dichlorobenzylamino)-2(S)-hydroxypropyl(3,4-diméthoxybenzyl)phosphinique,
l'acide 3-[2-(p-chlorophényl)prop-2-ylamino]propyl(diéthoxyméthyl)phosphinique,
l'acide 3-[2-(3,4-dichlorophényl)prop-2-ylamino]propyl(diéthoxyméthyl)phosphinique,
l'acide 3-[2-(p-chlorophényl)prop-2-ylamino]-2(S)-hydroxypropyl(benzyl)phosphinique,
l'acide 3-[2-(3,4-dichlorophényl)prop-2-ylamino]-2(S)-hydroxypropyl(benzyl)phosphinique,
l'acide 3-(3,4-dichlorobenzylamino)-2(S)-hydroxypropyl(3,4,5-trihydroxycyclohexylméthyl)phosphinique,
l'acide 3-(4-chloro-3-méthoxybenzylamino)propyl(diéthoxyméthyl)phosphinique,
l'acide 3-(4-chloro-3-méthoxybenzylamino)-2(S)-hydroxypropyl(benzyl)phosphinique,
l'acide 3-(2-phényléthylamino)propyl(diéthoxyméthyl)phosphinique,
l'acide 3-(2-phényléthylamino)-2(S)-hydroxypropyl(diéthoxyméthyl)phosphinique,
l'acide 3-(p-chlorobenzylamino)-2(S)-hydroxypropyl(4-méthoxycyclohexyl)phosphinique,
l'acide 3-(3,4-dichloro-6-iodobenzylamino)-2(S)-hydroxypropyl(benzyl)phosphinique,
l'acide 3-(3,4-dichloro-6-iodobenzylamino)propyl(diéthoxyméthyl)phosphinique,
l'acide 3-(4-chloro-3-iodobenzylamino)propyl(diéthoxyméthyl)phosphinique,
l'acide 3-(3-chloro-4-iodobenzylamino)propyl(diéthoxyméthyl)phosphinique,
l'acide 3-(4-chloro-3-iodobenzylamino)-2(S)-hydroxypropyl(benzyl)phosphinique,
l'acide 3-(3-chloro-4-iodobenzylamino)-2(S)-hydroxypropyl(benzyl)phosphinique,
l'acide 3-di-(p-chlorobenzyl)aminopropyl(diéthoxyméthyl)phosphinique,
l'acide 3-di-(3,4-dichlorobenzyl)amino-2(S)-hydroxypropyl(benzyl)phosphinique,
l'acide 3-(m-chlorobenzylamino)-2(S)-hydroxypropyl(benzyl)phosphinique,
l'acide 3-(3,4-dichlorobenzyl)amino-2(S)-hydroxypropyl(cis-4,5-dihydroxycyclohexylméthyl)-phosphinique,
l'acide 3-(3,4-dichlorobenzyl)amino-2(S)-hydroxypropyl(cyclohex-3-énylméthyl)phosphinique,
l'acide 3-[1-(3,4-dichlorophényl)éthylamino]-2(S)-hydroxypropyl(cyclohex-3-énylméthyl)phosphinique ou
l'acide 3-[1-(3,4-dichlorophényl)éthylamino]-2(S)-hydroxypropyl(cis-4,5-dihydroxycyclohexylméthyl)-phosphinique
selon la revendication 1 ou l'un de leurs sels.

**36.** Un composé de formule I

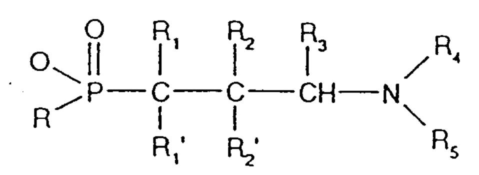

(I),

où $R_1$, $R_1'$, $R_2'$ et $R_3$ représentent l'hydrogène, $R_2$ représente l'hydrogène ou l'hydroxy, $R_4$ un reste phénylalkyle en $C_1$-$C_4$, diphénylalkyle en $C_1$-$C_4$ ou naphtylalkyle en $C_1$-$C_4$ non substitué ou mono-, di- ou trisubstitué dans la partie phényle ou naphtyle par un alkyle en $C_1$-$C_4$, un alcoxy en $C_1$-$C_4$, un hydroxy et/ou un halogène ou un reste thiényl-, furyl- ou pyridylalkyle en $C_1$-$C_4$ non substitué ou substitué dans la partie thiényle, furyle ou pyridyle par un halogène, $R_5$ représente l'hydrogène, un alkyle en $C_1$-$C_4$ ou un groupe $R_4$ et R représente un alkyle en $C_1$-$C_4$, un alcényle en $C_2$-$C_4$, un alcynyle en $C_2$-$C_7$, un oxoalkyle en $C_2$-$C_7$, un hydroxyalkyle en $C_2$-$C_7$ ou un dihydroxyalkyle en $C_2$-$C_7$, un $\alpha$-hydroxyalcényle en $C_3$-$C_5$, un mono-, di- ou trifluoroalkyle en $C_2$-$C_5$, un mono-, di- ou trifluoroalcényle en $C_2$-$C_5$, un mono-, di- ou trifluoro-$\alpha$-hydroxyalkyle en $C_2$-$C_5$, un mono-, di- ou trifluoro-$\alpha$-hydroxyalcényle en $C_2$-$C_5$, un alcoxy en $C_1$-$C_4$ alkyle en $C_1$-$C_4$, un dialcoxy en $C_1$-$C_4$ alkyle en $C_1$-$C_4$, un alcoxy en $C_1$-$C_4$ alcoxy en $C_1$-$C_4$(hydroxy)alkyle en $C_2$-$C_7$, un alcoxy en $C_1$-$C_4$(halo)alkyle en $C_2$-

$C_5$, un alkylthio en $C_1$-$C_4$ alkyle en $C_1$-$C_4$, un dialkyle en $C_1$-$C_4$ thioalkyle en $C_1$-$C_4$, un cycloalkyle en $C_3$-$C_8$, un $\alpha$-hydroxycycloalkyle en $C_3$-$C_6$, un oxa- ou thiacycloalkyle en $C_3$-$C_6$, un 1,3-dioxa- ou 1,3-dithiacycloalkyle en $C_3$-$C_8$, un cycloalkyle en $C_3$-$C_8$ alkyle en $C_1$-$C_4$, un cycloalcényle en $C_3$-$C_8$ alkyle en $C_1$-$C_4$, un mono-, di- ou trihydroxycycloalcényle en $C_3$-$C_8$, un cycloalkyle en $C_3$-$C_6$(hydroxy)alkyle en $C_1$-$C_4$, un 1-(alkyle en $C_1$-$C_4$ thiocycloalkyle en $C_3$-$C_6$-1-hydroxyalkyle en $C_1$-$C_4$, un mono- ou diphénylalkyle en $C_1$-$C_4$ non substitué ou mono-, di- ou trisubstitué par un alkyle en $C_1$-$C_4$, un alcoxy en $C_1$-$C_4$, un halogène, un hydroxy et/ou le trifluorométhyle, un naphtylalkyle en $C_1$-$C_4$ ou un thiényl-, furyl- ou pyridylalkyle en $C_1$-$C_4$ non substitué ou substitué par un halogène, sous forme libre ou sous forme de sels pharmaceutiquement utilisables destinés à être utilisés dans un procédé pour le traitement thérapeutique du corps humain ou animal.

**37.** Composés selon l'une des revendications 2, 4 et 6 à 11 destinés à être utilisés selon la revendication 36.

**38.** Composés selon l'une des revendications 1, 3, 5 et 12 à 35 destinés à être utilisés selon la revendication 36.

**39.** Préparations pharmaceutiques contenant, comme substance active pharmaceutique, un composé selon la revendication 36 sous forme libre ou sous forme d'un sel pharmaceutiquement utilisable.

**40.** Préparations pharmaceutiques selon la revendication 36 contenant, comme substance active pharmaceutique, un composé selon l'une des revendications 2, 4 et 6 à 11 sous forme libre ou sous forme d'un sel pharmaceutiquement utilisable.

**41.** Préparations pharmaceutiques selon la revendication 36 contenant, comme substance active pharmaceutique, un composé selon l'une des revendications 1, 3, 5 et 12 à 35 sous forme libre ou sous forme d'un sel pharmaceutiquement utilisable.

**42.** Préparations pharmaceutiques selon l'une des revendications 39 à 41 destinées au traitement des épilepsies des différentes formes du "petit mal" ainsi qu'à la suppression des états analogues au "petit mal" pouvant intervenir lors du traitement avec la phénytoïne, la carbamazépine, la Vigabatrine® et avec les antiépileptiques présentant un profil d'action identique ou analogue.

**43.** Procédé pour la préparation des nouveaux acides aminoalcanephosphiniques araliphatiques N-substitués, caractérisé en ce que l'on libère dans un composé de formule II

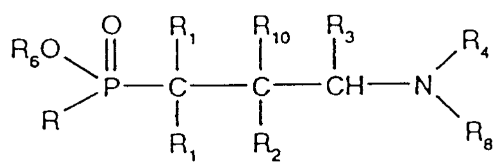

(II),

où $R_1'$, $R_2'$ et $R_3$ représentent l'hydrogène, $R_6$ un groupe protecteur de l'hydroxy, $R_8$ un groupe $R_5$ ou un groupe protecteur de l'amino et $R_{10}$ représente l'hydrogène ou un hydroxy protégé, R, $R_1$, $R_4$ et $R_5$ ayant la signification qui a été indiquée ou dans l'un de ses sels les groupes hydroxy, en remplaçant le groupe protecteur de l'hydroxy $R_6$ par l'hydrogène et en ce que éventuellement l'on clive le groupe protecteur de l'amino $R_8$ et en ce que éventuellement l'on libère les groupes hydroxy $R_2$ des groupes hydroxy protégés $R_{10}$ et, si désiré, en ce que l'on transforme le composé obtenu en un autre composé de formule I en ce que l'on sépare le mélange d'isomères obtenu conformément au procédé de l'invention en ses composants et en ce que l'on sépare l'isomère préféré et/ou en ce que l'on transforme le composé libre obtenu par le procédé conforme à l'invention en un sel ou le sel obtenu par le procédé conforme à l'invention en un composé libre correspondant.

**44.** Utilisation des composés ayant des propriétés antagonistes $GABA_B$ pour la fabrication d'une préparation pharmaceutique destinée au traitement des épilepsies des différentes formes du "petit mal" ou à supprimer les états analogues au "petit mal" pouvant intervenir lors du traitement avec la phénytoïne, la carbamazépine, la Vigabatrine® et les antiépileptiques présentant un profil d'action indentique ou

analogue.

**45.** Utilisation selon la revendication 44 pour la fabrication d'une préparation pharmaceutique destinée au traitement des épilepsies des différentes formes du "petit mal".

**46.** Utilisation selon la revendication 44 pour la fabrication de préparations pharmaceutiques contenant, comme substance active pharmaceutique, un composé de formule I

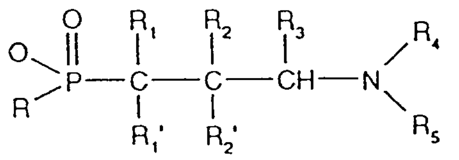

(I),

où l'un des restes $R_1$, $R_2$ et $R_3$ représente l'hydrogène, un alkyle en $C_1$-$C_7$, un alcényle en $C_2$-$C_7$, un alcynyle en $C_2$-$C_7$, un cycloalkyle en $C_3$-$C_8$, un cycloalcényle en $C_3$-$C_8$ alkyle en $C_1$-$C_4$, un mono-, di- ou trihydroxycycloalkyle en $C_3$-$C_8$ alkyle en $C_1$-$C_4$, un phénylalkyle en $C_1$-$C_4$, un diphénylalkyle en $C_1$-$C_4$ ou un naphtylalkyle en $C_1$-$C_4$ non substitué ou mono- ou polysubstitué dans la partie phényle ou naphtyle par un alkyle en $C_1$-$C_4$, un alcoxy en $C_1C_4$, un halogène et/ou le trifluorométhyle, un phényle ou un naphtyle non substitué ou substitué par un halogène, un alkyle en $C_1$-$C_4$, un alcoxy en $C_1$-$C_4$ et/ou le trifluorométhyle, un autre reste représente l'hydrogène ou dans le cas où $R_1$ ou $R_2$ représente l'hydroxy ou dans le cas où $R_1$ représente un halogène ou dans le cas où $R_2$ ensemble avec $R_2$' représente un oxo et le dernier reste représente l'hydrogène, $R_1$' est l'hydrogène ou un halogène, $R_2$' est l'hydrogène, un hydroxy ou ensemble avec $R_2$ un oxo, $R_4$ et $R_5$ représentent l'hydrogène ou $R_4$ un phénylalkyle en $C_1$-$C_4$, un diphénylalkyle en $C_1$-$C_4$, un naphtylalkyle en $C_1$-$C_4$, un thiénylalkyle en $C_1$-$C_4$, un furylalkyle en $C_1$-$C_4$ ou un pyridylalkyle en $C_1$-$C_4$ non substitué ou mono- ou polysubstitué dans la partie phényle, naphtyle, thiényle, furyle ou pyridyle par un alkyle en $C_1$-$C_4$, un alcoxy en $C_1$-$C_4$, un halogène et/ou le trifluorométhyle et $R_5$ représente l'hydrogène, un alkyle en $C_1$-$C_7$, un alcényle en $C_2$-$C_7$, un alcynyle en $C_2$-$C_7$ ou un groupe $R_4$ et R représente un alkyle en $C_2$-$C_7$, un alcényle en $C_2$-$C_7$, un alcynyle en $C_2$-$C_7$, un cycloalkyle en $C_3$-$C_8$, un cycloalkyle en $C_3$-$C_8$ alkyle en $C_1$-$C_4$, un cycloalcényle en $C_3$-$C_8$ alkyle en $C_1$-$C_4$, un mono-, di- ou trihydroxycycloalkyle en $C_3$-$C_8$ alkyle en $C_1$-$C_4$, un cycloalkyle en $C_3$-$C_8$ hydroxyalkyle en $C_1$-$C_4$, un (alkyle en $C_1$-$C_4$ thio)cycloalkyle(hydroxy)-alkyle en $C_1$-$C_4$, un phényl- ou naphtylalkyle inférieur en $C_1$-$C_4$ non substitué ou mono- ou polysubstitué dans la partie phényle ou naphtyle par un alkyle en $C_1$-$C_4$, un alcoxy en $C_1$-$C_4$, un halogène et/ou le trifluorométhyle, un phényle ou naphtyle non substitué ou substitué par un halogène, un alkyle en $C_1$-$C_4$, un alcoxy en $C_1$-$C_4$ et/ou le trifluorométhyle ou représente le méthyle, si $R_1$ représente l'hydrogène ou l'hydroxy, $R_2$ un reste aromatique tel que décrit précédemment et $R_1$', $R_2$' ainsi que $R_3$ représentent l'hydrogène ou l'un de ses sels pharmaceutiquement utilisable.

**47.** Utilisation selon la revendication 44 pour la fabrication de préparations pharmaceutiques contenant, comme substance active pharmaceutique, un composé de formule I où l'un des restes $R_1$, $R_2$ et $R_3$ représente l'hydrogène, un alkyle en $C_1$-$C_4$, un cycloalkyle en $C_3$-$C_6$, un phénylalkyle en $C_1$-$C_4$ non substitué ou mono- ou polysubstitué dans la partie phényle ou naphtyle par un alkyle en $C_1$-$C_4$, un alcoxy en $C_1$-$C_4$, un halogène et/ou le trifluorométhyle, un phényle non substitué ou mono- ou polysubstitué dans la partie phényle ou naphtyle par un alkyle en $C_1$-$C_4$, un alcoxy en $C_1$-$C_4$, un halogène et/ou le trifluorométhyle, un autre reste représente l'hydrogène ou dans le cas où $R_1$ ou $R_2$ représente l'hydroxy, le dernier geste représente l'hydrogène, $R_1$' est l'hydrogène, $R_2$' représente l'hydrogène, l'hydroxy ou ensemble avec $R_2$ l'oxo, $R_4$ et $R_5$ représentent l'hydrogène ou $R_4$ représente un reste phénylalkyle en $C_1$-$C_4$ non substitué ou mono- ou polysubstitué dans la partie phényle par un alkyle en $C_1$-$C_4$, un alcoxy en $C_1$-$C_4$, un halogène et/ou le trifluorométhyle et $R_5$ représente l'hydrogène, un alkyle en $C_1$-$C_4$ ou un groupe $R_4$ et R représente un alkyle en $C_2$-$C_7$, un $\alpha,\alpha$-dialcoxy en $C_1$-$C_4$ alkyle en $C_2$-$C_7$, un $\alpha,\alpha$-dihalogénoalkyle en $C_2$-$C_7$, un cycloalkyle en $C_3$-$C_6$, un cycloalkyle en $C_3$-$C_6$ alkyle en $C_1$-$C_4$, un cycloalcényle en $C_3$-$C_8$ alkyle en $C_1$-$C_4$, un mono-, di- ou trihydroxycycloalkyle en $C_3$-$C_8$ alkyle en $C_1$-$C_4$, le benzyle ou, si $R_2$ représente un halogénophényle et $R_1$, $R_1$', $R_2$' ainsi que $R_3$ représentent l'hydrogène, R représente le méthyle, ou l'un de ses sels pharmaceutiquement utilisable.

**48.** Utilisation selon la revendication 44 pour la fabrication de préparations pharmaceutiques contenant, comme substance active pharmaceutique, un composé de formule I où

a) $R_1$, $R_1'$, $R_2'$, $R_3$, $R_4$ et $R_5$ représentent l'hydrogène, $R_2$ un alkyle en $C_1$-$C_4$, un cycloalkyle en $C_3$-$C_6$, le benzyle, le phényle, un halogénophényle ou le naphtyle et $R_2'$ l'hydrogène ou l'hydroxy ou $R_2$ l'hydroxy et $R_2'$ l'hydrogène ou $R_2$ et $R_2'$ représentent ensemble un groupe oxo ou

b) $R_1$ représente l'hydroxy, un alkyle en $C_1$-$C_4$,un cycloalkyle en $C_3$-$C_6$, le benzyle, le phényle, un halogénophényle ou le naphtyle et $R_1'$, $R_2$, $R_2'$, $R_3$, $R_4$ et $R_5$ représentent l'hydrogène ou

c) $R_1$ représente un halogène, $R_1'$ représentant l'hydrogène ou un halogène et $R_2$, $R_2'$, $R_3$, $R_4$ et $R_5$ représentent l'hydrogène et R représente un alkyle en $C_2$-$C_7$, un $\alpha,\alpha$-dialcoxy en $C_1$-$C_4$ alkyle en $C_2$-$C_7$, un $\alpha,\alpha$-dihalogénoalkyle en $C_2$-$C_7$, un cycloalkyle en $C_3$-$C_6$, un cycloalkyle en $C_3$-$C_6$ alkyle en $C_1$-$C_4$, le benzyle ou, si $R_2$ représente un halogénophényle ou le naphtyle et $R_1$, $R_1'$, $R_2'$ ainsi que $R_3$ représentent l'hydrogène, R représente le méthyle, ou l'un de ses sels pharmaceutiquement utilisables.

**49.** Utilisation selon la revendication 44 pour la fabrication de préparations pharmaceutiques contenant, comme substance active pharmaceutique, un composé de formule I où $R_1$, $R_1'$, $R_2'$ et $R_3$ représentent l'hydrogène, $R_2$ représente l'hydrogène ou l'hydroxy, $R_4$ et $R_5$ représentent l'hydrogène ou $R_4$ un reste phénylalkyle en $C_1$-$C_4$ non substitué ou mono- ou polysubstitué dans la partie phényle par un alkyle en $C_1$-$C_4$, un alcoxy en $C_1$-$C_4$, un halogène et/ou le trifluorométhyle et $R_5$ représente l'hydrogène ou un alkyle en $C_1$-$C_4$ et R représente un alkyle en $C_2$-$C_7$, un $\alpha,\alpha$-dialcoxy en $C_1$-$C_4$ alkyle en $C_1$-$C_4$, un $\alpha,\alpha$-dihalogénoalkyle en $C_2$-$C_7$, un cycloalkyle en $C_3$-$C_6$, un cycloalkyle en $C_3$-$C_6$ alkyle en $C_1$-$C_4$, un a-(cycloalcényle en $C_3$-$C_6$)alkyle en $C_1$-$C_4$, un a-(mono-, di- ou trihydroxycycloalkyle en $C_3$-$C_6$)alkyle en $C_1$-$C_4$ ou un reste phénylalkyle en $C_1$-$C_4$ non substitué ou mono- ou polysubstitué dans la partie phényle par un alkyle en $C_1$-$C_4$, un alcoxy en $C_1$-$C_4$, un halogène et/ou le trifluorométhyle, ou l'un de ses sels pharmaceutiquement utilisables.

**50.** Utilisation selon la revendication 45 pour la fabrication de préparations pharmaceutiques contenant, comme substance active pharmaceutique, un composé de formule I où $R_1$, $R_1'$, $R_2'$, $R_3$, $R_4$ et $R_5$ représentent l'hydrogène, $R_2$ l'hydrogène ou l'hydroxy et R représente un alkyle en $C_2$-$C_7$, un $\alpha,\alpha$-dialcoxy en $C_1$-$C_4$ alkyle en $C_1$-$C_4$, un $\alpha,\alpha$-dihalogénoalkyle en $C_2$-$C_7$, un cycloalkyle en $C_3$-$C_6$, un cycloalkyle en $C_3$-$C_6$ alkyle en $C_1$-$C_4$ ou le benzyle ou l'un de ses sels pharmaceutiquement utilisables.

**51.** Utilisation selon la revendication 44 pour la fabrication de préparations pharmaceutiques contenant, comme substance active pharmaceutique, l'acide 3-aminopropyl(cyclohexylméthyl)phosphinique ou l'un de ses sels pharmaceutiquement utilisables.

**52.** Utilisation selon la revendication 44 pour la fabrication de préparations pharmaceutiques contenant, comme substance active pharmaceutique, l'acide 3-aminopropyl(n-butyl)phosphinique ou l'un de ses sels pharmaceutiquement utilisables.

**53.** Utilisation selon la revendication 44 pour la fabrication de préparations pharmaceutiques contenant, comme substance active pharmaceutique, l'acide 3-aminopropyl(diéthoxyméthyl)phosphinique ou l'un de ses sels pharmaceutiquement utilisables.

**54.** Utilisation selon la revendication 44 pour la fabrication de préparations pharmaceutiques contenant, comme substance active pharmaceutique, l'acide 3-aminopropyl(benzyl)phosphinique ou l'un de ses sels pharmaceutiquement utilisables.

**55.** Utilisation selon la revendication 44 pour la fabrication de préparations pharmaceutiques contenant, comme substance active pharmaceutique, l'acide 3-aminopropyl(1,1-difluorobutyl)phosphinique ou l'un de ses sels pharmaceutiquement utilisables.

**56.** Utilisation selon la revendication 44 pour la fabrication de préparations pharmaceutiques contenant, comme substance active pharmaceutique, l'acide 3-amino-2-hydroxypropyl(cyclohexylméthyl)-phosphinique ou l'un de ses sels pharmaceutiquement utilisables.

**57.** Utilisation selon la revendication 44 pour la fabrication de préparations pharmaceutiques contenant, comme substance active pharmaceutique, l'acide 3-aminopropyl(cyclohexylméthyl)phosphinique ou l'un de ses sels pharmaceutiquement utilisables.

**58.** Utilisation selon la revendication 44 pour la fabrication de préparations pharmaceutiques contenant, comme substance active pharmaceutique, l'acide 3-amino-2-(p-chlorophényl)propyl(méthyl)-phosphinique ou l'un de ses sels pharmaceutiquement utilisables.

**59.** Utilisation selon la revendication 44 pour la fabrication de préparations pharmaceutiques contenant, comme substance active pharmaceutique, l'acide 3-aminopropyl(n-butyl)phosphinique ou l'un de ses sels pharmaceutiquement utilisables.

**60.** Utilisation selon la revendication 44 pour la fabrication de préparations pharmaceutiques contenant, comme substance active pharmaceutique, l'acide 3-aminopropyl(diéthoxyméthyl)phosphinique ou l'un de ses sels pharmaceutiquement utilisables.

**61.** Utilisation selon la revendication 44 pour la fabrication de préparations pharmaceutiques contenant, comme substance active pharmaceutique, l'acide 3-aminopropyl(benzyl)phosphinique ou l'un de ses sels pharmaceutiquement utilisables.

**62.** Utilisation selon la revendication 44 pour la fabrication de préparations pharmaceutiques contenant, comme substance active pharmaceutique, l'acide 3-aminopropyl(1,1-difluorobutyl)phosphinique ou l'un de ses sels pharmaceutiquement utilisables.

**63.** Utilisation selon la revendication 44 pour la fabrication de préparations pharmaceutiques contenant, comme substance active pharmaceutique, l'acide 3-amino-2-hydroxypropyl(cyclohexylméthyl)-phosphinique ou l'un de ses sels pharmaceutiquement utilisables.

**64.** Utilisation selon la revendication 44 pour la fabrication de préparations pharmaceutiques contenant, comme substance active pharmaceutique, l'acide 3-amino-2(R)-hydroxypropyl(cyclohexylméthyl)-phosphinique ou l'un de ses sels pharmaceutiquement utilisables.

**Revendications pour les Etats contractants suivants : ES, GR**

**1.** Procédé pour la préparation des nouveaux acides aminoalcanephosphiniques araliphatiques N-substitués de formule I

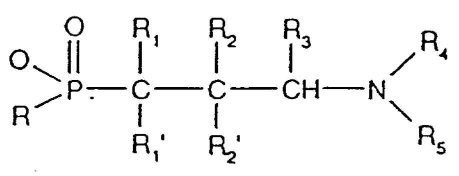

(I),

où $R_1$, $R_1'$, $R_2'$ et $R_3$ représentent l'hydrogène, $R_2$ représente l'hydrogène ou l'hydroxy, $R_4$ un reste phénylalkyle en $C_1$-$C_4$, diphénylalkyle en $C_1$-$C_4$ ou naphtylalkyle en $C_1$-$C_4$ mono-, di- ou trisubstitué dans la partie phényle ou naphtyle par un alkyle en $C_1$-$C_4$, un alcoxy en $C_1$-$C_4$, un hydroxy et/ou un halogène ou un reste thiényl-, furyl- ou pyridylalkyle en $C_1$-$C_4$ non substitué ou substitué dans la partie thiényle, furyle ou pyridyle par un halogène, $R_5$ représente l'hydrogène, un alkyle en $C_1$-$C_4$ ou un groupe $R_4$ et R représente un alkyle en $C_2$-$C_4$, un alcényle en $C_2$-$C_4$, un alcynyle en $C_2$-$C_7$, un oxoalkyle en $C_2$-$C_7$, un hydroxyalkyle en $C_2$-$C_7$ ou un dihydroxyalkyle en $C_2$-$C_7$, un $\alpha$-hydroxyalcényle en $C_3$-$C_5$, un mono-, di- ou trifluoroalkyle en $C_2$-$C_5$, un mono-, di- ou trifluoroalcényle en $C_2$-$C_5$, un mono, di- ou trifluoro-$\alpha$-hydroxyalkyle en $C_2$-$C_5$, un mono-, di- ou trifluoro-$\alpha$-hydroxyalcényle en $C_2$-$C_5$, un alcoxy en $C_1$-$C_4$ alkyle en $C_1$-$C_4$, un dialcoxy en $C_1$-$C_4$ alkyle en $C_1$-$C_4$, un alcoxy en $C_1$-$C_4$ alcoxy en $C_1$-$C_4$(hydroxy)alkyle en $C_2$-$C_7$, un alcoxy en $C_1$-$C_4$(halo)alkyle en $C_2$-$C_5$, un alkylthio en $C_1$-$C_4$ alkyle en $C_1$-$C_4$, un dialkyle en $C_1$-$C_4$ thioalkyle en $C_1$-$C_4$, un cycloalkyle en $C_3$-$C_8$, un $\alpha$-hydroxycycloalkyle en $C_3$-$C_6$, un oxa- ou thiacycloalkyle en $C_3$-$C_6$, un 1,3-dioxa- ou 1,3-dithiacycloalkyle en $C_3$-$C_8$, un cycloalkyle en $C_3$-$C_8$ alkyle en $C_1$-$C_4$, un cycloalcényle en $C_3$-$C_8$ alkyle en $C_1$-$C_4$, un mono-, di-

ou trihydroxycycloalcényle en $C_3$-$C_8$, un cycloalkyle en $C_3$-$C_6$(hydroxy)alkyle en $C_1$-$C_4$, un 1-(alkyle en $C_1$-$C_4$ thiocycloalkyle en $C_3$-$C_6$)-1-hydroxyalkyle en $C_1$-$C_4$, un mono- ou diphénylalkyle en $C_1$-$C_4$ non substitué ou mono-, di- ou trisubstitué par un alkyle en $C_1$-$C_4$, un alcoxy en $C_1$-$C_4$, un halogène, un hydroxy et/ou le trifluorométhyle, un naphtyle en $C_1$-$C_4$ ou un thiényl-, furyl- ou pyridylalkyle en $C_1$-$C_4$ non substitué ou substitué par un halogène et de leurs sels, caractérisé en ce que l'on libère dans un composé de formule II

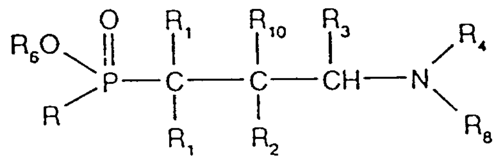

(II),

où $R_1$, $R_1$', $R_2$' et $R_3$ représentent l'hydrogène, $R_6$ un groupe protecteur de l'hydroxy, $R_8$ un groupe $R_5$ ou un groupe protecteur de l'amino et $R_{10}$ représente l'hydrogène ou un hydroxy protégé, R, $R_1$, $R_4$ et $R_5$ ayant la signification qui a été indiquée ou dans l'un de ses sels les groupes hydroxy, en remplaçant le groupe protecteur de l'hydroxy $R_6$ par l'hydrogène et en ce que éventuellement l'on clive le groupe protecteur de l'amino $R_8$ et en ce que éventuellement l'on libère les groupes hydroxy $R_2$ des groupes hydroxy protégés $R_{10}$ et, si désiré, en ce que l'on transforme le composé obtenu en un autre composé de formule I, en ce que l'on sépare le mélange d'isomères obtenu conformément au procédé de l'invention en ses composants et en ce que l'on sépare l'isomère préféré et/ou en ce que l'on transforme le composé libre obtenu par le procédé conforme à l'invention en un sel ou le sel obtenu par le procédé conforme à l'invention en un composé libre correspondant.

**2.** Procédé selon l revendication 1 pour la préparation des composés de formule I où $R_1$, $R_1$', $R_2$' et $R_3$ représentent l'hydrogène, $R_2$ l'hydrogène ou l'hydroxy, $R_4$ un reste phényl- ou naphtylalkyle en $C_1$-$C_4$ substitué par un alkyle en $C_1$-$C_4$, un alcoxy en $C_1$-$C_4$, l'hydroxy et/ou un halogène ou un reste thiényl-, furyl- ou pyridylalkyle en $C_1$-$C_4$ non substitué ou substitué par un halogène, $R_5$ représente l'hydrogène, un alkyle inférieur ou un groupe $R_4$ et R représente un alkyle en $C_2$-$C_4$, un alcényle en $C_2$-$C_4$, un alcynyle en $C_2$-$C_7$, un oxoalkyle en $C_2$-$C_7$, un hydroxyalkyle en $C_2$-$C_7$ ou un dihydroxyalkyle en $C_2$-$C_7$, un $\alpha$-hydroxyalcényle en $C_3$-$C_5$, un mono-, di- ou trifluoroalkyle en $C_2$-$C_5$, un mono-, di- ou trifluoroalcényle en $C_2$-$C_5$, un mono-, di- ou trifluoro-$\alpha$-hydroxyalkyle en $C_2$-$C_5$, un mono-, di- ou trifluoro-$\alpha$-hydroxyalcényle en $C_2$-$C_5$, un alcoxy en $C_1$-$C_4$ alkyle en $C_1$-$C_4$, un dialcoxy en $C_1$-$C_4$ alkyle en $C_1$-$C_4$, un alcoxy en $C_1$-$C_4$ (hydroxy)alkyle en $C_2$-$C_7$, un alcoxy en $C_1$-$C_4$ (halo)alkyle en $C_2$-$C_5$, un alkyle en $C_1$-$C_4$ thioalkyle en $C_1$-$C_4$, un dialkyle en $C_1$-$C_4$ thioalkyle en $C_1$-$C_4$, un cycloalkyle en $C_3$-$C_8$, un $\alpha$-hydroxycycloalkyle en $C_3$-$C_6$, un oxa- ou thiacycloalkyle en $C_3$-$C_6$, un 1,3-dioxa- ou 1,3-dithiacycloalkyle en $C_3$-$C_8$, un cycloalkyle en $C_3$-$C_6$ (hydroxy)alkyle en $C_1$-$C_4$, un 1-(alkyle en $C_1$-$C_4$ thiocycloalkyle en $C_3$-$C_6$)-1-hydroxyalkyle en $C_1$-$C_4$ ou un phénylalkyle en $C_1$-$C_4$ ou un naphtylalkyle en $C_1$-$C_4$ non substitué ou substitué par un alkyle en $C_1$-$C_4$, un alcoxy en $C_1$-$C_4$, un hydroxy et/ou un halogène et de leurs sels.

**3.** Procédé selon la revendication 1 pour la préparation des composés de formule I où R représente un alkyle en $C_3$-$C_7$, un $\alpha,\alpha$-dialcoxy en $C_1$-$C_4$ alkyle en $C_1$-$C_4$, un cycloalkyle en $C_3$-$C_6$ alkyle en $C_1$-$C_4$, un cycloalcényle en $C_3$-$C_6$ alkyle en $C_1$-$C_4$, un mono-, di- ou trihydroxycycloalkyle en $C_3$-$C_6$ alkyle en $C_1$-$C_4$ ou un benzyle non substitué ou mono-, di- ou trisubstitué par un alkyle en $C_1$-$C_4$, un alcoxyen $C_1$-$C_4$, l'hydroxy et/ou un halogène, $R_2$ représente l'hydrogène ou un hydroxy, $R_1$, $R_1$', $R_2$' et $R_3$ représentent l'hydrogène, $R_4$ représente un phényl- ou diphénylalkyle en $C_1$-$C_4$ mono-, di- ou trisubstitué par un alkyle en $C_1$-$C_4$, un alcoxy en $C_1$-$C_4$ et/ou un halogène ou un naphtylalkyle en $C_1$-$C_4$ non substitué ou monosubstitué par un halogène ayant un numéro atomique allant jusqu'à 35 et $R_5$ représente l'hydrogène, un alkyle en $C_1$-$C_4$ ou un groupe $R_4$ et de leurs sels.

**4.** Procédé selon la revendication 1 pour la préparation des composés de formule I où $R_1$, $R_1$', $R_2$' et $R_3$ représentent l'hydrogène $R_2$ représente l'hydrogène ou l'hydroxy, $R_4$ un phénylalkyle en $C_1$-$C_4$ monosubstitué par un alkyle en $C_1$-$C_4$ ou par un alcoxy en $C_1$-$C_4$ ou mono- ou disubstitué par un halogène ayant un numéro atomique allant jusqu'à 35 ou un naphtyl-, thiényl-, furyl- ou pyridylalkyle en $C_1$-$C_4$ non substitué ou monosubstitué par un halogène ayant un numéro atomique allant jusqu'à 35, $R_5$ représente l'hydrogène, un alkyle en $C_1$-$C_4$ ou un groupe $R_4$ et R représente un alkyle en $C_3$-$C_7$, un

α,α-dialcoxy en $C_1$-$C_4$ alkyle en $C_1$-$C_4$, un cycloalkyle en $C_3$-$C_6$ alkyle en $C_1$-$C_4$ ou un benzyle non substitué ou substitué par un alkyle en $C_1$-$C_4$, un alcoxy en $C_1$-$C_4$ ou un halogène ayant un numéro atomique allant jusqu'à 35 et de leurs sels.

**5.** Procédé selon la revendication 1 pour la préparation des composés de formule I où $R_1$, $R_1'$, $R_2'$ et $R_3$ représentent l'hydrogène, $R_2$ représente l'hydrogène ou l'hydroxy, $R_4$ représente un α-phénylalkyle en $C_1$-$C_4$ mono- ou disubstitué par un halogène ayant un numéro atomique allant jusqu'à 35 ou un α-naphtylalkyle en $C_2$-$C_4$, un α-thiénylalkyle en $C_1$-$C_4$, un α-furylalkyle en $C_1$-$C_4$ ou un α-pyridylalkyle en $C_1$-$C_4$ non substitué, $R_5$ représente l'hydrogène, un alkyle en $C_1$-$C_4$ ou un groupe $R_4$ et R un alkyle en $C_3$-$C_5$, un α,α-dialcoxy en $C_1$-$C_4$méthyle, un cycloalkyle en $C_3$-$C_6$ alkyle en $C_1$-$C_4$, un α-(cycloalcényle en $C_3$-$C_6$)alkyle en $C_1$-$C_4$, un α-(mono-, di- ou trihydroxycycloalkyle en $C_3$-$C_6$)alkyle en $C_1$-$C_4$ ou un benzyle et de leurs sels.

**6.** Procédé selon la revendication 1 pour la préparation des composés de formule I où $R_1$, $R_1'$, $R_2'$ et $R_3$ représentent l'hydrogène $R_2$ représente l'hydrogène ou l'hydroxy, $R_4$ un phénylalkyle en $C_1$-$C_4$ mono- ou disubstitué par un halogène ayant un numéro atomique allant jusqu'à 35 ou un naphtyl-, thiényl-, furyl- ou pyridylalkyle en $C_1$-$C_4$ non substitué, $R_5$ l'hydrogène, un alkyle en $C_1$-$C_4$ ou un groupe $R_4$ et R représente un alkyle en $C_3$-$C_5$, un α,α-dialcoxy en $C_1$-$C_4$ méthyle, un cycloalkyle en $C_3$-$C_6$ alkyle en $C_1$-$C_4$ ou un benzyle et de leurs sels.

**7.** Procédé selon la revendication 1 pour la préparation de l'acide 3-(p-chlorobenzylamino)propyl-(diéthoxyméthyl)phosphinique ou de l'un de ses sels.

**8.** Procédé selon la revendication 1 pour la préparation de l'acide 3-(p-chlorobenzylamino)-2(S)-hydroxypropyl(benzyl)phosphinique ou de l'un de ses sels.

**9.** Procédé selon la revendication 1 pour la préparation de l'acide 3-(p-chlorobenzylamino)propyl(n-butyl)-phosphinique ou de l'un de ses sels.

**10.** Procédé selon la revendication 1 pour la préparation de l'acide 3-(p-chlorobenzylamino)propyl-(cyclohexylméthyl)phosphinique ou de l'un de ses sels.

**11.** Procédé selon la revendication 1 pour la préparation de l'acide 3-(p-chlorobenzylamino)-2(R)-hydroxypropyl(benzyl)phosphinique ou de l'un de ses sels.

**12.** Procédé selon la revendication 1 pour la préparation de l'acide 3-(3,4-dichlorobenzylamino)propyl-(diéthoxyméthyl)phosphinique ou de l'un de ses sels.

**13.** Procédé selon la revendication 1 pour la préparation de l'acide 3-[1-(p-chlorophényl)éthylamino]propyl-(diéthoxyméthyl)phosphinique ou de l'un de ses sels.

**14.** Procédé selon la revendication 1 pour la préparation de l'acide 3-(naphtyl-1-ylméthylamino)propyl-(diéthoxyméthyl)phosphinique ou de l'un de ses sels.

**15.** Procédé selon la revendication 1 pour la préparation de l'acide 3-(3,4-dichlorobenzylamino)propyl-(cyclohexylméthyl)phosphinique ou de l'un de ses sels.

**16.** Procédé selon la revendication 1 pour la préparation de l'acide 3-(pyrid-2-ylméthylamino)-2(S)-hydroxypropyl(benzyl)phosphinique ou de l'un de ses sels.

**17.** Procédé selon la revendication 1 pour la préparation de l'acide 3-(p-chlorobenzylaminoyl)-2(S)-hydroxypropyl(cyclohexylméthyl)phosphinique ou de l'un de ses sels.

**18.** Procédé selon la revendication 1 pour la préparation de l'acide 3-(3,4-dichlorobenzylaminoyl)-2(S)-hydroxypropyl(benzyl)phosphinique ou de l'un de ses sels.

**19.** Procédé selon la revendication 1 pour la préparation de l'acide 3-(p-fluorobenzylamino)propyl-(diéthoxyméthyl)phosphinique ou de l'un de ses sels.

**20.** Procédé selon la revendication 1 pour la préparation de l'acide 3-(4-chloro-3-trifluorométhylbenzylamino)propyl(diéthoxyméthyl)phosphinique ou de l'un de ses sels.

**21.** Procédé selon la revendication 1 pour la préparation de l'acide 3-(m-chlorobenzylamino)propyl-(diéthoxyméthyl)phosphinique ou de l'un de ses sels.

**22.** Procédé selon la revendication 1 pour la préparation de l'acide 3-(3,4-dichlorobenzylamino)-2(S)-hydroxypropyl(cyclohexylméthyl)phosphinique ou de l'un de ses sels.

**23.** Procédé selon la revendication 1 pour la préparation de l'acide 3-[1-(3,4-dichlorophényl)éthylamino]-propyl(diéthoxyméthyl)phosphinique ou de l'un de ses sels.

**24.** Procédé selon la revendication 1 pour la préparation de l'acide 3-[1-(p-chlorophényl)éthylamino]-2(S)-hydroxypropyl(benzyl)phosphinique ou de l'un de ses sels.

**25.** Procédé selon la revendication 1 pour la préparation de l'acide 3-(p-iodobenzylamino)propyl-(diéthoxyméthyl)phosphinique ou de l'un de ses sels.

**26.** Procédé selon la revendication 1 pour la préparation de l'acide 3-(2,4-dichlorobenzylamino)propyl-(diéthoxyméthyl)phosphinique ou de l'un de ses sels.

**27.** Procédé selon la revendication 1 pour la préparation de l'acide 3-(o-chlorobenzylamino)propyl-(diéthoxyméthyl)phosphinique ou de l'un de ses sels.

**28.** Procédé selon la revendication 1 pour la préparation de l'acide 3-(3,4-dichlorobenzylamino)propyl-(cyclopropylméthyl)phosphinique ou de l'un de ses sels.

**29.** Procédé selon la revendication 1 pour la préparation de l'acide 3-(p-chlorobenzylamino)propyl-(tétrahydrofuran-2-yl)phosphinique ou de l'un de ses sels.

**30.** Procédé selon la revendication 1 pour la préparation de l'acide 3-[N-(p-chlorobenzyl)-N-méthylamino]-propyl(diéthoxyméthyl)phosphinique ou de l'un de ses sels.

**31.** Procédé selon la revendication 1 pour la préparation de l'acide 3-(p-chlorobenzylamino)-2(S)-hydroxypropyl(p-méthylbenzyl)phosphinique ou de l'un de ses sels.

**32.** Procédé selon la revendication 1 pour la préparation de l'acide 3-(3,4-dichlorobenzylamino)-2(S)-hydroxypropyl(p-méthylbenzyl)phosphinique ou de l'un de ses sels.

**33.** Procédé selon la revendication 1 pour la préparation de l'acide 3-(p-chlorobenzylamino)-2(S)-hydroxypropyl(p-chlorobenzyl)phosphinique ou de l'un de ses sels.

**34.** Procédé selon la revendication 1 pour la préparation de l'acide 3-(3,4-dichlorobenzylamino)-2(S)-hydroxypropyl(p-chlorobenzyl)phosphinique ou de l'un de ses sels.

**35.** Procédé selon la revendication 1 pour la préparation de l'acide 3-(p-chlorobenzylamino)-2(S)-hydroxypropyl(diéthoxyméthyl)phosphinique,
l'acide 3-[1-(p-chlorophényl)éthylamino]-2(S)-hydroxypropyl(diéthoxyméthyl)phosphinique,
l'acide 3-(3,4-dichlorobenzylamino)-2(S)-hydroxypropyl(diéthoxyméthyl)phosphinique,
l'acide 3-[1-(3,4-dichlorophényl)éthylamino]-2(S)-hydroxypropyl(diéthoxyméthyl)phosphinique,
l'acide 3-[N-(p-chlorobenzyl)-N-méthylamino]-2(S)-hydroxypropyl(benzyl)phosphinique,
l'acide 3-(p-chlorobenzylamino)-2(S)-hydroxypropyl(p-méthoxybenzyl)phosphinique,
l'acide 3-(p-chlorobenzylamino)-2(S)-hydroxypropyl(3,4-diméthoxybenzyl)phosphinique,
l'acide 3-(3,4-dichlorobenzylamino)-2(S)-hydroxypropyl(3,4-diméthoxybenzyl)phosphinique,
l'acide 3-[2-(p-chlorophényl)prop-2-ylamlno]propyl(diéthoxyméthyl)phosphinique,
l'acide 3-[2-(3,4-dichlorophényl)prop-2-ylamino]propyl(diéthoxyméthyl)phosphinique,
l'acide 3-[2-(p-chlorophényl)prop-2-ylamino]-2(S)-hydroxypropyl(benzyl)phosphinique,
l'acide 3-[2-(3,4-dichlorophényl)prop-2-ylamino]-2(S)-hydroxypropyl(benzyl)phosphinique,

l'acide 3-(3,4-dichlorobenzylamino)-2(S)-hydroxypropyl(3,4,5-trihydroxycyclohexylméthyl)phosphinique,
l'acide 3-(4-chloro-3-méthoxybenzylamino)propyl(diéthoxyméthyl)phosphinique,
l'acide 3-(4-chloro-3-méthoxybenzylamino)-2(S)-hydroxypropyl(benzyl)phosphinique,
l'acide 3-(2-phényléthylamino)propyl(diéthoxyméthylphosphinique,
l'acide 3-(2-phényléthylamino)-2(S)-hydroxypropyl(diéthoxyméthyl)phosphinique,
l'acide 3-(p-chlorobenzylamino)-2(S)-hydroxypropyl(4-méthoxycyclohexyl)phosphinique,
l'acide 3-(3,4-dichloro-6-iodobenzylamino)-2(S)-hydroxypropyl(benzyl)phosphinique,
l'acide 3-(3,4-dichloro-6-iodobenzylamino)propyl(diéthoxyméthyl)phosphinique,
l'acide 3-(4-chloro-3-iodobenzylamino)propyl(diéthoxyméthyl)phosphinique,
l'acide 3-(3-chloro-4-iodobenzylamino)propyl(diéthoxyméthyl)phosphinique,
l'acide 3-(4-chloro-3-iodobenzylamino)-2(S)-hydroxypropyl(benzyl)phosphinique,
l'acide 3-(3-chloro-4-iodobenzylamino)-2(S)-hydroxypropyl(benzyl)phosphinique,
l'acide 3-di(p-chlorobenzyl)aminopropyl(diéthoxyméthyl)phosphinique,
l'acide 3-di(3,4-dichlorobenzyl)amino-2(S)-hydroxypropyl(benzyl)phosphinique,
l'acide 3-(m-chlorobenzylamino)-2(S)-hydroxypropyl(benzyl)phosphinique,
l'acide 3-(3,4-dichlorobenzyl)amino-2(S)-hydroxypropyl(cis-4,5-dihydroxycyclohexylméthyl)-phosphinique,
l'acide 3-(3,4-dichlorobenzyl)amino-2(S)-hydroxypropyl(cyclohex-3-énylméthyl)phosphinique,
l'acide 3-[1-(3,4-dichlorophényl)éthylamino]-2(S)-hydroxypropyl(cyclohex-3-énylméthyl)phosphinique ou
l'acide 3-[1-(3,4-dichlorophényl)éthylamino]-2(S)-hydroxypropyl(cis-4,5-dihydroxycyclohexylméthyl)-phosphinique
ou l'un de leurs sels.

**36.** Procédé pour l'obtention de préparations pharmaceutiques, caractérisé en ce que l'on mélange un composé de formule I

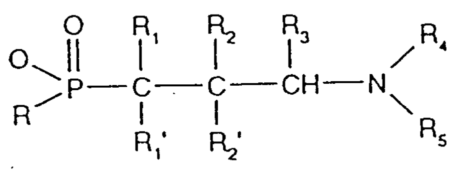

(I),

où $R_1$, $R_1$', $R_2$' et $R_3$ représentent l'hydrogène, $R_2$ représente l'hydrogène ou l'hydroxy, $R_4$ un reste phénylalkyle en $C_1$-$C_4$, diphénylalkyle en $C_1$-$C_4$ ou naphtylalkyle en $C_1$-$C_4$ non substitué ou mono-, di- ou trisubstitué dans la partie phényle ou naphtyle par un alkyle en $C_1$-$C_4$, un alcoxy en $C_1$-$C_4$, un hydroxy et/ou un halogène ou un reste thiényl-, furyl- ou pyridylalkyle en $C_1$-$C_4$ non substitué ou substitué dans la partie thiényle, furyle ou pyridyle par un halogène, $R_5$ représente l'hydrogène, un alkyle en $C_1$-$C_4$ ou un groupe $R_4$ et R représente un alkyle en $C_2$-$C_4$, un alcényle en $C_2$-$C_4$, un alcynyle en $C_2$-$C_7$, un oxoalkyle en $C_2$-$C_7$, un hydroxyalkyle en $C_2$-$C_7$ ou un dihydroxyalkyle en $C_2$-$C_7$, un $\alpha$-hydroxyalcényle en $C_3$-$C_5$, un mono-, di- ou trifluoroalkyle en $C_2$-$C_5$, un mono-, di- ou trifluoroalcényle en $C_2$-$C_5$, un mono-, di- ou trifluoro-$\alpha$-hydroxyalkyle en $C_2$-$C_5$, un mono-, di- ou trifluoro-$\alpha$-hydroxyalcényle en $C_2$-$C_5$, un alcoxy en $C_1$-$C_4$ alkyle en $C_1$-$C_4$, un dialcoxy en $C_1$-$C_4$ alkyle en $C_1$-$C_4$, un alcoxy en $C_1$-$C_4$ alcoxy en $C_1$-$C_4$(hydroxy)alkyle en $C_2$-$C_7$, un alcoxy en $C_1$-$C_4$(halo)alkyle en $C_2$-$C_5$, un alkylthio en $C_1$-$C_4$ alkyle en $C_1$-$C_4$, un dialkyle en $C_1$-$C_4$ thioalkyle en $C_1$-$C_4$, un cycloalkyle en $C_3$-$C_8$, un $\alpha$-hydroxycycloalkyle en $C_3$-$C_6$, un oxa- ou thiacycloalkyle en $C_3$-$C_6$, un 1,3-dioxa- ou 1,3-dithiacycloalkyle en $C_3$-$C_8$, un cycloalkyle en $C_3$-$C_8$alkyle en $C_1$-$C_4$, un cycloalcényle en $C_3$-$C_8$ alkyle en $C_1$-$C_4$, un mono-, di- ou trihydroxycycloalcényle en $C_3$-$C_8$, un cycloalkyle en $C_3$-$C_6$(hydroxy)alkyle en $C_1$-$C_4$, un 1-(alkyle en $C_1$-$C_4$ thiocycloalkyle en $C_3$-$C_6$)-1-hydroxyalkyle en $C_1$-$C_4$, un mono- ou diphénylalkyle en $C_1$-$C_4$ non substitué ou mono-, di- ou trisubstitué par un alkyle en $C_1$-$C_4$, un alcoxy en $C_1$-$C_4$, un halogène, un hydroxy et/ou le trifluorométhyle, un naphtylalkyle en $C_1$-$C_4$ ou un thiényl-, furyl- ou pyridylalkyle en $C_1$-$C_4$ non substitué ou substitué par un halogène, sous forme libre ou sous forme d'un sel pharmaceutiquement utilisable avec des adjuvants pharmaceutiques usuels.

**37.** Procédé selon la revendication 36 pour l'obtention de préparations pharmaceutiques, caractérisé en ce que l'on mélange un composé selon l'une des revendications 1 à 35 sous forme libre ou sous forme d'un sel pharmaceutiquement utilisable avec des adjuvants pharmaceutiques usuels.

**38.** Procédé selon la revendication 36 ou 37 pour l'obtention de préparations pharmaceutiques destinées au traitement des épilepsies des différentes formes du "petit mal", ainsi que pour la suppression des états analogues au "petit mal" pouvant intervenir lors du traitement avec la phénytoïne, la carbamazépine, la Vigabatrine® et des antiépileptiques présentant un profil d'action identique ou analogue.